# EUROPEAN PATENT APPLICATION

(11) **EP 4 424 324 A2**
(43) Date of publication of application: **04.09.2024**
(21) Application number: 24174774.0
(22) Date of filing: 02.10.2018
(51) Int. Cl.: A61K 39/00

(54) **DOSAGE AND ADMINISTRATION OF ANTI-C5 ANTIBODIES FOR TREATMENT OF PATIENTS WITH MEMBRANOPROLIFERATIVE GLOMERULONEPHRITIS**

(30) Priority: 04.10.2017 US 201762568060 P; 04.04.2018 US 201862652615 P
(62) Divisional of application: 18796165.1
(71) Applicant: ALEXION PHARMACEUTICALS, INC., Boston, MA 02210 (US)
(72) Inventor: REMUZZI, Giuseppe, 24129 Bergamo (IT); RUGGENENTI, Piero, 24128 Bergamo (IT); GAO, Xiang, Guilford, 06437 (US)
(74) Representative: J A Kemp LLP

(57) **Abstract**

Provided are methods for clinical treatment of Membranoproliferative glomerulonephritis (MPGN) by administering an anti-C5 antibody, or antigen binding fragment thereof.

## Description

### RELATED APPLICATIONS

This application claims priority to U.S. Provisional Application No. 62/652,615, filed on April 4, 2018, and U.S. Provisional Application No. 62/568,060, filed on October 4, 2017. The entire contents of the aforementioned applications is incorporated herein by reference.

### SEQUENCE LISTING

The instant application contains a Sequence Listing which has been submitted electronically in ASCII format and is hereby incorporated by reference in its entirety. Said ASCII copy, created on September 27, 2018, is named AXJ-201PC_SL.txt and is 33,104 bytes in size.

### BACKGROUND

Membranoproliferative glomerulonephritis (also known as "MPGN") is an uncommon cause of chronic nephritis that can occur at any age, mainly in children and young adults (see, *e.g.,* Alchi B., et al., Pediatr. Nephrol. (2010) 25:1409-1418). MPGN is diagnosed on the basis of a glomerular-injury pattern that may be secondary to a heterogeneous group of diseases and may be secondary to chronic infection (hepatitis C and B, bacterial, fungal and parasitic infection), autoimmune disease (LES, and occasionally Sjogren syndrome, rheumatoid arthritis and mixed connective-tissue disorders), malignancies (monoclonal gammopathy, B cell lymphoma, chronic lymphocytic leukemia) or may be primary (see, *e.g.,* Sethi S., et al., Semin. Nephrol. 2011; 31:341-8).

MPGN accounts for approximately 7 to 10% of all cases of biopsy-confirmed glomerulonephritis and ranks as the third or fourth leading cause of end-stage renal disease (ESRD) among the primary glomerulonephritis. The typical features of MPGN on light microscopy include mesangial hypercellularity, endocapillary proliferation, and capillary-wall remodeling (with the formation of double contours); all of which result in lobular accentuation of the glomerular tufts. These changes result from the deposition of immunoglobulins, complement factors, or both in the glomerular mesangium and along the glomerular capillary walls (see, *e.g.,* Sethi S., et al., N. Engl. J. Med., 2012; 366(12):1119-31).

Hyperactivation of complement system play a main role in the pathogenesis of MPGN through the classical pathway activated by immune complexes and through the alternative pathway (see, *e.g.,* Fakhouri F., et al., Nat. Rev. Nephrol., 2010; 6:494-499). Dysregulation of the alternative pathway results in activated complement products, including C3b and terminal complement factors, that are delivered indiscriminately to all cell surfaces, including glomerular capillary surface, in which this deposition triggers inflammation with subsequent development of MPGN (see, *e.g.,* Fervenza FC, et al., Nephrol. Dial Transplant 2012; 27: 4288-4294).

Persistently decreased serum levels of complement C3, C4, or both are commonly seen in patients with MPGN. Low C3 and low C4 complement levels are more common in immune-complex mediated MPGN, whereas low C3 and normal C4 levels are more common in alternative-pathway dysfunction, particularly in the acute phase. A normal C3 level does not rule out alternative pathway dysfunction (see, *e.g.,* Sethi S, Fervenza FC., N. Engl. J. Med., 2012; 366(12):1119-31).

Familial forms for all types of MPGN have been reported suggesting that genetic abnormalities may play a predisposing role to the disease. Acquired and genetic abnormalities associated with hyperactivation of complement system in primary MPGN include antibodies to complement-regulating proteins [C3 convertase (C3 Nephrotic factor), Factor H, Factor I, Factor B], mutations in the complement and in complement-regulating proteins (C3, Factor H, Factor I, MCP, CFHR 5, CFHR 3-1) (see, *e.g.,* Bomback et al., Nat. Rev. Nephrol., 2012; 8,634-642; and Servais A, et al., Kidney Int., 2012 82, 454-464) and allele variants (Factor H, C3, MCP) (see, *e.g.,* Sethi S., et al., Kidney Int., 2012 82, 465-473).

The clinical presentation and course are extremely variable: from benign and slowly progressive to rapidly progressive decline in renal function and most patients with the Nephrotic/Nephrotic phenotype progress to ESRD (see, *e.g.,* Sethi S, Fervenza FC., N. Engl. J. Med., 2012; 366(12):1119-31). Recurrent disease is also a common feature (30-65%) in MPGN patients who receive a renal transplant (Lorenz EC, et al., Kidney Int., 2010; 77:721-8).

In patients with secondary MPGN, treatment should aim at attaining remission of the primary disease (infection, autoimmune disease, hematological dyscrasia). Patients with normal kidney function, no active urinary sediment, and non-nephrotic-range proteinuria can be treated conservatively with angiotensin II blockade to control blood pressure and reduce proteinuria (see, *e.g.,* Ruggenenti P, et al., Lancet 1999; 354:359-364).

As for those with primary MPGN with the Nephrotic-nephrotic phenotype, numerous therapeutic regimens have been tried, including the use of corticosteroids and immunosuppressants (cyclophosphamide, mycophenolate mofetil, cyclosporine, rituximab) anticoagulants, thrombolytics, plasmapheresis and plasma exchange (see, *e.g.,* Alchi B, Jayne D., Pediatr. Nephrol. (2010) 25:1409-1418). While corticosteroid therapy seems to be effective in children, there is no evidence that steroids are effective in modifying disease progression in adults with idiopathic MPGN (see, *e.g.,* Tarshish P, et al., Pediatr. Nephrol. 1992;6:123-30). The humanized monoclonal anti-CD20 antibody rituximab has been used in attempt to deplete the B cells responsible for producing C3NeF, but the results have thus far been limited (see, *e.g.,* Smith R, et al., J. Am. Soc. Nephrol, 2007; 18:2247-2456).

The lack of randomized, controlled trials and the current understanding that multiple pathogenic processes lead to MPGN make it impossible to give strong treatment recommendations in this patient population, in particular for those with primary forms and more severe disease. Accordingly, it is an object of the present invention to provide improved methods for treating patients with MPGN.

### SUMMARY

Provided herein are compositions and methods for treating MPGN in a human patient, comprising administering to the patient an anti-C5 antibody, or antigen binding fragment thereof. In one embodiment, the anti-C5 antibody, or antigen binding fragment thereof, is administered (or is for administration) according to a particular clinical dosage regimen (*i.e*., at a particular dose amount and according to a specific dosing schedule).

In one embodiment, the MPGN is "immune-complex-mediated MPGN" (IC-mediated MPGN). In another embodiment, the MPGN is "complement-mediated MPGN" (*e.g*., a "C3 Glomerulopathy" (also known as "C3G"). In one embodiment, the C3 Glomerulopathy is dense deposit disease (DDD) or C3 glomerulonephritis.

An exemplary anti-C5 antibody is Eculizumab comprising heavy and light chains having the sequences shown in SEQ ID NOs: 10 and 11, respectively, or antigen binding fragments and variants thereof. In other embodiments, the antibody comprises the heavy and light chain CDRs or variable regions of Eculizumab. In another embodiment, the antibody comprises the CDR1, CDR2, and CDR3 domains of the VH region of Eculizumab having the sequence set forth in SEQ ID NO: 7, and the CDR1, CDR2 and CDR3 domains of the VL region of Eculizumab having the sequence set forth in SEQ ID NO: 8. In another embodiment, the antibody comprises heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. In another embodiment, the antibody comprises VH and VL regions having the amino acid sequences set forth in SEQ ID NO: 7 and SEQ ID NO: 8, respectively.

Another exemplary anti-C5 antibody is antibody BNJ441 (also known as ALXN1210) comprising the heavy and light chains having the sequences shown in SEQ ID NOs:14 and 11, respectively, or antigen binding fragments and variants thereof. In other embodiments, the antibody comprises the heavy and light chain complementarity determining regions (CDRs) or variable regions (VRs) of antibody BNJ441. In another embodiment, the antibody comprises the CDR1, CDR2, and CDR3 domains of the heavy chain variable (VH) region of antibody BNJ441 having the sequence shown in SEQ ID NO:12, and the CDR1, CDR2 and CDR3 domains of the light chain variable (VL) region of antibody BNJ441 having the sequence shown in SEQ ID NO:8. In another embodiment, the antibody comprises CDR1, CDR2 and CDR3 heavy chain sequences as set forth in SEQ ID NOs:19, 18, and 3, respectively, and CDR1, CDR2 and CDR3 light chain sequences as set forth in SEQ ID NOs:4, 5, and 6, respectively.

In another embodiment, the antibody comprises VH and VL regions having the amino acid sequences set forth in SEQ ID NO: 12 and SEQ ID NO:8, respectively.

In another embodiment, the antibody comprises a heavy chain constant region as set forth in SEQ ID NO:13.

In another embodiment, the antibody comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434, each in EU numbering.

In another embodiment, the antibody comprises CDR1, CDR2 and CDR3 heavy chain sequences as set forth in SEQ ID NOs:19, 18, and 3, respectively, and CDR1, CDR2 and CDR3 light chain sequences as set forth in SEQ ID NOs:4, 5, and 6, respectively and a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434, each in EU numbering.

Another exemplary anti-C5 antibody is antibody BNJ421 (also known as ALXN1211) comprising heavy and light chains having the sequences shown in SEQ ID NOs:20 and 11, respectively, or antigen binding fragments and variants thereof. In another embodiment, the antibody comprises the heavy and light chain CDRs or variable regions of BNJ421. In another embodiment, the antibody comprises the CDR1, CDR2, and CDR3 domains of the VH region of BNJ421 having the sequence set forth in SEQ ID NO:12, and the CDR1, CDR2 and CDR3 domains of the VL region of BNJ421 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO: 12 and SEQ ID NO:8, respectively.

In another embodiment, the antibody competes for binding with, and/or binds to the same epitope on C5 as, the above-mentioned antibodies. In another embodiment, the antibody has at least about 90% variable region amino acid sequence identity with the above-mentioned antibodies (*e*.*g*., at least about 90%, 95% or 99% variable region identity with SEQ ID NO: 12 and SEQ ID NO:8).

Accordingly, in one aspect, methods of treating a human patient with MPGN are provided, the methods comprising administering to the patient an effective amount of an anti-C5 antibody, or antigen binding fragment thereof. In one embodiment, the patient is an adult patient who has been determined to have biopsy-proven MPGN, creatinine clearance greater than 20 ml/min per 1.73 m2, and/or 24-hour proteinuria exceeding 3.5 g. In another embodiment, the patient is a pediatric patient who has been determined to have biopsy-proven MPGN, creatinine clearance greater than 20 ml/min per 1.73 m2, and/or 24-hour proteinuria exceeding 40 mg/h/m2 (or exceeding 2 mg protein/mg creatinine in spot urine samples). In a further embodiment, the patient (*e*.*g*., pediatric or adult patient) has also been determined to have persistently low C3 levels in at least two consecutive evaluations and/or persistently high sC5b9 levels (>1000 ng/ml) in at least two previous consecutive evaluations.

In one embodiment, the dose of the anti-C5 antibody, or antigen binding fragment thereof, is a flat-fixed dose. For example, the anti-C5 antibody, or antigen binding fragment thereof, may be administered at a fixed dose of 300 mg, 600 mg, 900 mg or 1,200 mg. In certain embodiments, dosage regimens are adjusted to provide the optimum desired response (e.g., an effective response).

In one embodiment, the anti-C5 antibody, or antigen binding fragment thereof, (*e*.*g*., eculizumab) is administered to an adult patient (a) weekly at a dose of 900 mg for four weeks and (b) once every 14 ± 2 days (*e*.*g*., about two weeks) thereafter at a dose of 1,200 mg. In another embodiment, the anti-C5 antibody, or antigen binding fragment thereof, (*e*.*g*., eculizumab) is administered to an adult patient according to an administration cycle comprising (a) an induction phase followed by (b) a maintenance phase, wherein: (a) the induction phase comprises a period of four weeks, wherein the anti-C5 antibody, or antigen binding fragment thereof, is administered at a dose of 900 mg once per week; and (b) during the maintenance phase, the anti-C5 antibody, or antigen binding fragment thereof, is administered once at a dose of 1200 mg on the fifth week of the administration cycle, followed by 1200 mg every 14 ± 2 days thereafter.

In another embodiment, the anti-C5 antibody, or antigen binding fragment thereof, (*e*.*g*., eculizumab) is administered to a pediatric patient according to an administration cycle, wherein the administration cycle comprises (a) an induction phase followed by (b) a maintenance phase, wherein:
(a) the anti-C5 antibody, or antigen binding fragment thereof, is administered during the induction phase at a dose of:
   1. 900 mg once per week for four weeks to a ≥ 40 kg patient;
   2. 600 mg once per week for two weeks to a 30 kg to < 40 kg patient;
   3. 600 mg once per week for two weeks to a 20 kg to < 30 kg patient;
   4. 600 mg once per week for one week to a 10 kg to < 20 kg patient;
   5. 300 mg once per week for one week to a 5 kg to < 10 kg patient; and
(b) the anti-C5 antibody, or antigen binding fragment thereof, is administered during the maintenance phase at a dose of:
   1. 1200 mg on the fifth week of the administration cycle, followed by 1200 mg every two weeks thereafter, to a ≥ 40 kg patient;
   2. 900 mg on the third week of the administration cycle, followed by 900 mg every two weeks thereafter, to a 30 kg to < 40 kg patient;
   3. 600 mg on the third week of the administration cycle, followed by 600 mg every two weeks thereafter, to a 20 kg to < 30 kg patient;
   4. 300 mg on the second week of the administration cycle, followed by 300 mg every two weeks thereafter, to a 10 kg to < 20 kg patient; or
   5. 300 mg on the second week of the administration cycle, followed by 300 mg every three weeks thereafter, to a 5 kg to < 10 kg patient.

In another embodiment, methods of treating a pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein the anti-C5 antibody, or antigen binding fragment thereof, is administered at a dose of:
a) 900 mg once per week for four weeks during the induction phase, 1200 mg on the fifth week of the administration cycle, followed by 1200 mg every two weeks thereafter during the maintenance phase, to a ≥ 40 kg patient;
b) 600 mg once per week for two weeks during the induction phase, 900 mg on the third week of the administration cycle, followed by 900 mg every two weeks thereafter during the maintenance phase, to a 30 kg to < 40 kg patient;
c) 600 mg once per week for two weeks during the induction phase, 600 mg on the third week of the administration cycle, followed by 600 mg every two weeks thereafter during the maintenance phase, to a 20 kg to < 30 kg patient;
d) 600 mg once per week for one week during the induction phase, 300 mg on the second week of the administration cycle, followed by 300 mg every two weeks thereafter during the maintenance phase, to a 10 kg to < 20 kg patient;
e) 300 mg once per week for one week during the induction phase, 300 mg on the second week of the administration cycle, followed by 300 mg every three weeks thereafter during the maintenance phase, to a 5 kg to < 10 kg patient.

In another embodiment, methods of treating a ≥ 40 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 900 mg once per week for four weeks; and
(b) the anti-C5 antibody, or antigen binding fragment thereof, is administered during the maintenance phase at a dose of 1200 mg on the fifth week of the administration cycle, followed by 1200 mg every two weeks thereafter.

In another embodiment, methods of treating a 30 kg to < 40 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 600 mg once per week for two weeks; and
(b) the anti-C5 antibody, or antigen binding fragment thereof, is administered during the maintenance phase at a dose of 900 mg on the third week of the administration cycle, followed by 900 mg every two weeks thereafter.

In another embodiment, methods of treating a 20 kg to < 30 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 600 mg once per week for two weeks; and
(b) the anti-C5 antibody, or antigen binding fragment thereof, is administered during the maintenance phase at a dose of 600 mg on the third week of the administration cycle, followed by 600 mg every two weeks thereafter.

In another embodiment, methods of treating a 10 kg to < 20 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 600 mg once per week for one week; and
(b) the anti-C5 antibody, or antigen binding fragment thereof, is administered during the maintenance phase at a dose of 300 mg on the second week of the administration cycle, followed by 300 mg every two weeks thereafter.

In another embodiment, methods of treating a 5 kg to < 10 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 300 mg once per week for one week; and
(b) the anti-C5 antibody, or antigen binding fragment thereof, is administered during the maintenance phase at a dose of 300 mg on the second week of the administration cycle, followed by 300 mg every three weeks thereafter.

In another embodiment, a method of treating an adult human patient with a MPGN is provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively,
wherein the patient has been determined to have biopsy-proven MPGN, creatinine clearance greater than 20 ml/min per 1.73 m2, and/or 24-hour proteinuria exceeding 3.5 g in adults, and
wherein the method comprises an administration cycle comprising (a) an induction phase followed by (b) a maintenance phase, wherein:
   (a) the induction phase comprises a period of four weeks, wherein the anti-C5 antibody, or antigen binding fragment thereof, is administered at a dose of 900 mg once per week; and
   (b) during the maintenance phase, the anti-C5 antibody, or antigen binding fragment thereof, is administered once at a dose of 1200 mg on the fifth week of the administration cycle, followed by 1200 mg every 14 ± 2 days thereafter.

In another embodiment, a method of treating a pediatric human patient with a MPGN is provided, the method comprising administering to the patient an anti-C5 antibody, or antigen binding fragment thereof, comprising CDR1, CDR2, and CDR3 heavy chain sequences as set forth in SEQ ID NOs:1, 2, and 3, respectively, and CDR1, CDR2, and CDR3 light chain sequences as set forth in SEQ ID NOs:4, 5, and 6, respectively,
wherein the patient has been determined to have biopsy-proven MPGN, creatinine clearance greater than 20 ml/min per 1.73 m2, and/or 24-hour proteinuria exceeding 40 mg/h/m2 in children (or exceeding 2 mg protein/mg creatinine in children spot urine samples), and
wherein the method comprises an administration cycle comprising (a) an induction phase followed by (b) a maintenance phase, wherein:
   (a) the anti-C5 antibody, or antigen binding fragment thereof, is administered during the induction phase at a dose of:
      1. 900 mg once per week for four weeks to a ≥ 40 kg patient;
      2. 600 mg once per week for two weeks to a 30 kg to < 40 kg patient;
      3. 600 mg once per week for two weeks to a 20 kg to < 30 kg patient;
      4. 600 mg once per week for one week to a 10 kg to < 20 kg patient;
      5. 300 mg once per week for one week to a 5 kg to < 10 kg patient; and
   (b) the anti-C5 antibody, or antigen binding fragment thereof, is administered during the maintenance phase at a dose of:
      1. 1200 mg on the fifth week of the administration cycle, followed by 1200 mg every two weeks thereafter, to a ≥ 40 kg patient;
      2. 900 mg on the third week of the administration cycle, followed by 900 mg every two weeks thereafter, to a 30 kg to < 40 kg patient;
      3. 600 mg on the third week of the administration cycle, followed by 600 mg every two weeks thereafter, to a 20 kg to < 30 kg patient;
      4. 300 mg on the second week of the administration cycle, followed by 300 mg every two weeks thereafter, to a 10 kg to < 20 kg patient; or
      5. 300 mg on the second week of the administration cycle, followed by 300 mg every three weeks thereafter, to a 5 kg to < 10 kg patient.

In another embodiment, 2400 mg or 3000 mg of the anti-C5 antibody, or antigen binding fragment thereof, (*e*.*g*., BNJ441) is administered to a patient weighing ≥ 40 to < 60 kg. In another embodiment, 2700 mg or 3300 mg of the anti-C5 antibody, or antigen binding fragment thereof, (*e*.*g*., BNJ441) is administered to a patient weighing ≥ 60 to < 100 kg. In another embodiment, 3000 mg or 3600 mg of the anti-C5 antibody, or antigen binding fragment thereof, (*e*.*g*., BNJ441) is administered to a patient weighing ≥ 100 kg.

In another embodiment, the anti-C5 antibody, or antigen binding fragment thereof, (*e*.*g*., BNJ441) is administered for one or more administration cycles. In one embodiment, the administration cycle is 26 weeks. In one embodiment, the anti-C5 antibody, or antigen binding fragment thereof, (*e*.*g*., BNJ441) is administered once on Day 1 of the administration cycle, once on Day 15 of the administration cycle, and every eight weeks thereafter.

In another embodiment, the anti-C5 antibody, or antigen binding fragment thereof, (e.g., BNJ441) is administered:
(a) once on Day 1 of the administration cycle at a dose of : 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 of the administration cycle and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

In another embodiment, a method of treating an adult human patient with a MPGN is provided, the method comprising administering to the patient an anti-C5 antibody, or antigen binding fragment thereof, comprising CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6, respectively,
wherein the patient has been determined to have biopsy-proven MPGN, creatinine clearance greater than 20 ml/min per 1.73 m2, and/or 24-hour proteinuria exceeding 3.5 g, and
wherein the method comprises an administration cycle and, wherein the anti-C5 antibody, or antigen binding fragment thereof, is administered:
   (a) once on Day 1 of the administration cycle at a dose of : 2400 mg to a patient weighing ≥ 40 to < 60 kg, 2700 mg to a patient weighing ≥ 60 to < 100 kg, or 3000 mg to a patient weighing ≥ 100 kg; and
   (b) on Day 15 of the administration cycle and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

In another embodiment, the anti-C5 antibody, or antigen binding fragment thereof, is administered for at least 20, 21, 22, 23, 24, 25, 26, 27, 28, 29, 30, 31, 32, 33, 34, 35, 40, 41, 42, 43, 44, 45, 46, 47, 48, 49, 50, 51, 52, 53, 54, 55, 56, 57, 58, 59, or 60 weeks. In another embodiment, the anti-C5 antibody, or antigen binding fragment thereof, is administered for at least one, two, three, four, five, or six years. In another embodiment, the anti-C5 antibody, or antigen binding fragment thereof, is administered chronically and continuously.

The anti-C5 antibodies, or antigen binding fragments thereof, can be administered to a patient by any suitable means. In one embodiment, the antibodies are formulated for intravenous administration. In one embodiment, the anti-C5 antibody, or antigen binding fragment thereof, is administered to an adult human patient by intravenous infusion over a 25 minute to 45 minute period. In another embodiment, the anti-C5 antibody, or antigen binding fragment thereof, is administered to an adult human patient by intravenous infusion over a period not to exceed two hours. In another embodiment, the anti-C5 antibody, or antigen binding fragment thereof, is administered to a pediatric human patient aged 12 years to under 18 years by intravenous infusion over a period not to exceed two hours. In another embodiment, the anti-C5 antibody, or antigen binding fragment thereof, is administered to a pediatric human patient less than 12 years old by intravenous infusion over a period not to exceed four hours.

In addition, the patient can be administered one or more suitable therapeutic agents, prior to administration of the anti-C5 antibodies, or antigen binding fragments thereof. For example, in one embodiment, the patient is administered an antimeningococcal vaccine prior to treatment with the anti-C5 antibody, or antigen binding fragment thereof. In another embodiment, the patient is administered one or more antibiotics prior to treatment with the anti-C5 antibody, or antigen binding fragment thereof.

Prior to treatment with the anti-C5 antibodies, or antigen binding fragments thereof, the patient may exhibit one or more particular characteristics, including, but not limited to, "proteinuria" (leakage of protein from the blood into the urine), "hematuria" (blood in the urine), changes in mental status (*e*.*g*., decreased alertness or decreased concentration), cloudy, dark, or foamy urine, a decrease in urine volume, decreased serum levels of complement C3 or C4, increased levels of sC5b9 (*e*.*g*., >1000 mg/ml), and/or swelling of any part of the body.

The efficacy of the treatment methods provided herein can be assessed using any suitable means. Patients treated according to the methods disclosed herein preferably experience improvement in at least one sign of MPGN. For example, the treatment may produce at least one therapeutic effect selected from the group consisting of a reduction or cessation of proteinuria and/or hematuria, complete or partial remission of MPGN, decreased swelling, improved kidney function and renal hemodynamics parameters, and/or baseline levels of C3, C4, and/or sC5b9.

In another embodiment, the treatment reduces 24 hour proteinuria at week 4 (1 month), week 8 (2 month), week 12 (3 month), week 16 (4 month), week 20 (5 months), week 24 (6 months), week 28 (7 months), week 32 (8 months), week 36 (9 months), week 40 (10 months), week 44 (11 months), or week 48 (12 months) compared to baseline. In a particular embodiment, the treatment reduces 24 hour proteinuria at week 24 (6 months) compared to baseline. In another particular embodiment, the treatment reduces 24 hour proteinuria at week 48 (12 months) compared to baseline. In one embodiment, the treatments reduces proteinuria by about 20%, 30%, 40%, 50%, 60%, 70%, 80% or more compared to no treatment.

In another embodiment, the treatment results in a complete or partial remission of MPGN. In another embodiment, the treatment produces a shift toward normal levels of urinary albumin/creatinine ratio, serum creatinine, creatinine clearance, serum total proteins, serum albumin, LDL, HDL cholesterol and triglycerides levels, hematocrit and/or haemoglobin concentration. In another embodiment, the treatment improves one or more renal functional parameters selected from the group consisting of Glomerular Filtration Rate (GFR) (*e*.*g*., as assessed by Iohexol plasma clearance measurement), Albumin, IgG, sodium, potassium fractional clearance, and renal resistivity index (*e*.*g*., as assessed by ultrasound evaluation).

Further provided are kits that include a pharmaceutical composition containing an anti-C5 antibody, or antigen binding fragment thereof, such as Eculizumab, BNJ441, or BNJ421, and a pharmaceutically-acceptable carrier, in a therapeutically effective amount adapted for use in the methods described herein.

In one embodiment, the kit comprises a dose of an anti-C5 antibody, or antigen binding fragment thereof, comprising: CDR1, CDR2, and CDR3 heavy chain sequences as set forth in SEQ ID NOs:1, 2, and 3, respectively, and CDR1, CDR2, and CDR3 light chain sequences as set forth in SEQ ID NOs: 4, 5, and 6, and; and instructions for using the anti-C5 antibody, or antigen binding fragment thereof, in any of the methods described herein.

In another embodiment, the kit comprises a dose of an anti-C5 antibody, or antigen binding fragment thereof, comprising CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6; and instructions for using the anti-C5 antibody, or antigen binding fragment thereof, in any of the methods described herein.

Also provided are anti-C5 antibodies, or antigen binding fragments thereof, for administration according to a particular clinical dosage regimen (*i.e*., at a particular dose amount and according to a specific dosing schedule).

### BRIEF DESCRIPTION OF THE DRAWINGS

**Figure 1** is a flow chart depicting the study visit schedule.
**Figure 2** is a flow chart depicting patient enrollment.
**Figures 3A and 3B** depict sC5b9 levels **(****Figure 3A****)** and24-hour urinary protein excretion **(****Figure 3B****)** over the course of treatment (1 year), recovery (3 months), and the extension phase (1 year).
**Figures 4A and 4B** depict serum 24-hour urinary albumin excretion levels **(****Figure 4A****)** and glomerular filtration rate **(****Figure 4B****)** over the course of treatment (1 year), recovery (3 months), and the extension phase (1 year).
**Figures 5A and 5B** depict sC5b9 levels **(****Figure 5A****)** and 24-hour urinary protein excretion **(****Figure 5B****)** over the course of treatment (1 year), recovery (3 months), and the extension phase (1 year) for the apparent "non-responder".
**Figures 6A and 6B** depict serum 24-hour urinary albumin excretion levels **(****Figure 6A****)** and glomerular filtration rate **(****Figure 6B****)** over the course of treatment (1 year), recovery (3 months), and the extension phase (1 year) for the apparent "non-responder".
**Figure 7** sets forth the changes in clinical and laboratory parameters during the two treatment periods with eculizumab (week 0a to week 48a and week 0b to week 48b) and the washout period (week 48a to week 0b), as compared to baseline.
**Figures 8A-8D** depict the histology of the apparent "non-responder" at baseline **(****Figures 8A and 8B****)** and 2 years **(****Figures 8C and 8D****).**
**Figure 9** depicts the estimated (eGFR) and measured glomerular filtration rates (mGFR) over the course of treatment (1 year), recovery (3 months), and the extension phase (1 year) for eculizumab treated patients.
**Figures 10A-10D** are representative pre- and post-treatment renal biopsy findings from two patients diagnosed with IC-MPGN.

### DETAILED DESCRIPTION

### I. Definitions

As used herein, the term "subject" or "patient" is a human patient *(e.g.,* a pediatric or adult patient having Membranoproliferative Glomerulonephritis).

As used herein, the term "glomerulonephritis" refers to inflammation of the glomeruli (structures of the kidney, which help filter wastes and fluids from the blood to form urine).

As used herein, "Membranoproliferative Glomerulonephritis" (also known as "MPGN", "mesangiocapillary glomerulonephritis" or "MCGN") is a form of glomerulonephritis caused by an abnormal immune response. Deposits of antibodies build up in a part of the kidneys called the glomerular basement membrane (which helps filter wastes and extra fluid from the blood). Damage to this membrane affects the kidney's ability to create urine normally. It may allow blood and protein to leak into the urine. If enough protein leaks into the urine, fluid may leak out of the blood vessels into body tissues, leading to edema (e.g., swelling). Nitrogen waste products may also build up in the blood (also known as "azotemia"). Symptoms include, but are not limited to, "proteinuria" (leakage of protein from the blood into the urine), "hematuria" (blood in the urine), changes in mental status (*e*.*g*., decreased alertness or decreased concentration), cloudy, dark, or foamy urine, a decrease in urine volume, decreased serum levels of complement C3 or C4, increased levels of sC5b9 *(e.g.,* >1000 mg/ml), and/or swelling of any part of the body).

MPGN is an uncommon cause of chronic proteinuric nephropathy (the incidence is ~5 per million persons per year) that may be primary or secondary to hepatitis C virus and other infections, autoimmune diseases, and malignancies (see, Marina Noris, et al., American Journal of Kidney Diseases, Volume 66, Issue 2, August 2015, Pages 359-375). The onset of MPGN can occur from childhood to late adulthood. The clinical presentation is variable and ranges from asymptomatic hematuria and proteinuria to acute nephritic syndrome, nephrotic syndrome, chronic kidney disease, or even rapidly progressing glomerulonephritis resulting in end-stage renal disease.

MPGN is characterized by mesangial hypercellularity and matrix expansion, with thickening of glomerular capillaries (with the formation of double contours), interposition of leukocytes and mesangial cells, and synthesis of new GBM resulting in lobular accentuation of glomerular tufts (see, Marina Noris, et al., American Journal of Kidney Diseases, Volume 66, Issue 2, August 2015, Pages 359-375). This pattern of injury results from deposition of immune complexes and/or complement factors in the glomerular mesangium and along the glomerular capillary walls and is easily recognized by light and immunofluorescence microscopy.

Traditionally, on the basis of electron microscopy findings, MPGN was classified as type I, with subendothelial electron-dense deposits; type II (also called "dense deposit disease" or "DDD"), with intramembranous highly electron-dense deposits; and type III, with both subendothelial and subepithelial deposits. However, these categories had limited prognostic value because of their complexity and the occurrence of features suggestive of more than 1 type in the same biopsy samples.

A new classification based on the pathogenesis and composition of glomerular deposits as analyzed by immunofluorescence microscopy has been proposed. MPGN associated with substantial immunoglobulin deposits has been termed "immune-complex-mediated MPGN" ("IC-mediated MPGN") and is commonly associated with chronic infections or autoimmune diseases (see Marina Noris, et al., American Journal of Kidney Diseases, Volume 66, Issue 2, August 2015, Pages 359-375). In these secondary forms, careful characterization of deposits can often help identify the underlying cause. Deposits of IgM, IgG, C3, and κ and λ light chains are typically found in MPGN associated with hepatitis C virus infection. Multiple immunoglobulins and complement proteins (IgG, IgM, IgA, C1q, C3, and κ and λ light chains) are also observed in MPGN associated with autoimmune diseases. However, monotypic immunoglobulin with κ and λ light chain restriction is observed in MPGN associated with monoclonal gammopathy. Immune-complex-mediated MPGN has been considered to include most cases of MPGN types I and III according to the older classification, with electron microscopy typically revealing mesangial and subendothelial deposits and, in some cases, intramembranous and subepithelial deposits.

MPGN cases that present clear glomerular C3 staining with absent or scanty immunoglobulins are considered "complement-mediated MPGN" and are referred to as a "C3 Glomerulopathy" (also known as "C3G"). C3G is less common than immune-complex-mediated MPGN, has a prevalence of about 1-2 cases per million inhabitants, and is further divided on the basis of the quality of the deposits seen in glomeruli on electron microscopy. DDD is diagnosed in cases with distinctive highly electron-dense osmiophilic deposits that are typically found within the glomerular basement membrane (GBM). These deposits extend along the central part of the GBM, but may also involve the subendothelial and subepithelial region. Globular deposits appear in the mesangium and in about half the patients with DDD, are also seen in Bowman capsule and the tubular basement membrane.

Cases of C3G that lack the electron-dense deposits of DDD are called "C3 glomerulonephritis". In C3 glomerulonephritis, deposits have subendothelial and sometimes subepithelial and intramembranous localization, morphologic characteristics likely resembling MPGN types I and III. Thus, with the exception of DDD, electron microscopy cannot distinguish between immune-complex-mediated MPGN and C3G.

However, immunofluorescence microscopy cannot always confirm a diagnosis because immunoglobulins may be present in glomeruli of patients with C3G. Small amounts of immunoglobulin may become trapped in areas of sclerosis or in podocytes of patients with proteinuric glomerular diseases, and about one-third of patients with DDD have glomerular IgG staining. Thus, a subsequent broader classification of C3G proposed by a recent consensus report suggested including all cases that have dominant immunofluorescence microscopy staining of C3 of 2 or more orders of magnitude greater than any other immune reactants (using a scale of 0-3). Even with this broader classification, a substantial proportion (20%) of patients with DDD would not be classified as having C3G. Conversely, isolated staining for C3 on immunofluorescence microscopy may be observed in cases of postinfectious glomerulonephritis. Therefore, the issue of MPGN classification has not yet been fully resolved (see, Marina Noris, et al., American Journal of Kidney Diseases, Volume 66, Issue 2, August 2015, Pages 359-375).

The high variability of clinical presentation and course is likely caused by differences in the pathogenesis of the disease, the histologic lesion in the kidney, and the timing of the diagnosis (that is based on kidney biopsy) relative to the clinical course.

### II. Anti-C5 Antibodies

The anti-C5 antibodies described herein bind to complement component C5 (*e.g*., human C5) and inhibit the cleavage of C5 into fragments C5a and C5b. Anti-C5 antibodies (or VH/VL domains derived therefrom) suitable for use in the invention can be generated using methods well known in the art. Alternatively, art recognized anti-C5 antibodies can be used. Antibodies that compete with any of these art-recognized antibodies for binding to C5 also can be used.

The term "antibody" describes polypeptides comprising at least one antibody derived antigen binding site (*e*.*g*., VH/VL region or Fv, or CDR). Antibodies include known forms of antibodies. For example, the antibody can be a human antibody, a humanized antibody, a bispecific antibody, or a chimeric antibody. The antibody also can be a Fab, Fab'2, ScFv, SMIP, Affibody^{®}, nanobody, or a domain antibody. The antibody also can be of any of the following isotypes: IgG1, IgG2, IgG3, IgG4, IgM, IgA1, IgA2, IgAsec, IgD, and IgE. The antibody may be a naturally occurring antibody or may be an antibody that has been altered *(e.g.,* by mutation, deletion, substitution, conjugation to a non-antibody moiety). For example, an antibody may include one or more variant amino acids (compared to a naturally occurring antibody) which changes a property *(e.g.,* a functional property) of the antibody. For example, numerous such alterations are known in the art which affect, *e.g.,* half-life, effector function, and/or immune responses to the antibody in a patient. The term antibody also includes artificial polypeptide constructs which comprise at least one antibody-derived antigen binding site.

An exemplary anti-C5 antibody is Eculizumab comprising heavy and light chains having the sequences shown in SEQ ID NOs: 10 and 11, respectively, or antigen binding fragments and variants thereof. Eculizumab (also known as Soliris^{®}) is described in US Patent No: 6,355,245, the teachings or which are hereby incorporated by reference. Eculizumab is a humanized monoclonal antibody that is a terminal complement inhibitor.

In other embodiments, the antibody comprises the heavy and light chain CDRs or variable regions of Eculizumab. Accordingly, in one embodiment, the antibody comprises the CDR1, CDR2, and CDR3 domains of the VH region of Eculizumab having the sequence set forth in SEQ ID NO: 7, and the CDR1, CDR2 and CDR3 domains of the VL region of Eculizumab having the sequence set forth in SEQ ID NO: 8. In another embodiment, the antibody comprises heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. In another embodiment, the antibody comprises VH and VL regions having the amino acid sequences set forth in SEQ ID NO: 7 and SEQ ID NO: 8, respectively.

Another exemplary anti-C5 antibody is antibody BNJ441 comprising heavy and light chains having the sequences shown in SEQ ID NOs:14 and 11, respectively, or antigen binding fragments and variants thereof. BNJ441 (also known as ALXN1210) is described in PCT/US2015/019225 and US Patent No. 9,079,949, the teachings or which are hereby incorporated by reference. BNJ441 is a humanized monoclonal antibody that is structurally related to Eculizumab (Soliris^{®}). BNJ441 selectively binds to human complement protein C5, inhibiting its cleavage to C5a and C5b during complement activation. This inhibition prevents the release of the proinflammatory mediator C5a and the formation of the cytolytic pore-forming membrane attack complex C5b-9 while preserving the proximal or early components of complement activation (*e*.*g*., C3 and C3b) essential for the opsonization of microorganisms and clearance of immune complexes.

In other embodiments, the antibody comprises the heavy and light chain CDRs or variable regions of BNJ441. Accordingly, in one embodiment, the antibody comprises the CDR1, CDR2, and CDR3 domains of the VH region of BNJ441 having the sequence set forth in SEQ ID NO:12, and the CDR1, CDR2 and CDR3 domains of the VL region of BNJ441 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO:12 and SEQ ID NO:8, respectively.

Another exemplary anti-C5 antibody is antibody BNJ421 comprising heavy and light chains having the sequences shown in SEQ ID NOs:20 and 11, respectively, or antigen binding fragments and variants thereof. BNJ421 (also known as ALXN1211) is described in PCT/US2015/019225 and US Patent No.:9,079,949, the teachings or which are hereby incorporated by reference.

In other embodiments, the antibody comprises the heavy and light chain CDRs or variable regions of BNJ421. Accordingly, in one embodiment, the antibody comprises the CDR1, CDR2, and CDR3 domains of the VH region of BNJ421 having the sequence set forth in SEQ ID NO:12, and the CDR1, CDR2 and CDR3 domains of the VL region of BNJ421 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO:12 and SEQ ID NO:8, respectively.

The exact boundaries of CDRs have been defined differently according to different methods. In some embodiments, the positions of the CDRs or framework regions within a light or heavy chain variable domain can be as defined by Kabat et al. [(1991) "Sequences of Proteins of Immunological Interest." NIH Publication No. 91-3242, U.S. Department of Health and Human Services, Bethesda, MD]. In such cases, the CDRs can be referred to as "Kabat CDRs" (*e*.*g*., "Kabat LCDR2" or "Kabat HCDR1"). In some embodiments, the positions of the CDRs of a light or heavy chain variable region can be as defined by Chothia et al. (1989) Nature 342:877-883. Accordingly, these regions can be referred to as "Chothia CDRs" (e.g., "Chothia LCDR2" or "Chothia HCDR3"). In some embodiments, the positions of the CDRs of the light and heavy chain variable regions can be as defined by a Kabat-Chothia combined definition. In such embodiments, these regions can be referred to as "combined Kabat-Chothia CDRs". Thomas et al. [(1996) Mol Immunol 33(17/18):1389-1401] exemplifies the identification of CDR boundaries according to Kabat and Chothia definitions.

Methods for determining whether an antibody binds to a protein antigen and/or the affinity for an antibody to a protein antigen are known in the art. For example, the binding of an antibody to a protein antigen can be detected and/or quantified using a variety of techniques such as, but not limited to, Western blot, dot blot, surface plasmon resonance (SPR) method (*e*.*g*., BIAcore system; Pharmacia Biosensor AB, Uppsala, Sweden and Piscataway, N.J.), or enzyme-linked immunosorbent assay (ELISA). See, *e.g.,* Benny K. C. Lo (2004) "Antibody Engineering: Methods and Protocols," Humana Press (ISBN: 1588290921); Johne et al. (1993) J Immunol Meth 160:191-198; Jonsson et al. (1993) Ann Biol Clin 51:19-26; and Jonsson et al. (1991) Biotechniques 11:620-627.

In one embodiment, the antibody competes for binding with, and/or binds to the same epitope on C5 as, the antibodies described herein. The term "binds to the same epitope" with reference to two or more antibodies means that the antibodies bind to the same segment of amino acid residues, as determined by a given method. Techniques for determining whether antibodies bind to the "same epitope on C5" with the antibodies described herein include, for example, epitope mapping methods, such as, x-ray analyses of crystals of antigen:antibody complexes which provides atomic resolution of the epitope and hydrogen/deuterium exchange mass spectrometry (HDX-MS). Other methods monitor the binding of the antibody to peptide antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same VH and VL or the same CDR1, 2 and 3 sequences are expected to bind to the same epitope.

Antibodies that "compete with another antibody for binding to a target" refer to antibodies that inhibit (partially or completely) the binding of the other antibody to the target. Whether two antibodies compete with each other for binding to a target, *i.e.,* whether and to what extent one antibody inhibits the binding of the other antibody to a target, may be determined using known competition experiments. In certain embodiments, an antibody competes with, and inhibits binding of another antibody to a target by at least 10%, 20%, 30%, 40%, 50%, 60%, 70%, 80%, 90% or 100%. The level of inhibition or competition may be different depending on which antibody is the "blocking antibody" (i.e., the cold antibody that is incubated first with the target). Competing antibodies bind to the same epitope, an overlapping epitope or to adjacent epitopes (*e*.*g*., as evidenced by steric hindrance).

Anti-C5 antibodies, or antigen-binding fragments thereof described herein, used in the methods described herein can be generated using a variety of art-recognized techniques. Monoclonal antibodies may be obtained by various techniques familiar to those skilled in the art. Briefly, spleen cells from an animal immunized with a desired antigen are immortalized, commonly by fusion with a myeloma cell (see, Kohler & Milstein, Eur. J. Immunol. 6: 511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according to the general protocol outlined by Huse, et al., Science 246: 1275-1281 (1989).

### III. Compositions

Also, provided herein are compositions comprising an anti-C5 antibody, or antigen binding fragment thereof. In one embodiment, the composition comprises an antibody comprising the CDR1, CDR2, and CDR3 domains of the VH region of Eculizumab having the sequence set forth in SEQ ID NO: 7, and the CDR1, CDR2 and CDR3 domains of the VL region of Eculizumab having the sequence set forth in SEQ ID NO: 8. In another embodiment, the antibody comprises heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. In another embodiment, the antibody comprises VH and VL regions having the amino acid sequences set forth in SEQ ID NO: 7 and SEQ ID NO: 8, respectively.

In another embodiment, the antibody comprises the heavy and light chain CDRs or variable regions of BNJ441. In another embodiment, the antibody comprises the CDR1, CDR2, and CDR3 domains of the VH region of BNJ441 having the sequence set forth in SEQ ID NO:12, and the CDR1, CDR2 and CDR3 domains of the VL region of BNJ441 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO:12 and SEQ ID NO:8, respectively.

In another embodiment, the antibody comprises the CDR1, CDR2, and CDR3 domains of the VH region of BNJ421 having the sequence set forth in SEQ ID NO:12, and the CDR1, CDR2 and CDR3 domains of the VL region of BNJ421 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO:12 and SEQ ID NO:8, respectively.

The compositions can be formulated as a pharmaceutical solution, *e*.*g*., for administration to a subject for the treatment or prevention of a complement-associated disorder. The pharmaceutical compositions will generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, *e.g.,* an acid addition salt or a base addition salt, sugars, carbohydrates, polyols and/or tonicity modifiers.

The compositions can be formulated according to standard methods. Pharmaceutical formulation is a well-established art, and is further described in, *e.g.,* Gennaro (2000) "Remington: The Science and Practice of Pharmacy," 20th Edition, Lippincott, Williams & Wilkins (ISBN: 0683306472); Ansel et al. (1999) "Pharmaceutical Dosage Forms and Drug Delivery Systems," 7th Edition, Lippincott Williams & Wilkins Publishers (ISBN: 0683305727); and Kibbe (2000) "Handbook of Pharmaceutical Excipients American Pharmaceutical Association," 3rd Edition (ISBN: 091733096X). In some embodiments, a composition can be formulated, for example, as a buffered solution at a suitable concentration and suitable for storage at 2-8°C (*e.g.,* 4°C). In some embodiments, a composition can be formulated for storage at a temperature below 0°C (*e.g.,* -20°C or -80°C). In some embodiments, the composition can be formulated for storage for up to 2 years (*e.g.,* one month, two months, three months, four months, five months, six months, seven months, eight months, nine months, 10 months, 11 months, 1 year, 1½ years, or 2 years) at 2-8°C (*e.g.,* 4°C). Thus, in some embodiments, the compositions described herein are stable in storage for at least 1 year at 2-8°C (*e.g.,* 4°C).

The pharmaceutical compositions can be in a variety of forms. These forms include, *e.g.,* liquid, semi-solid and solid dosage forms, such as liquid solutions (*e.g.,* injectable and infusible solutions), dispersions or suspensions, tablets, pills, powders, liposomes and suppositories. The preferred form depends, in part, on the intended mode of administration and therapeutic application. For example, compositions containing a composition intended for systemic or local delivery can be in the form of injectable or infusible solutions. Accordingly, the compositions can be formulated for administration by a parenteral mode (*e*.*g*., intravenous, subcutaneous, intraperitoneal, or intramuscular injection). "Parenteral administration," "administered parenterally," and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid and intrasternal injection and infusion.

### IV. Methods of Treatment

Provided herein are methods for treating MPGN in human patients (*e*.*g*., adult and pediatric human patients), comprising administering to the patient an anti-C5 antibody, or antigen binding fragment thereof according to a particular clinical dosage regimen (*i.e*., at a particular dose amount and according to a specific dosing schedule). In one embodiment, the MPGN is "immune-complex-mediated MPGN" (IC-mediated MPGN). In another embodiment, the MPGN is "complement-mediated MPGN" (*e*.*g*., a "C3 Glomerulopathy" (also known as "C3G"). In one embodiment, the C3 Glomerulopathy is dense deposit disease (DDD) or C3 glomerulonephritis.

The terms "treat," "treating," and "treatment," as used herein, refer to therapeutic measures described herein. The methods of treatment employ administration to a human the combination disclosed herein in order to cure, delay, reduce the severity of, or ameliorate one or more symptoms of MPGN or in order to prolong the survival of a subject beyond that expected in the absence of such treatment.

As used herein, "effective treatment" refers to treatment producing a beneficial effect, *e*.*g*., amelioration of at least one symptom of a disease or disorder. A beneficial effect can take the form of an improvement over baseline, *i.e*., an improvement over a measurement or observation made prior to initiation of therapy according to the method. Effective treatment may refer to alleviation of at least one symptom of MPGN (*e*.*g*., proteinuria, hematuria, changes in mental status (*e*.*g*., decreased alertness or decreased concentration), cloudy, dark, or foamy urine, a decrease in urine volume, decreased serum levels of complement C3 or C4, increased levels of sC5b9, and/or swelling of any part of the body). For example, the treatment may produce at least one therapeutic effect selected from the group consisting of a reduction or cessation of proteinuria and/or hematuria, complete or partial remission of MPGN, decreased swelling, improved kidney function and renal hemodynamics parameters, and/or baseline levels of C3, C4, and/or sC5b9.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In one example, an "effective amount" is the amount of anti-C5 antibody, or antigen binding fragment thereof, clinically proven to alleviate at least one symptom of MPGN. An effective amount can be administered in one or more administrations.

In one embodiment, the patient is an adult patient who has been determined to have biopsy-proven MPGN, creatinine clearance greater than 20 ml/min per 1.73 m2, and/or 24-hour proteinuria exceeding 3.5 g. In another embodiment, the patient is a pediatric patient who has been determined to have biopsy-proven MPGN, creatinine clearance greater than 20 ml/min per 1.73 m2, and/or 24-hour proteinuria exceeding 40 mg/h/m2 (or exceeding 2 mg protein/mg creatinine in spot urine samples). In a further embodiment, the patient (*e*.*g*., pediatric or adult patient) has also been determined to have persistently low C3 levels in at least two consecutive evaluations and/or persistently high sC5b9 levels (>1000 ng/ml) in at least two previous consecutive evaluations.

Prior to treatment with the anti-C5 antibodies, or antigen binding fragments thereof, the patient may exhibit one or more particular characteristics, including, but not limited to, "proteinuria" (leakage of protein from the blood into the urine), "hematuria" (blood in the urine), changes in mental status (*e*.*g*., decreased alertness or decreased concentration), cloudy, dark, or foamy urine, a decrease in urine volume, decreased serum levels of complement C3 or C4, increased levels of sC5b9 (*e*.*g*., >1000 mg/ml), and/or swelling of any part of the body.

In one aspect, methods of treating a human patient with MPGN are provided, the methods comprising administering to the patient an effective amount of an anti-C5 antibody, or antigen binding fragment thereof. In one embodiment, the dose of the anti-C5 antibody, or antigen binding fragment thereof, is a flat-fixed dose. For example, the anti-C5 antibody, or antigen binding fragment thereof, may be administered at a fixed dose of 300 mg, 600 mg, 900 mg or 1,200 mg. In certain embodiments, dosage regimens are adjusted to provide the optimum desired response (*e*.*g*., an effective response).

In one embodiment, the anti-C5 antibody, or antigen binding fragment thereof, (*e*.*g*., eculizumab) is administered to an adult patient (a) weekly at a dose of 900 mg for four weeks and (b) once every 14 + 2 days (*e*.*g*., about two weeks) thereafter at a dose of 1,200 mg. In another embodiment, the anti-C5 antibody, or antigen binding fragment thereof, (*e*.*g*., eculizumab) is administered to an adult patient according to an administration cycle comprising (a) an induction phase followed by (b) a maintenance phase, wherein: (a) the induction phase comprises a period of four weeks, wherein the anti-C5 antibody, or antigen binding fragment thereof, is administered at a dose of 900 mg once per week; and (b) during the maintenance phase, the anti-C5 antibody, or antigen binding fragment thereof, is administered once at a dose of 1200 mg on the fifth week of the administration cycle, followed by 1200 mg every 14 ± 2 days thereafter.

In another embodiment, the anti-C5 antibody, or antigen binding fragment thereof, (*e*.*g*., eculizumab) is administered to a pediatric patient according to an administration cycle, wherein the administration cycle comprises (a) an induction phase followed by (b) a maintenance phase, wherein:
(a) the anti-C5 antibody, or antigen binding fragment thereof, is administered during the induction phase at a dose of:
   1. 900 mg once per week for four weeks to a ≥ 40 kg patient;
   2. 600 mg once per week for two weeks to a 30 kg to < 40 kg patient;
   3. 600 mg once per week for two weeks to a 20 kg to < 30 kg patient;
   4. 600 mg once per week for one week to a 10 kg to < 20 kg patient;
   5. 300 mg once per week for one week to a 5 kg to < 10 kg patient; and
(b) the anti-C5 antibody, or antigen binding fragment thereof, is administered during the maintenance phase at a dose of:
   1. 1200 mg on the fifth week of the administration cycle, followed by 1200 mg every two weeks thereafter, to a ≥ 40 kg patient;
   2. 900 mg on the third week of the administration cycle, followed by 900 mg every two weeks thereafter, to a 30 kg to < 40 kg patient;
   3. 600 mg on the third week of the administration cycle, followed by 600 mg every two weeks thereafter, to a 20 kg to < 30 kg patient;
   4. 300 mg on the second week of the administration cycle, followed by 300 mg every two weeks thereafter, to a 10 kg to < 20 kg patient; or
   5. 300 mg on the second week of the administration cycle, followed by 300 mg every three weeks thereafter, to a 5 kg to < 10 kg patient.

In another embodiment, methods of treating a pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein the anti-C5 antibody, or antigen binding fragment thereof, is administered at a dose of:
a) 900 mg once per week for four weeks during the induction phase, 1200 mg on the fifth week of the administration cycle, followed by 1200 mg every two weeks thereafter during the maintenance phase, to a ≥ 40 kg patient;
b) 600 mg once per week for two weeks during the induction phase, 900 mg on the third week of the administration cycle, followed by 900 mg every two weeks thereafter during the maintenance phase, to a 30 kg to < 40 kg patient;
c) 600 mg once per week for two weeks during the induction phase, 600 mg on the third week of the administration cycle, followed by 600 mg every two weeks thereafter during the maintenance phase, to a 20 kg to < 30 kg patient;
d) 600 mg once per week for one week during the induction phase, 300 mg on the second week of the administration cycle, followed by 300 mg every two weeks thereafter during the maintenance phase, to a 10 kg to < 20 kg patient;
e) 300 mg once per week for one week during the induction phase, 300 mg on the second week of the administration cycle, followed by 300 mg every three weeks thereafter during the maintenance phase, to a 5 kg to < 10 kg patient.

In another embodiment, methods of treating a ≥ 40 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 900 mg once per week for four weeks; and
(b) the anti-C5 antibody, or antigen binding fragment thereof, is administered during the maintenance phase at a dose of 1200 mg on the fifth week of the administration cycle, followed by 1200 mg every two weeks thereafter.

In another embodiment, methods of treating a 30 kg to < 40 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 600 mg once per week for two weeks; and
(b) the anti-C5 antibody, or antigen binding fragment thereof, is administered during the maintenance phase at a dose of 900 mg on the third week of the administration cycle, followed by 900 mg every two weeks thereafter.

In another embodiment, methods of treating a 20 kg to < 30 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 600 mg once per week for two weeks; and
(b) the anti-C5 antibody, or antigen binding fragment thereof, is administered during the maintenance phase at a dose of 600 mg on the third week of the administration cycle, followed by 600 mg every two weeks thereafter.

In another embodiment, methods of treating a 10 kg to < 20 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 600 mg once per week for one week; and
(b) the anti-C5 antibody, or antigen binding fragment thereof, is administered during the maintenance phase at a dose of 300 mg on the second week of the administration cycle, followed by 300 mg every two weeks thereafter.

In another embodiment, methods of treating a 5 kg to < 10 kg pediatric human patient with MPGN are provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively, wherein the method comprises an administration cycle comprising (a) an induction phase followed by (b) a maintenance phase, wherein:
(a) the anti-C5 antibody, or antigen binding fragment thereof, is administered during the induction phase at a dose of 300 mg once per week for one week; and
(b) the anti-C5 antibody, or antigen binding fragment thereof, is administered during the maintenance phase at a dose of 300 mg on the second week of the administration cycle, followed by 300 mg every three weeks thereafter.

In another embodiment, a method of treating an adult human patient with a MPGN is provided, the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively,
wherein the patient has been determined to have biopsy-proven MPGN, creatinine clearance greater than 20 ml/min per 1.73 m2, and/or 24-hour proteinuria exceeding 3.5 g in adults, and
wherein the method comprises an administration cycle comprising (a) an induction phase followed by (b) a maintenance phase, wherein:
   (a) the induction phase comprises a period of four weeks, wherein the anti-C5 antibody, or antigen binding fragment thereof, is administered at a dose of 900 mg once per week; and
   (b) during the maintenance phase, the anti-C5 antibody, or antigen binding fragment thereof, is administered once at a dose of 1200 mg on the fifth week of the administration cycle, followed by 1200 mg every 14 ± 2 days thereafter.

In another embodiment, a method of treating a pediatric human patient with a MPGN is provided, the method comprising administering to the patient an anti-C5 antibody, or antigen binding fragment thereof, comprising CDR1, CDR2, and CDR3 heavy chain sequences as set forth in SEQ ID NOs:1, 2, and 3, respectively, and CDR1, CDR2, and CDR3 light chain sequences as set forth in SEQ ID NOs:4, 5, and 6, respectively,
wherein the patient has been determined to have biopsy-proven MPGN, creatinine clearance greater than 20 ml/min per 1.73 m2, and/or 24-hour proteinuria exceeding 40 mg/h/m2 in children (or exceeding 2 mg protein/mg creatinine in children spot urine samples), and
wherein the method comprises an administration cycle comprising (a) an induction phase followed by (b) a maintenance phase, wherein:
   (a) the anti-C5 antibody, or antigen binding fragment thereof, is administered during the induction phase at a dose of:
      1. 900 mg once per week for four weeks to a ≥ 40 kg patient;
      2. 600 mg once per week for two weeks to a 30 kg to < 40 kg patient;
      3. 600 mg once per week for two weeks to a 20 kg to < 30 kg patient;
      4. 600 mg once per week for one week to a 10 kg to < 20 kg patient;
      5. 300 mg once per week for one week to a 5 kg to < 10 kg patient; and
   (b) the anti-C5 antibody, or antigen binding fragment thereof, is administered during the maintenance phase at a dose of:
      1. 1200 mg on the fifth week of the administration cycle, followed by 1200 mg every two weeks thereafter, to a ≥ 40 kg patient;
      2. 900 mg on the third week of the administration cycle, followed by 900 mg every two weeks thereafter, to a 30 kg to < 40 kg patient;
      3. 600 mg on the third week of the administration cycle, followed by 600 mg every two weeks thereafter, to a 20 kg to < 30 kg patient;
      4. 300 mg on the second week of the administration cycle, followed by 300 mg every two weeks thereafter, to a 10 kg to < 20 kg patient; or
      5. 300 mg on the second week of the administration cycle, followed by 300 mg every three weeks thereafter, to a 5 kg to < 10 kg patient.

In another embodiment, 2400 mg or 3000 mg of the anti-C5 antibody, or antigen binding fragment thereof, (*e*.*g*., BNJ441) is administered to a patient weighing ≥ 40 to < 60 kg. In another embodiment, 2700 mg or 3300 mg of the anti-C5 antibody, or antigen binding fragment thereof, (*e.g.,* BNJ441) is administered to a patient weighing ≥ 60 to < 100 kg. In another embodiment, 3000 mg or 3600 mg of the anti-C5 antibody, or antigen binding fragment thereof, (*e.g.,* BNJ441) is administered to a patient weighing ≥ 100 kg.

In another embodiment, the anti-C5 antibody, or antigen binding fragment thereof, (*e.g.,* BNJ441) is administered for one or more administration cycles. In one embodiment, the administration cycle is 26 weeks. In one embodiment, the anti-C5 antibody, or antigen binding fragment thereof, (*e*.*g*., BNJ441) is administered once on Day 1 of the administration cycle, once on Day 15 of the administration cycle, and every eight weeks thereafter.

In another embodiment, the anti-C5 antibody, or antigen binding fragment thereof, (*e*.*g*., BNJ441) is administered:
(a) once on Day 1 of the administration cycle at a dose of : 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 of the administration cycle and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

In another embodiment, a method of treating an adult human patient with a MPGN is provided, the method comprising administering to the patient an anti-C5 antibody, or antigen binding fragment thereof, comprising CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6, respectively,
wherein the patient has been determined to have biopsy-proven MPGN, creatinine clearance greater than 20 ml/min per 1.73 m2, and/or 24-hour proteinuria exceeding 3.5 g, and
wherein the method comprises an administration cycle and, wherein the anti-C5 antibody, or antigen binding fragment thereof, is administered:
   (a) once on Day 1 of the administration cycle at a dose of : 2400 mg to a patient weighing ≥ 40 to < 60 kg, 2700 mg to a patient weighing ≥ 60 to < 100 kg, or 3000 mg to a patient weighing ≥ 100 kg; and
   (b) on Day 15 of the administration cycle and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

In another embodiment, the anti-C5 antibody, or antigen binding fragment thereof, is administered for at least 20, 21, 22, 23, 24, 25, 26, 27, 28, 29, 30, 31, 32, 33, 34, 35, 40, 41, 42, 43, 44, 45, 46, 47, 48, 49, 50, 51, 52, 53, 54, 55, 56, 57, 58, 59, or 60 weeks. In another embodiment, the anti-C5 antibody, or antigen binding fragment thereof, is administered for at least one, two, three, four, five, or six years.

The anti-C5 antibodies, or antigen binding fragments thereof, can be administered to a patient by any suitable means. In one embodiment, the antibodies are formulated for intravenous administration. In one embodiment, the anti-C5 antibody, or antigen binding fragment thereof, is administered to an adult human patient by intravenous infusion over a 25 minute to 45 minute period. In another embodiment, the anti-C5 antibody, or antigen binding fragment thereof, is administered to an adult human patient by intravenous infusion over a period not to exceed two hours. In another embodiment, the anti-C5 antibody, or antigen binding fragment thereof, is administered to a pediatric human patient aged 12 years to under 18 years by intravenous infusion over a period not to exceed two hours. In another embodiment, the anti-C5 antibody, or antigen binding fragment thereof, is administered to a pediatric human patient less than 12 years old by intravenous infusion over a period not to exceed four hours.

In addition, the patient can be administered one or more suitable therapeutic agents, prior to administration of the anti-C5 antibodies, or antigen binding fragments thereof. For example, in one embodiment, the patient is administered an antimeningococcal vaccine prior to treatment with the anti-C5 antibody, or antigen binding fragment thereof. In another embodiment, the patient is administered one or more antibiotics prior to treatment with the anti-C5 antibody, or antigen binding fragment thereof.

### V. Outcomes

Provided herein are methods for treating MPGN in a human patient comprising administering to the patient an anti-C5 antibody, or antigen binding fragment thereof. Symptoms of MPGN include, but are not limited to, proteinuria, hematuria, changes in mental status (*e.g*., decreased alertness or decreased concentration), cloudy, dark, or foamy urine, a decrease in urine volume, decreased serum levels of complement C3 or C4, increased levels of sC5b9 (*e*.*g*., >1000 mg/ml), and/or swelling of any part of the body.

Patients (*e*.*g*., adult patients) treated according to the methods disclosed have been determined to have biopsy-proven MPGN, creatinine clearance greater than 20 ml/min per 1.73 m2, and/or 24-hour proteinuria exceeding 3.5 g. Pediatric patients treated according to the methods disclosed herein have been determined to have biopsy-proven MPGN, creatinine clearance greater than 20 ml/min per 1.73 m2, and/or 24-hour proteinuria exceeding 40 mg/h/m2 (or exceeding 2 mg protein/mg creatinine in spot urine samples). In one embodiment, the patient (*e*.*g*., pediatric or adult patient) has also been determined to have persistently low C3 levels in at least two consecutive evaluations and/or persistently high sC5b9 levels (>1000 ng/ml) in at least two previous consecutive evaluations.

Patients treated according to the methods disclosed herein preferably experience improvement in at least one sign of MPGN. For example, the treatment may produce at least one therapeutic effect selected from the group consisting of a reduction or cessation of proteinuria and/or hematuria, complete or partial remission of MPGN, decreased swelling, improved kidney function and renal hemodynamics parameters, and/or baseline levels of C3, C4, and/or sC5b9.

In one embodiment, the treatment reduces 24 hour proteinuria at week 4 (1 month), week 8 (2 month), week 12 (3 month), week 16 (4 month), week 20 (5 months), week 24 (6 months), week 28 (7 months), week 32 (8 months), week 36 (9 months), week 40 (10 months), week 44 (11 months), or week 48 (12 months) compared to baseline. In a particular embodiment, the treatment reduces 24 hour proteinuria at week 24 (6 months) compared to baseline. In another particular embodiment, the treatment reduces 24 hour proteinuria at week 48 (12 months) compared to baseline. In another embodiment, the treatments reduces proteinuria by about 20%, 30%, 40%, 50%, 60%, 70%, 80% or more compared to no treatment.

In another embodiment, the treatment results in a complete or partial remission of MPGN. In another embodiment, the treatment produces a shift toward normal levels of urinary albumin/creatinine ratio, serum creatinine, creatinine clearance, serum total proteins, serum albumin, LDL, HDL cholesterol and triglycerides levels, hematocrit and/or haemoglobin concentration. In another embodiment, the treatment improves one or more renal functional parameters selected from the group consisting of Glomerular Filtration Rate (GFR) (e.g., as assessed by Iohexol plasma clearance measurement), Albumin, IgG, sodium, potassium fractional clearance, and renal resistivity index (e.g., as assessed by ultrasound evaluation).

### VI. Kits and Unit Dosage Forms

Also provided herein are kits which include a pharmaceutical composition containing an anti-C5 antibody, or antigen binding fragment thereof, such as Eculizumab, and a pharmaceutically-acceptable carrier, in a therapeutically effective amount adapted for use in the preceding methods. The kits optionally also can include instructions, *e*.*g*., comprising administration schedules, to allow a practitioner (*e*.*g*., a physician, nurse, or patient) to administer the composition contained therein to administer the composition to a patient having MPGN. The kit also can include a syringe.

Optionally, the kits include multiple packages of the single-dose pharmaceutical compositions each containing an effective amount of the anti-C5 antibody, or antigen binding fragment thereof, for a single administration in accordance with the methods provided above. Instruments or devices necessary for administering the pharmaceutical composition(s) also may be included in the kits. For instance, a kit may provide one or more pre-filled syringes containing an amount of the anti-C5 antibody, or antigen binding fragment thereof.

In one embodiment, the kit comprises a dose of an anti-C5 antibody, or antigen binding fragment thereof, comprising: CDR1, CDR2, and CDR3 heavy chain sequences as set forth in SEQ ID NOs:1, 2, and 3, respectively, and CDR1, CDR2, and CDR3 light chain sequences as set forth in SEQ ID NOs: 4, 5, and 6, and; and instructions for using the anti-C5 antibody, or antigen binding fragment thereof, in any of the methods described herein.

In another embodiment, the kit comprises a dose of an anti-C5 antibody, or antigen binding fragment thereof, comprising CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6; and instructions for using the anti-C5 antibody, or antigen binding fragment thereof, in any of the methods described herein.

The following examples are merely illustrative and should not be construed as limiting the scope of this disclosure in any way as many variations and equivalents will become apparent to those skilled in the art upon reading the present disclosure.

The contents of all references, Genbank entries, patents and published patent applications cited throughout this application are expressly incorporated herein by reference.

### EXAMPLES

### EXAMPLE 1: "Eagle Study"

A phase II trial ("Eagle Study") is conducted to explore the efficacy of Eculizumab in patients with PNH biopsy-proven MPGN and Nephrotic syndrome.

### 1. Objectives

The primary objective of the study is to evaluate whether Eculizumab therapy may reduce 24 hour proteinuria, considered as a continuous parameter, at 6 months (week-24) and 12 months (week-48) versus baseline.

Secondary objectives include assessing: (1) whether Eculizumab therapy may achieve persistent, either complete or partial, remission of the nephrotic syndrome (defined as 24-hour urinary protein excretion reduction to <0.3 grams or to <3.5 grams with at least 50% reduction from baseline for adults or <40 mg/h/m2 with at least 50% reduction from baseline for children, respectively), (2) the effect of Eculizumab therapy on relapses of nephrotic syndrome defined as increase of 24-hour urinary protein excretion to 3.5 grams for adults or >40 mg/h/m2 for children after a period of complete or partial remission, (3) the effect of Eculizumab therapy on clinical (body weight, systolic and diastolic blood pressure) and laboratory parameters (urinary albumin/creatinine ratio, serum creatinine, creatinine clearance, serum total proteins, serum albumin, LDL, HDL cholesterol and triglycerides levels, hematocrit and haemoglobin concentration), (4) the effect of Eculizumab therapy on renal functional parameters (Glomerular Filtration Rate (GFR) by Iohexol plasma clearance measurement, Albumin, IgG, Sodium, Potassium fractional clearance, renal resistivity index by ultrasound evaluation), (5) the effect of Eculizumab therapy on markers of complement activity (C3, C4, C3a, C5a, Bb and sC5b9), (6) the immunohystochemical (C3, IgG, C4d, C5b-9), structural and ultrastructural changes associated with remission of proteinuria in patients with evidence of complete or partial remission of the nephrotic syndrome, (7) the safety profile of the Eculizumab treatment, (8) the cost/effectiveness of the study treatment, (9) and the evaluate biomarkers to be tested in case of significant treatment effect on primary efficacy variable.

### 2. Patients

Inclusion criteria are as follows:
A. Biopsy-proven primary MPGN;
B. Creatinine clearance >20 ml/min per 1.73m2;
C. 24-hour proteinuria persistently exceeding 3.5 g in adults or exceeding 40 mg/h/m2 in children (or exceeding 2 mg protein/mg creatinine in children spot urine samples);
D. Persistently low C3 levels in at least two consecutive evaluations;
E. Persistently high sC5b9 levels (>1000 ng/ml) in at least two previous consecutive
F. evaluations; and
G. Written informed consent (by parents or tutors if underage).

Exclusion criteria are as follows:
A. Age ≥ 75 years
B. Secondary MPGN (evidence of infection, immunological disease including vasculitis, systemic diseases and proliferative disorders);
C. Evidence at kidney biopsy evaluation of severe chronic histological changes that very unlikely could benefit of eculizumab therapy;
D. Concomitant steroid or immunosuppressive therapy;
E. Pregnancy or lactating;
F. Childbearing potential without effective contraception;
G. Any clinically relevant condition that might affect completion of the study participation and/or confound study results;
H. Inability to understand the potential risks and benefits of the study; and
I. Legal incapacity.

### 3. Study Design

This is a pilot pathophysiology, prospective, sequential, open label study. Ten patients with primary MPGN and persistent nephrotic range proteinuria are enrolled in the study. In all these patients, biological samples for biochemical and genetic screening (including antibodies to complement-regulating proteins, mutations in the complement and in complement-regulating proteins and allele variants) have been collected.

After baseline evaluation of renal biopsy, 24-hour urine collection, morning urine analysis, routine laboratory tests (markers of complement activity test - C3, C4, C3a, C5a, Bb and sC5b9, renal function profile, protein electrophoresis, electrolytes, hepatic profile, lipid profile, inflammatory index, hemocrome and complete blood cell count, platelets, ferritine, CPK, LDH, glucose), glomerular filtration rate (GFR) and IgG, albumin and sodium fractional clearance, ultrasound evaluation with contrast enhancer, patients receive a first intravenous infusion of Eculizumab.

Baseline evaluations are repeated at week 24 (excluding renal biopsy) and at week 48. At week 48 renal biopsy is performed only in patients with evidence of complete or partial remission of the nephrotic syndrome to evaluate the immunohystochemical (C3, C4, IgG, C5b-9), structural and ultrastructural changes associated with remission of proteinuria. During the induction phase (4 weeks) safety parameters and markers of complement activity are measured weekly. During the maintenance phase (44 weeks) safety parameters are measured monthly. Additional evaluations are allowed whenever deemed clinically appropriate, in particular for safety reasons. Additional plasma, serum, and urine samples will be collected, at basal, at week 2, 4, 12, 24, 36, and 48 for evaluation.

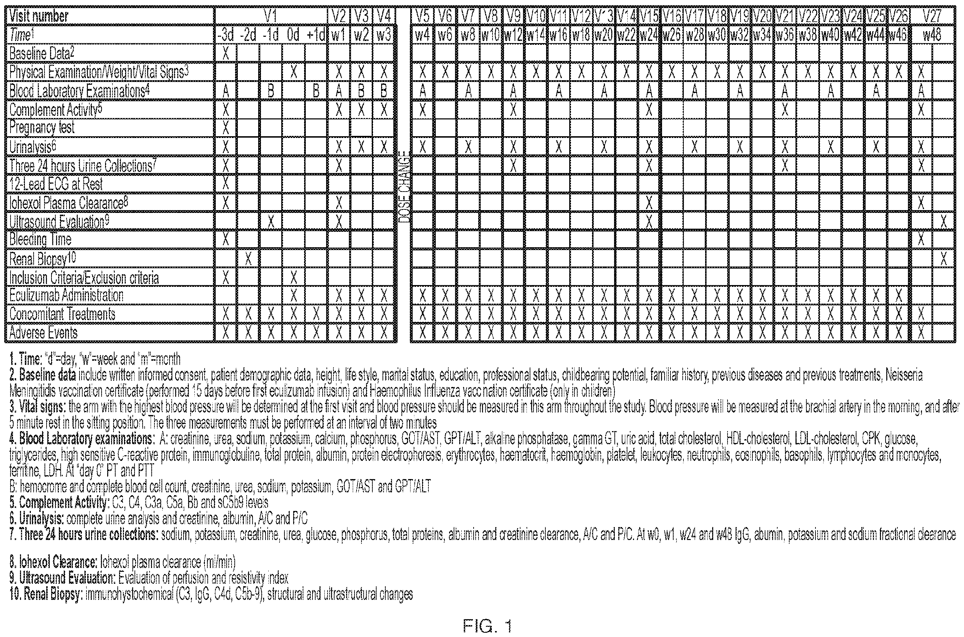

The study visit schedule is as follows and is depicted in Figure 1:

### Visit 1 (week0)

### Day -3:

Baseline data include written informed consent, patient demographic data, height, life style, marital status, education, professional status, childbearing potential, familiar history, previous diseases and previous treatments, Neisseria Meningitidis vaccination certificate (performed 15 days before first Eculizumab infusion) and Haemophilus Influenza vaccination certificate (only in children).

Blood laboratory examinations include assessment of creatinine, urea, sodium, potassium, calcium, phosphorus, GOT/AST, GPT/ALT, alkaline phosphatase, gamma GT, uric acid, total cholesterol, HDL-cholesterol, LDL-cholesterol, CPK, glucose, triglycerides, high sensitive C-reactive protein, immunoglobuline, total proteins, albumin, protein electrophoresis, erythrocytes, haematocrit, haemoglobin, platelet, leukocytes, neutrophils, eosinophils, basophils, lymphocytes and monocytes, ferritine, LDH, PT and PTT.

Complement activity is assessed via C3, C4, C3a, C5a, Bb and sC5b9 levels.

A pregnancy test for beta Hcg is conducted.

A urinalysis is performed, including a complete urine analysis and creatinine, albumin, A/C and P/C. Specifically, three 24 hours urine collections are performed to assess sodium, potassium, creatinine, urea, glucose, phosphorus, total proteins, albumin, creatinine clearance, A/C, P/C. Albumin, IgG, sodium and potassium fractional clearance.

A 12-Lead ECG is performed at rest.

Iohexol clearance is assessed by Iohexol plasma clearance (ml/min).

Bleeding time is assessed for biopsy evaluation.

Inclusion/Exclusion criteria are assessed.

### Day -2:

A renal biopsy is performed to assess immunohystochemical (C3, IgG, C4d, C5b-9), structural and ultrastructural changes.

### Day -1:

An ultrasound evaluation is performed to evaluate perfusion and resistivity index.

Select blood laboratory examinations include assessment of hemocrome and complete blood cell count, creatinine, urea, sodium, potassium, GOT/AST and GPT/ALT.

### Day 0:

A physical examination is performed and weight/vital signs are evaluated.

Inclusion/Exclusion criteria are assessed.

Eculizumab is administered.

### Day 1:

Blood laboratory examination includes assessment of hemocrome and complete blood cell count, creatinine, urea, sodium, potassium, GOT/AST and GPT/ALT.

### Visit 2 (week1) and Visit 15 (week24)

A physical examination is performed and weight/vital signs are evaluated.

Blood laboratory examination includes assessment of creatinine, urea, sodium, potassium, calcium, phosphorus, GOT/AST, GPT/ALT, alkaline phosphatase, gamma GT, uric acid, total cholesterol, HDL-cholesterol, LDL-cholesterol, CPK, glucose, triglycerides, high sensitive C-reactive protein, immunoglobuline, total proteins, albumin, protein electrophoresis, erythrocytes, haematocrit, haemoglobin, platelet, leukocytes, neutrophils, eosinophils, basophils, lymphocytes and monocytes, ferritine, LDH, cystatin C. Complement activity is assessed by C3, C4, C3a, C5a, Bb and sC5b9 levels. Plasma and urinary biomarkers are assessed on visit 15.

An ultrasound evaluation is performed to evaluate perfusion and resistivity index.

A urinalysis is performed, including a complete urine analysis and assessment of creatinine, albumin, A/C and P/C. Specifically, three 24 hours urine collections are performed to evaluate sodium, potassium, creatinine, urea, glucose, phosphorus, total proteins, albumin, creatinine clearance, A/C, P/C. Albumin, IgG, sodium and potassium fractional clearance.

Iohexol clearance is assessed by Iohexol plasma clearance (ml/min).

Eculizumab is administered.

### Visit 3 (week2), visit 4 (week3)

A physical examination is performed and weight/vital signs are evaluated.

Blood laboratory examination includes assessment of hemocrome and complete blood cell count, creatinine, urea, sodium, potassium, GOT/AST and GPT/ALT.

Complement activity is assessed by C3, C4, C3a, C5a, Bb and sC5b9 levels. Biomarkers are assessed on visit 3.

A urinalysis is performed, including a complete urine analysis and assessment of creatinine, albumin, A/C and P/C.

Eculizumab is administered.

### Visit 5 (week4), visit 7 (week8), visit 11 (week16), visit 13 (week20), visit 17 (week28), visit 19 (week32), visit 23 (week40), visit 25 (week44)

A physical examination is performed and weight/vital signs are evaluated.

Blood laboratory examination includes assessment of creatinine, urea, sodium, potassium, calcium, phosphorus, GOT/AST, GPT/ALT, alkaline phosphatase, gamma GT, uric acid, total cholesterol, HDL-cholesterol, LDL-cholesterol, CPK, glucose, triglycerides, high sensitive C-reactive protein, immunoglobuline, total proteins, albumin, protein electrophoresis, erythrocytes, haematocrit, haemoglobin, platelet, leukocytes, neutrophils, eosinophils, basophils, lymphocytes and monocytes, ferritine, and LDH.

Biomarkers are assessed on visit 5.

A urinalysis is performed and includes a complete urine analysis and assessment of creatinine, albumin, A/C and P/C.

Eculizumab is administered.

Complement activity is assessed by C3, C4, C3a, C5a, Bb and sC5b9 levels only for visit 5 *(week4).*

### Visit 6 (week6), visit 8 (week10), visit 10 (week14), visit 12 (week18), visit 14 (week22), visit 16 (week26), visit 18 (week30), visit 20 (week34), visit 22 (week38), visit 24 (week42), visit 26(week46)

A physical examination is performed and weight/vital signs are evaluated.

Eculizumab is administered.

### Visit 9 (week12), visit 21 (week36)

A physical examination is performed and weight/vital signs are evaluated.

Blood laboratory examination includes assessment of creatinine, urea, sodium, potassium, calcium, phosphorus, GOT/AST, GPT/ALT, alkaline phosphatase, gamma GT, uric acid, total cholesterol, HDL-cholesterol, LDL-cholesterol, CPK, glucose, triglycerides, high sensitive C-reactive protein, immunoglobuline, total proteins, albumin, protein electrophoresis, erythrocytes, haematocrit, haemoglobin, platelet, leukocytes, neutrophils, eosinophils, basophils, lymphocytes and monocytes, ferritine, and LDH.

Complement activity is assessed by C3, C4, C3a, C5a, Bb and sC5b9 levels.

Biomarkers are assessed.

A urinalysis is performed and includes a complete urine analysis and assessment of creatinine, albumin, A/C and P/C. Specifically, three 24 hours urine collections are performed to assess sodium, potassium, creatinine, urea, glucose, phosphorus, total proteins, albumin, creatinine clearance, A/C, and P/C.

Eculizumab is administered.

### Visit 27 (week 48)

### Day 1:

A physical examination is performed and weight/vital signs are evaluated.

Blood laboratory examination includes assessment of creatinine, urea, sodium, potassium, calcium, phosphorus, GOT/AST, GPT/ALT, alkaline phosphatase, gamma GT, uric acid, total cholesterol, HDLcholesterol, LDL-cholesterol, CPK, glucose, triglycerides, high sensitive C-reactive protein, immunoglobuline, total proteins, albumin, protein electrophoresis, erythrocytes, haematocrit, haemoglobin, platelet, leukocytes, neutrophils, eosinophils, basophils, lymphocytes and monocytes, ferritine, LDH, PT, PTT, and cystatin C.

Complement activity is assessed by C3, C4, C3a, C5a, Bb and sC5b9 levels.

A urinalysis is performed and includes assessment of creatinine, albumin, A/C and P/C. Specifically, three 24 hours urine collections are performed to assess sodium, potassium, creatinine, urea, glucose, phosphorus, total proteins, albumin, creatinine clearance, A/C, P/C. Albumin, IgG, sodium and potassium fractional clearance.

Iohexol clearance is evaluated by Iohexol plasma clearance (ml/min).

Bleeding time is evaluated for biopsy evaluation.

### Day 2:

A renal biopsy is performed to assess immunohystochemical (C3, IgG, C4d, C5b-9), structural and ultrastructural changes (only in patients with evidence of complete or partial remission of Nephrotic syndrome).

An ultrasound evaluation is performed to evaluate perfusion and resistivity index.

At the end of first year the patients are followed every six months. Examinations include:
- Physical examination/weight/vital signs;
- Blood laboratory examinations: creatinine, urea, sodium, potassium, calcium, phosphorus, GOT/AST, GPT/ALT, alkaline phosphatase, gamma GT, uric acid, total cholesterol, HDLcholesterol, LDL-cholesterol, CPK, glucose, triglycerides, high sensitive C-reactive protein, immunoglobuline, total proteins, albumin, protein electrophoresis, erythrocytes, haematocrit, haemoglobin, platelet, leukocytes, neutrophils, eosinophils, basophils, lymphocytes and monocytes, ferritine, LDH, PT and PTT;
- Assessment of complement activity: C3, C4, C3a, C5a, Bb and sC5b9 levels;
- Urinalysis: complete urine analysis and creatinine, albumin, A/C and P/C;
- Three 24 hours urine collections: sodium, potassium, creatinine, urea, glucose, phosphorus, total proteins, albumin, creatinine clearance, A/C, P/C. Albumin, IgG, sodium and potassium fractional clearance;
- Iohexol Clearance: Iohexol plasma clearance (ml/min);
- Ultrasound evaluation: Evaluation of perfusion and resistivity index.

### 4. Outcome Variables

The primary efficacy variable is reduction of 24 hour proteinuria. Secondary efficacy variables include (1) complete or partial remission and relapses of nephrotic syndrome , (2) normalization (reduction to <303 ng/ml) if sC5b-9 plasma levels, (3) normalization in plasma levels of other components of the complement system including, C3, C4, C3a, C5a, and Bb, (4) amelioration of kidney function/perfusion parameters including measured and estimated glomerular filtration rate (GFR); albumin, IgG, sodium and potassium fractional clearance; renal resistivity index (5) amelioration of renal immunohistochemical (C3, C5b-9, IgG, IgA, IgM, C4d, C1q, kappa light chain, lambda light chain, CD21, C5aR), structural and ultrastructural changes in patients achieving complete or partial remission of the nephrotic syndrome, and (6) changes in serum albumin, lipids and other clinical and laboratory parameters.

Safety outcomes include serious and non serious adverse events, including acute reactions during Eculizumab infusion, infectious episodes (including meningoencephalitis). Outcome data and treatment costs are used for cost/effectiveness analyses.

### 5. Methods

### Determination of sC5b-9 in plasma

Blood is collected on EDTA and centrifuged at 2000xg for 20 min at 4°C. Plasma is stored at - 20°C (till six months after the sampling) or at - 80°C. sC5b-9 levels re assessed by enzyme-linked immunoassay commercially available from Quidel (MicroVue SC5b-9 Plus).

### Immunofluorescence staining for IgG, C3, and C5b9

Frozen sections are fixed in acetone for 10 min at 4 °C. The sites of nonspecific binding are blocked with PBS1X/BSA3%. Then, sections are incubated with the following specific antibodies: fluorescein isothiocyanate (FITC)-conjugated rabbit anti-human C3 (1:25, Dako), FITC-conjugated rabbit anti-human IgG (1:25, Dako), and rabbit anti-human C5b9 (1:200, Calbiochem). For C5b9 staining, the sections are incubated with Cy3-conjugated secondary antibody goat anti-rabbit IgG (1:100, Jackson ImmunoResearch Laboratories). Negative controls are obtained by omitting the primary antibody. Evaluation is done by two blinded investigators. At least 10 glomeruli for each section are analyzed and the signal intensity is graded on a scale of 0-3 (0 = no staining; 1 = weak staining; 2 = staining of moderate intensity; 3 = strong staining).

### Immunoperoxidase staining for C4d

Dubosq-Brazil -fixed and paraffin embedded sections are treated with citrate buffer (10 mM Citric Acid, pH6) and proteinase K (10 min at 37°C, 20 µg/ml) for antigen retrieval. The sites of non-specific binding are blocked with PBS1X/BSA1%/ 5% goat serum. Then, sections are incubated with the primary antibody (C4d, 1:50, Pantec-Biomedica), and with the biotinylated secondary antibody goat anti-rabbit IgG (1:150, Vector Laboratories). Signals re developed with 3, 3'-diaminobenzidine (DAB, Vector Laboratories), and the sections are counterstained with hematoxylin-eosin. Negative controls are obtained by omitting the primary antibody.

At least 10-20 non overlapping field for each section are examined by two blinded investigators, and the signal intensity is graded on a scale of 0-3 (0 = no staining; 1 = weak staining; 2 = staining of moderate intensity; 3 = strong staining).

### Glomerular Filtration Rate

The GFR is centrally determined using the iohexol plasma clearance technique. On the morning of renal function evaluation, 5 ml of iohexol solution (Omnipaque 300, GE Healthcare, Milan, Italy) is injected intravenously over 2 minutes. Multiple blood samples are taken at different times and blood iohexol plasma levels are measured by high-performance liquid chromatography. The clearance of iohexol is calculated according to a one-compartment model (CL1) by the formula: CL1=Dose/AUC, where AUC is the area under the plasma concentration-time curve16. Plasma clearances are then corrected by using the formula CL =(0.9907786CL11)-(0.0012186CL12), and GFR values are normalized to 1.73 m2 of body surface area (BSA). This procedure has remarkable precision over a wide range of kidney function as documented by the low mean intra-individual coefficient of variation (5.59%) and good reproducibility index (6.28%) observed in repeated measurements in subjects with near terminal kidney failure, normal GFR or even hyperfiltration.

### 6. Investigational Medicinal Product (IMP)

The Name of the IMP is Soliris^{®} 300 mg concentrate for solution for infusion. Soliris^{®} (Eculizumab) is a humanised monoclonal (IgG2/4) antibody produced in NS0 cell line by recombinant DNA technology. One vial of 30 ml contains 300 mg of Eculizumab (10 mg/ml). After dilution, the final concentration of the solution infused is 5 mg/ml.

Excipients with known effect: Sodium (5 mmol per vial). Excipients include sodium phosphate (monobasic), sodium phosphate (dibasic), sodium chloride, Polysorbate 80, and water for injections.

Eculizumab is stored in a refrigerator (2°C - 8°C) and is not frozen. It is stored in the original package in order to protect from light. Eculizumab vials in the original package can be removed from refrigerated storage for only one single period of up to 3 days. At the end of this period the product is put back in the refrigerator. After dilution, the medicinal product is used immediately. However, chemical and physical stability has been demonstrated for 24 hours at 2°C - 8°C.

To reduce the risk of infection, all patients are vaccinated at least 2 weeks prior to receiving Eculizumab. Patients who are treated with Eculizumab less than 2 weeks after receiving a meningococcal vaccine receive treatment with appropriate prophylactic antibiotics until 2 weeks after vaccination. Patients are re-vaccinated according to current medical guidelines for vaccination use. Tetravalent vaccines against serotypes A, C, Y and W135 are strongly recommended, preferably conjugated ones. Patients for which vaccination is contraindicated, receive treatment with appropriate prophylactic antibiotics during all treatment period.

Patients less than 18 years of age are vaccinated against haemophilus influenzae and pneumococcal infections, and strictly adhere to the national vaccination recommendations for each age group.

Eculizumab is administered by a healthcare professional and under the supervision of a physician experienced in the management of patients with haematological and/or renal disorders. For the first administration, an intensivist attends the start of treatment and is on call throughout the whole duration of infusion. For all the other administrations, the intensivist is on call.

The dosing regimen for adult patients (≥18 years of age) consists of a 4 week initial phase followed by a maintenance phase as shown in Table 1:

**Table 1: Dosing Regimen for Adults**

| | |
|---|---|
| **Initial phase** | 900 mg of Eculizumab via a 25 - 45 minute intravenous infusion every week for the first 4 weeks |
| **Maintenance phase** | 1200 mg of Eculizumab administered via a 25 - 45 minute intravenous infusion for the fifth week, followed by 1200 mg of Eculizumab administered via a 25 - 45 minute intravenous infusion every 14 ± 2 days |

In pediatric patients (less than 18 years), the Eculizumab dosing regimen is as shown in Table 2:

**Table 2: Dosing Regimen for Pediatric Patients**

| **Patient Body Weight** | **Initial Phase** | **Maintenance Phase** |
|---|---|---|
| ≥40 kg | 900 mg weekly x 4 | 1200 mg at week 5; then 1200 mg every 2 weeks |
| 30 - <40 kg | 600 mg weekly x 2 | 900 mg at week 3; then 900 mg every 2 weeks |
| 20 - <30 kg | 600 mg weekly x 2 | 600 mg at week 3; then 600 mg every 2 weeks |
| 10 - <20 kg | 600 mg weekly x 1 | 300 mg at week 2; then 300 mg every 2 weeks |
| 5 - <10 kg | 300 mg Weekly x 1 | 300 mg at week 2; then 300 mg every 3 weeks |

Eculizumab is not administered as an intravenous push or bolus injection. Eculizumab is only administered via intravenous infusion as described below. Prior to administration, the Eculizumab solution is visually inspected for particulate matter and discolouration. Reconstitution and dilution is performed in accordance with good practices rules, particularly for the respect of asepsis. The total amount of Eculizumab is drawn from the vial(s) using a sterile syringe. The recommended dose is transferred to an infusion bag. Eculizumab is diluted to a final concentration of 5 mg/ml by addition to the infusion bag using sodium chloride 9 mg/ml (0.9%) solution for injection, sodium chloride 4.5 mg/ml (0.45%) solution for injection, or 5% dextrose in water, as the diluents. The final volume of a 5 mg/ml diluted solution is 60 ml for 300 mg doses, 120 ml for 600 mg doses, 180 ml for 900 mg doses and 240 ml for 1200 mg doses. The solution is clear and colourless. The infusion bag containing the diluted solution is gently agitated to ensure thorough mixing of the product and diluents. The diluted solution is allowed to warm to room temperature prior to administration by exposure to ambient air. Any unused portion left in a vial is discarded, as the product contains no preservatives. Any unused medicinal product or waste material is disposed of in accordance with local requirements.

The diluted solution of Eculizumab is administered by intravenous infusion over 25 - 45 minutes via gravity feed, a syringe-type pump, or an infusion pump. It is not necessary to protect the diluted solution of Eculizumab from light during administration to the patient. Patients are monitored for one hour following infusion. If an adverse event occurs during the administration of Eculizumab, the infusion is slowed or stopped at the discretion of the physician. If the infusion is slowed, the total infusion time does not exceed two hours in adults and adolescents (aged 12 years to under 18 years) and four hours in children aged less than 12 years. All the patients are maintained on active follow-up until six months after the last Eculizumab administration. For patients with evidence of complete or partial remission of the nephrotic syndrome after one year of treatment, Eculizumab therapy is continued as compassionate use. In the case of drug withdrawal (side effects such as serious infections and leucopenia, noncompliance of the patient), the patient continues conservative therapy and clinical monitoring at its reference center up to one year from the beginning of the study.

Steroid and immunosoppressive drugs are withdrawn six months before Eculizumab administration.

Study drug is manufactured by Alexion Pharmaceuticals, Inc. Re-labelling of IMP is committed to a certified company. Bottles are re-labeled with "tear-off" labels. When administering the study medication, then investigator peels off the respective part of the label from the each box and fixes these to the provided space in the appropriate form.

### 7. Statistical Methods

A Statistical Analysis Plan (SAP) detailing the analyses described below is developed prior to the database lock. Analyses re carried out using SAS (version 9.1) or higher and Stata (version 12) software or higher or other validated software. The SAP, which describes in detail the methods to be used for the primary and secondary endpoints, serves as the final arbiter of all statistical analyses.

### Sample Size Estimation

This is a clinical pilot study, with exploratory purposes where the number of participants is based on the available patients, instead of a sample size calculated on the expected change in considered outcome variables.

### Statistical Analysis

All continuous outcome variables are evaluated by means of a linear mixed-effect model which will include pre-defined baseline covariates. The above model incorporates random effects (with an unstructured covariance matrix) to account for correlated observations on the same patient. The Kaplan-Meier method is used for survival data. Survival time are determined from the beginning of the first treatment until the event of interest. In a multivariable context, the Cox regression model is carried out. It is expected that proteinuria, triglycerides, and duration of persistent proteinuria show a skewed distribution and then are log-transformed before statistical analyses. The rate of GFR decline is evaluated by a single linear model by using at least three GFR values, including baseline. The correlation between proteinuria reduction on follow-up and GFR decline after Eculizumab administration is evaluated by using the Spearman's rank correlation test. The data of baseline characteristics is presented as numbers and percentages, means and SDs, or medians and interquartile ranges (IQRs), as appropriate. Comparisons among groups is made using one-way ANOVA, the Kruskal-Wallis test, the chi-squared test, or the Cochran-Armitage test for trend, as appropriate. The multiple comparisons issue is addressed by means of Bonferroni adjustment. The follow-up data are expressed as medians and ranges or IQRs. Normality for continuous variables is assessed by means of the Q-Q plot. All P values are two sided.

### 8. Withdrawal of Patients

Patients are withdrawn (1) at their own request without giving reasons, (2) at the discretion of the investigator, or (3) if adverse events (including intercurrent illnesses) develop, which rule out continuation of the study medication.

The reason for withdrawal is documented in the case report form and in the patient's medical records. Whenever feasible, patients who will prematurely stop the study treatment are maintained on active follow-up up to completion of the planned-observation period.

### 9. Premature Discontinuation of the Study

The investigator has the right to discontinue the study at any time for reasonable medical and/or administrative reasons. Reasons for discontinuation are documented appropriately and information is issued according to local requirements (e.g., to ethics committees/ authorities).

### 10. Adverse Event

An "adverse event" is any untoward medical occurrence in a patient or clinical investigation patient administered a pharmaceutical product and which does not necessarily have to have a causal relationship with this treatment. An adverse event (AE) can therefore, be any unfavorable and unintended sign (including an abnormal laboratory finding, for example), symptom, or disease temporally associated with the use of a medicinal product, whether or not considered related to the medicinal product.

An "adverse reaction" is any untoward and unintended response to the IMP related to any dose administered. A response to the medicinal product is given when a causal relationship between the adverse event and one of the IMPs is at least a reasonable possibility.

A "serious adverse event" is any untoward medical occurrence that at any dose: results in death, is immediately life-threatening, requires inpatient hospitalization or prolongation of existing hospitalization, results in persistent or significant disability/incapacity, is a congenital malformation/birth defect, or any other medically important condition

An event which does not meet the above definitions is considered to be "Not Serious".

A "suspected unexpected serious adverse reaction" (SAE) is a reaction, the nature or severity of which is not consistent with the applicable product information.

### EXAMPLE 2: "Eagle Study" Interim Results

A phase II trial was conducted to explore the efficacy of Eculizumab in patients with PNH biopsy-proven MPGN and Nephrotic syndrome substantially according to the protocol described above in Example 1.

### 1. Data

Up to June 24, 2015, ten included patients had a full set of data available for analyses at 12 weeks of follow-up (Cohort 1). Eight out of these ten patients also had a full set of data available for analyses at 24 weeks (Cohort 2). The baseline patient characteristics are set forth in Table 3.

**Table 3: Baseline Patient Characteristics**

| | | |
|---|---|---|
| Gender | Males / Females | 6/4 |
| Age | Years - median (range) | 18 (13-33) |
| Histology Pattern | C3GN/IC-MPGN | 4/6 |
| Circulating C3 Nef | Y/N | 4/6 |
| Identified Complement Mutation | Y/N | 2/8 |
| sC3b9 | mg/dl - median (IQR) | 2420 (1916-3331) |
| Serum creatinine | mg/dl | 1.18 ± 0.9 |
| Serum albumin | g/dl | 2.29 ± 0.5 |
| GFR | ml/min | 69.7 ± 4.3 |
| Proteinuria | g/2-h | 7.61 ± 4.3 |
| ACE inhibition therapy | Y/N | 10/0 |

| | | |
|---|---|---|
| Data are mean ± SD, if not differently stated. | | |

Results of the analyses of serum C5b9 levels are reported for both cohorts up to last available follow up (12 and 24 weeks, respectively), along with data on the primary efficacy variable (24-hour urinary protein excretion) and other key efficacy variables, including serum 24-hour urinary albumin excretion, measured glomerular filtration rate (GER), albumin and IgG fractional clearances, serum creatinine, albumin, total proteins, total, LDL and IIDL cholesterol, and triglyceride levels, and sytolic and diastolic blood pressure. The iohexol plasma clearance was not measured in one patient because of reported history of allergy. In another patient, data were still not available for analyses at the time of the present report. Thus data on GER and GER-related parameters, such as albumin and IgG fractional clearances, were available for eight patients of Cohort 1 and six patients of Cohort 2.

For each considered variable data were reported at inclusion (baseline) and at each available time point on follow-up (expressed as week post-baseline) as mean, standard deviation (SD), standard error of the mean (SEM), median, interquartile range (IQR), minimum (Min) and maximum (Max). Within-patients comparisons were between each time point versus baseline. The level of significance of each comparison is reported at the bottom of each table. Levels who failed to achieve the statistical nominal significance (p≤0.05) are not shown.

### Serum C5b9 Levels

In all patients serum C5b9 levels largely exceeded the upper limit of the normal range at inclusion and normalized throughout the whole observation period. Serum level decreases exceeded one order of magnitude in all patients and was highly significant at each time point compared with baseline (Table 4).

**Table 4: Serum sC5b9 (ng/mL)**

| Cohort 1 *(n = 10)* | | | | | | | |
|---|---|---|---|---|---|---|---|
| Visit | Mean | SD | SEM | Median | IQR | Min | Max |
| Baseline | 3099.25 | 2072.40 | 655.35 | 2420.20 | 1915.70 to 3330.00 | 988.00 | 8130.00 |
| Week 1 | 186.85* | 82.46 | 26.08 | 176.61 | 114.00 to 206.60 | 86.20 | 348.00 |
| Week 2 | 162.65* | 60.10 | 22.72 | 186.55 | 104.30 to 220.00 | 82.40 | 233.32 |
| Week 3 | 236.06* | 197.24 | 74.55 | 178.00 | 146.26 to 232.70 | 86.20 | 671.64 |
| Week 4 | 351.05* | 253.16 | 95.68 | 328.42 | 137.03 to 622.38 | 93.10 | 748.30 |
| Week 12 | 291.45* | 95.61 | 42.76 | 315.20 | 262.94 to 364.50 | 140.00 | 374.60 |

| Cohort 2 *(n=8)* | | | | | | | |
|---|---|---|---|---|---|---|---|
| Visit | Mean | SD | SEM | Median | IQR | Min | Max |
| Baseline | 3273.81 | 2311.31 | 817.17 | 2553.20 | 1804.53 to 4178.50 | 988.00 | 8130.00 |
| Week 1 | 186.48* | 93.05 | 32.90 | 172.50 | 107.50 to 248.80 | 86.20 | 348.00 |
| Week 2 | 162.65* | 60.10 | 22.72 | 186,55 | 104.30 to 220.00 | 82.40 | 233.32 |
| Week 3 | 236.06* | 197.24 | 74.55 | 178.00 | 146.26 to 232.70 | 86.20 | 671.64 |
| Week 4 | 351.05* | 253.16 | 95.68 | 328.42 | 137.03 to 622.38 | 93.10 | 748.30 |
| Week 12 | 291.45* | 95.61 | 42.76 | 315.20 | 262.94 to 364.50 | 140.00 | 374.60 |
| Week 24 | 279.17* | 92.05 | 32.54 | 247.05 | 214,00 to 348.60 | 174.08 | 440.00 |

| | | | | | | | |
|---|---|---|---|---|---|---|---|
| *p<0.0005 vs Baseline | | | | | | | |

### 24-hour Urinary Protein and Albumin Excretion

24-hour urinary protein excretion, primary efficacy variable of the study, and urinary albumin excretion decreased in all patients during the follow-up period, with the exception of one single patient who showed no appreciable change in both parameters at each considered time point as compared with baseline. Urinary protein excretion decreased significantly at each time point in both cohorts and the decrease was progressive over time up to the last available follow up (Table 5). The decrease in urinary albumin excretion was also progressive over time and achieved the nominal significance at week 1 in both cohorts and at week 24 in the Cohort 2 (Table 6). Failure to achieve the nominal significance at week 12 in both cohorts was mainly explained by a large variability of observed changes versus baseline.

**Table 5: 24-h Urinary protein excretion (grams)**

| Cohort 1 *(n=10)* | | | | | | | |
|---|---|---|---|---|---|---|---|
| Visit | Mean | SD | SEM | Median | IQR | Min | Max |
| Baseline | 7.61 | 4.31 | 1.36 | 6.03 | 4.84 to 12.41 | 3.28 | 14.82 |
| Week 1 | 6.55* | 4.68 | 1.48 | 4.47 | 3.61 to 11.86 | 1.68 | 14.80 |
| Week 12 | 4.65* | 1.69 | 0.53 | 4.83 | 3.63 to 5.36 | 2.25 | 8.02 |

| Cohort 2 *(n=8)* | | | | | | | |
|---|---|---|---|---|---|---|---|
| Visit | Mean | SD | SEM | Median | IQR | Min | Max |
| Baseline | 8.50 | 4.38 | 1.55 | 6.13 | 5.48 to 13.07 | 3.83 | 14.82 |
| Week 1 | 7.24* | 5.05 | 1.78 | 5.05 | 3.41 to 12.26 | 1.68 | 14.80 |
| Week 12 | 4.97* | 1.68 | 0.59 | 4.88 | 4.21 to 5.66 | 2.25 | 8.02 |
| Week 24 | 4.05° | 1.92 | 0.68 | 3.74 | 3.27 to 4.72 | 1.12 | 7.85 |

| | | | | | | | |
|---|---|---|---|---|---|---|---|
| *p=0.02, °p<0.01 vs Baseline | | | | | | | |

**Table 6: 24-h Urinary Albumin Excretion (mg)**

| Cohort 1 *(n=10)* | | | | | | | |
|---|---|---|---|---|---|---|---|
| Visit | Mean | SD | SEM | Median | IQR | Min | Max |
| Baseline | 3726.63 | 1950.08 | 616.67 | 3133.67 | 2433.00 to 6069.00 | 1339.33 | 6781.00 |
| Week 1 | 3216.63* | 2106.02 | 665.98 | 2512.33 | 1716.67 to 5484.33 | 904.00 | 6953.33 |
| Week 12 | 2693.68 | 826.30 | 275.43 | 2831.67 | 2024.33 to 3225.69 | 1274.33 | 3853.70 |

| Cohort 2 *(n=8)* | | | | | | | |
|---|---|---|---|---|---|---|---|
| Visit | Mean | SD | SEM | Median | IQR | Min | Max |
| Baseline | 4186.75 | 1895.82 | 670.27 | 3382.67 | 2872.00 to 6180.17 | 1843.33 | 6781.00 |
| Week 1 | 3552.79° | 2247.21 | 794.51 | 2630.83 | 1945.67 to 5706.00 | 904.00 | 6953.33 |
| Week 12 | 2992.06 | 629.33 | 237.86 | 3019.44 | 2561.33 to 3509.26 | 1943.33 | 3853.70 |
| Week 24 | 1985.44° | 838.67 | 296.52 | 2005.50 | 1612.00 to 2271.83 | 568.33 | 3536.50 |

| | | | | | | | |
|---|---|---|---|---|---|---|---|
| *p=0.05, ° p=0.02, °° p<0.01 vs. Baseline | | | | | | | |

### Glomerular Filtration Rate

The GFR progressively increased in all patients of both cohorts. The increase was already apparent at week 1 post treatment in both cohorts and achieved the nominal significance at week 24 in the Cohort 2. In Cohort 2 the GFR increase was progressive over time (Table 7).

**Table 7: Glomerular Filtration Rate (ml/min/1.73 m²)**

| Cohort 1 *(n=8)* | | | | | | | |
|---|---|---|---|---|---|---|---|
| Visit | Mean | SD | SEM | Median | IQR | Min | Max |
| Baseline | 67.08 | 36.65 | 12.96 | 68.83 | 30.24 to 93.94 | 27.29 | 123.38 |
| Week 1 | 71.63 | 37.56 | 13.28 | 78.08 | 32.25 to 101.38 | 26.90 | 122.76 |

| Cohort 2 *(n=6)* | | | | | | | |
|---|---|---|---|---|---|---|---|
| Visit | Mean | SD | SEM | Median | IQR | Min | Max |
| Baseline | 71.23 | 38.71 | 15.80 | 68.83 | 30.73 to 105.87 | 29.74 | 123.38 |
| Week 1 | 75.93 | 38.48 | 15.71 | 78.08 | 32.68 to 112.16 | 31.81 | 122.76 |
| Week 24 | 82.00* | 42.10 | 17.19 | 81.86 | 37.67 to 123.26 | 33.58 | 133.74 |

| | | | | | | | |
|---|---|---|---|---|---|---|---|
| *p=0.003 vs. Baseline | | | | | | | |

### Albumin and IgG Fractional Clearances

Albumin and IgG Fractional Clearances improved in all patients of both cohorts. The decrease in both parameters was significant in both cohorts at week 1 and in Cohort 2 also at week 24. In Cohort 2 the reduction in albumin and IgG fractional clearances was progressive over time (Tables 8 and 9).

**Table 8: Albumin Fractional Clearance**

| Cohort 1 *(n=8)* | | | | | | | |
|---|---|---|---|---|---|---|---|
| Visit | Mean | SD | SEM | Median | IQR | Min | Max |
| Baseline | 418.12 | 367.74 | 130.02 | 317.21 | 109.72 to 653.54 | 72.32 | 1111.69 |
| Week 1 | 301.42* | 259.42 | 91.72 | 230.57 | 87.10 to 500.06 | 38.94 | 737.01 |

| Cohort 2 *(n=6)* | | | | | | | |
|---|---|---|---|---|---|---|---|
| Visit | Mean | SD | SEM | Median | IQR | Min | Max |
| Baseline | 438.52 | 409.16 | 167.04 | 317.21 | 85.67 to 727.01 | 72.32 | 1111.69 |
| Week 1 | 308.97* | 290.82 | 118.73 | 217.87 | 73.85 to 568.26 | 38.94 | 737.01 |
| Week 24 | 144.58° | 108.31 | 48.44 | 117.93 | 81.33 to 229.62 | 14.78 | 279.23 |

| | | | | | | | |
|---|---|---|---|---|---|---|---|
| * p=0.02, ° p<0.01 vs. Baseline | | | | | | | |

**Table 9: IgG Fractional Clearance**

| Cohort 1 *(n = 8)* | | | | | | | |
|---|---|---|---|---|---|---|---|
| Visit | Mean | SD | SEM | Median | IQR | Min | Max |
| Baseline | 153.54 | 171.99 | 60.81 | 79.51 | 19.32 to 292.42 | 10.33 | 435.49 |
| Week 1 | 94.43* | 99.14 | 35.05 | 52.54 | 15.11 to 182.87 | 3.93 | 250.46 |

| Cohort 2 *(n=6)* | | | | | | | |
|---|---|---|---|---|---|---|---|
| Visit | Mean | SD | SEM | Median | IQR | Min | Max |
| Baseline | 167.79 | 193.77 | 79.10 | 79.51 | 12.92 to 389.00 | 10.33 | 435.49 |
| Week 1 | 97.70* | 110.78 | 45.22 | 49.84 | 10.94 to 221.20 | 3.93 | 250.46 |
| Week 24 | 23.73° | 18.06 | 7.37 | 21.12 | 12.67 to 28.40 | 3.95 | 55.09 |

| | | | | | | | |
|---|---|---|---|---|---|---|---|
| * p=0.01, ° p<0.05 vs. Baseline | | | | | | | |

### Serum Creatinine

Serum creatinine levels did not change appreciably throughout the whole study period in both cohorts (Table 10).

**Table 10: Serum Creatinine (mg/dl)**

| Cohort 1 *(n=10)* | | | | | | | |
|---|---|---|---|---|---|---|---|
| Visit | Mean | SD | SEM | Median | IQR | Min | Max |
| Baseline | 1.18 | 0.94 | 0.30 | 0.68 | 0.50 to 1.95 | 0.39 | 3.18 |
| Week 1 | 1.13 | 0.89 | 0.28 | 0.73 | 0.49 to 1.69 | 0.25 | 2.84 |
| Week 2 | 1.03 | 0.80 | 0.25 | 0.69 | 0.38 to 1.57 | 0.27 | 2.40 |
| Week 3 | 1.02 | 0.74 | 0.23 | 0.72 | 0.37 to 1.71 | 0.35 | 2.15 |
| Week 4 | 1.07 | 0.72 | 0.23 | 0.69 | 0.36 to 1.69 | 0.31 | 2.10 |
| Week 8 | 1.01 | 0.73 | 0.23 | 0.72 | 0.34 to 1.71 | 0.30 | 2.20 |
| Week 12 | 1.11 | 0.81 | 0.26 | 0.68 | 0.41 to 1.88 | 0.31 | 2.40 |

| Cohort 2 *(n = 8)* | | | | | | | |
|---|---|---|---|---|---|---|---|
| Visit | Mean | SD | SEM | Median | IQR | Min | Max |
| Baseline | 1.35 | 0.99 | 0.35 | 1.14 | 0.49 to 1.97 | 0.39 | 3.18 |
| Week 1 | 1.31 | 0.91 | 0.32 | 1.21 | 0.54 to 1.95 | 0.25 | 2.84 |
| Week 2 | 1.19 | 0.82 | 0.29 | 1.04 | 0.48 to 1.89 | 0.27 | 2.40 |
| Week 3 | 1.18 | 0.75 | 0.26 | 1.11 | 0.46 to 1.91 | 0.35 | 2.15 |
| Week 4 | 1.27 | 0.72 | 0.26 | 1.04 | 0.48 to 1.84 | 0.31 | 2.10 |
| Week 8 | 1.16 | 0.74 | 0.26 | 1.10 | 0.49 to 1.82 | 0.30 | 2.20 |
| Week 12 | 1.19 | 0.82 | 0.29 | 0.97 | 0.55 to 2.05 | 0.41 | 2.40 |
| Week 16 | 1.22 | 0.80 | 0.28 | 1.07 | 0.50 to 1.95 | 0.34 | 2.40 |
| Week 20 | 1.27 | 0.92 | 0.33 | 1.05 | 0.42 to 2.09 | 0.36 | 2.70 |
| Week 24 | 1.23 | 0.87 | 0.31 | 1.03 | 0.46 to 1.98 | 0.32 | 2.63 |

### Serum Albumin and Total Proteins

Albumin and total protein levels increased in all patients of both cohorts with the exception of one patient. The increase was progressive over time for both variables in both cohorts. In both cohorts the increase in serum albumin and total protein levels achieved the nominal significance at week 8 compared with baseline and the increase was persistently significant in all subsequent visits up to last available follow up (Tables 11 and 12).

**Table 11: Serum Albumin (g/dl)**

| Cohort 1 *(n = 10)* | | | | | | | |
|---|---|---|---|---|---|---|---|
| Visit | Mean | SD | SEM | Median | IQR | Min | Max |
| Baseline | 2.29 | 0.53 | 0.17 | 2.36 | 2.20 to 2.65 | 1.00 | 2.83 |
| Week 1 | 2.39 | 0.57 | 0.18 | 2.44 | 2.23 to 2.80 | 1.00 | 3.00 |
| Week 4 | 2.53 | 0.37 | 0.12 | 2.50 | 2.30 to 2.80 | 1.91 | 3.00 |
| Week 8 | 2.78* | 0.33 | 0.10 | 2.91 | 2.57 to 3.02 | 2.20 | 3.10 |
| Week 12 | 2.81 * | 0.54 | 0.20 | 2.90 | 2.20 to 3.19 | 2.04 | 3.60 |

| Cohort 2 *(n = 8)* | | | | | | | |
|---|---|---|---|---|---|---|---|
| Visit | Mean | SD | SEM | Median | IQR | Min | Max |
| Baseline | 2.26 | 0.55 | 0.19 | 2.36 | 2.25 to 2.55 | 1.00 | 2.80 |
| Week 1 | 2.37 | 0.65 | 0.23 | 2.49 | 2.17 to 2.84 | 1.00 | 3.00 |
| Week 4 | 2.50 | 0.38 | 0.14 | 2.50 | 2.20 to 2.84 | 1.91 | 3.00 |
| Week 8 | 2.79° | 0.36 | 0.13 | 2.95 | 2.50 to 3.06 | 2.20 | 3.10 |
| Week 12 | 2.81* | 0.54 | 0.20 | 2.90 | 2.20 to 3.19 | 2.04 | 3.60 |
| Week 16 | 3.03° | 0.35 | 0.12 | 3.15 | 2.79 to 3.20 | 2.44 | 3.50 |
| Week 20 | 3.04° | 0.39 | 0.14 | 3.20 | 2.73 to 3.31 | 2.36 | 3.50 |
| Week 24 | 2.75° | 0.35 | 0.12 | 2.89 | 2.52 to 2.99 | 2.07 | 3.13 |

| | | | | | | | |
|---|---|---|---|---|---|---|---|
| * p=0.05, ° p<0.01 vs. Baseline | | | | | | | |

**Table 12: Serum Protein (g/dl)**

| Cohort 1 *(n = 10)* | | | | | | | |
|---|---|---|---|---|---|---|---|
| Visit | Mean | SD | SEM | Median | IQR | Min | Max |
| Baseline | 4.56 | 0.77 | 0.24 | 4.65 | 4.40 to 5.20 | 2.80 | 5.40 |
| Week 1 | 4.87 | 0.76 | 0.24 | 4.95 | 4.60 to 5.20 | 3.00 | 5.90 |
| Week 4 | 4.90 | 0.39 | 0.12 | 4.95 | 4.60 to 5.20 | 4.30 | 5.40 |
| Week 8 | 5.13* | 0.41 | 0.13 | 5.15 | 4.90 to 5.20 | 4.40 | 5.90 |
| Week 12 | 5.23 ° | 0.47 | 0.15 | 5.20 | 4.90 to 5.80 | 4.50 | 5.90 |

| Cohort 2 *(n = 8)* | | | | | | | |
|---|---|---|---|---|---|---|---|
| Visit | Mean | SD | SEM | Median | IQR | Min | Max |
| Baseline | 4.56 | 0.80 | 0.28 | 4.65 | 4.45 to 5.05 | 2.80 | 5.40 |
| Week 1 | 4.90 | 0.86 | 0.30 | 5.10 | 4.75 to 5.30 | 3.00 | 5.90 |
| Week 4 | 4.84 | 0.42 | 0.15 | 4,75 | 4.50 to 5.25 | 4.30 | 5.40 |
| Week 8 | 5.13* | 0.47 | 0.17 | 5.10 | 4.85 to 5.40 | 4.40 | 5.90 |
| Week 12 | 5.28° | 0.52 | 0.18 | 5.25 | 4.85 to 5.80 | 4.50 | 5.90 |
| Week 16 | 5.41 * | 0.50 | 0.18 | 5.50 | 4,95 to 5.85 | 4,70 | 6.00 |
| Week 20 | 5.53° | 0.55 | 0.19 | 5.75 | 5.00 to 5.95 | 4.70 | 6.10 |
| Week 24 | 5.24° | 0.48 | 0.17 | 5.35 | 5.00 to 5.55 | 4.30 | 5.80 |

| | | | | | | | |
|---|---|---|---|---|---|---|---|
| * p=0.05, ° p<0.01 vs. Baseline | | | | | | | |

### Serum Lipids

Total and LDL cholesterol progressively decreased in both cohorts throughout the whole study period. The decrease in total and LDL cholesterol level achieved the nominal significance at week 8 in Cohort 1 and at week 12 in Cohort 2 compared with baseline. The decrease was persistently significant in all subsequent visits up to last available follow up (Tables 13 and 14).

**Table 13: Total cholesterol (mg/dl)**

| Cohort 1 *(n = 10)* | | | | | | | |
|---|---|---|---|---|---|---|---|
| Visit | Mean | SD | SEM | Median | IQR | Min | Max |
| Baseline | 228.40 | 29.75 | 9.41 | 234.00 | 198.00 to 241.00 | 191.00 | 280.00 |
| Week 1 | 231.90 | 55.85 | 17.66 | 236.00 | 190.00 to 246.00 | 150.00 | 357.00 |
| Week 4 | 217.50 | 48.39 | 15.30 | 214.00 | 194.00 to 251.00 | 136.00 | 310.00 |
| Week 8 | 199.70* | 40.80 | 12.90 | 189.00 | 165.00 to 239.00 | 152.00 | 272.00 |
| Week 12 | 204.00° | 33.47 | 10.58 | 194.50 | 177.00 to 236.00 | 160.00 | 257.00 |

| Cohort 2 *(n = 8)* | | | | | | | |
|---|---|---|---|---|---|---|---|
| Visit | Mean | SD | SEM | Median | IQR | Min | Max |
| Baseline | 231.00 | 32.63 | 11.54 | 239.50 | 197.50 to 251.50 | 191.00 | 280.00 |
| Week 1 | 235.38 | 60.98 | 21.56 | 236.00 | 201.50 to 250.50 | 150.00 | 357.00 |
| Week 4 | 214.00 | 53.00 | 18.74 | 211.50 | 180.50 to 241.00 | 136.00 | 310.00 |
| Week 8 | 204.00 | 45.10 | 15.95 | 194.50 | 164.00 to 245.50 | 152.00 | 272.00 |
| Week 12 | 209.50* | 35.50 | 12.55 | 214.50 | 178.00 to 237.00 | 160.00 | 257.00 |
| Week 16 | 195.38* | 42.22 | 14.93 | 195.50 | 159.50 to 228.00 | 143.00 | 254.00 |
| Week 20 | 191.38* | 31.46 | 11.12 | 204.00 | 170.50 to 213.50 | 131.00 | 224.00 |
| Week 24 | 190.88* | 44.68 | 15.80 | 187.00 | 155.00 to 214.50 | 138.00 | 276.00 |

| | | | | | | | |
|---|---|---|---|---|---|---|---|
| * p<0.01 vs. Baseline | | | | | | | |

**Table 14: LDL cholesterol (mg/dl)**

| Cohort 1 *(n = 10)* | | | | | | | |
|---|---|---|---|---|---|---|---|
| Visit | Mean | SD | SEM | Median | IQR | Min | Max |
| Baseline | 148.60 | 38.68 | 12.23 | 132.50 | 113.00 to 179.00 | 111.00 | 219.00 |
| Week 1 | 148.00 | 60.51 | 19.14 | 136.00 | 105.00 to 193.00 | 54.00 | 251.00 |
| Week 4 | 133.86 | 43.94 | 16.61 | 118.00 | 104.00 to 158.00 | 84.00 | 217.00 |
| Week 8 | 122.63° | 39.22 | 13.87 | 111.00 | 98.00 to 135.00 | 85.00 | 208.00 |
| Week 12 | 121.11° | 38.05 | 12.68 | 116.00 | 95.00 to 150.00 | 65.00 | 185.00 |

| Cohort 2 *(n = 8)* | | | | | | | |
|---|---|---|---|---|---|---|---|
| Visit | Mean | SD | SEM | Median | IQR | Min | Max |
| Baseline | 157.75 | 38.01 | 13.44 | 148.50 | 128.50 to 188.50 | 112.00 | 219.00 |
| Week 1 | 153.25 | 66.42 | 23.48 | 150.00 | 107.00 to 203.50 | 54.00 | 251.00 |
| Week 4 | 135.00 | 50.26 | 22.48 | 118.00 | 114.00 to 142.00 | 84.00 | 217.00 |
| Week 8 | 130.83 | 42.21 | 17.23 | 121.00 | 111.00 to 139.00 | 85.00 | 208.00 |
| Week 12 | 127.00° | 41.29 | 15.61 | 116.00 | 95.00 to 162.00 | 65.00 | 185.00 |
| Week 16 | 114.29** | 37.53 | 14.18 | 107.00 | 84.00 to 130.00 | 70.00 | 186.00 |
| Week 20 | 116.43** | 38.50 | 14.55 | 120.00 | 79.00 to 141.00 | 69.00 | 183.00 |
| Week 24 | 119.00* | 52.18 | 18.45 | 109.00 | 79.50 to 148.00 | 64.00 | 215.00 |

| | | | | | | | |
|---|---|---|---|---|---|---|---|
| °p<0.05, *p=0.01, **p<0.01 vs Baseline | | | | | | | |

HDL serum levels tended to increase in both cohorts throughout the whole observation period. The increase, however, failed to achieve the nominal level at each considered time point compared with baseline (Table 15).

**Table 15: HDL cholesterol (mg/dl)**

| Cohort 1 *(n = 10)* | | | | | | | |
|---|---|---|---|---|---|---|---|
| Visit | Mean | SD | SEM | Median | IQR | Min | Max |
| Baseline | 47.50 | 12.94 | 4.09 | 44.00 | 41.00 to 54.00 | 24.00 | 68.00 |
| Week 1 | 55.20 | 34.11 | 10.79 | 45.00 | 39.00 to 51.00 | 32.00 | 149.00 |
| Week 4 | 55.50 | 21.59 | 6.83 | 46.00 | 42.00 to 67.00 | 32.00 | 105.00 |
| Week 8 | 55.40 | 19.51 | 6.17 | 51.00 | 41.00 to 68.00 | 30.00 | 99.00 |
| Week 12 | 54.50 | 19.78 | 6.26 | 51.00 | 44.00 to 63.00 | 27.00 | 101.00 |

| Cohort 2 *(n = 8)* | | | | | | | |
|---|---|---|---|---|---|---|---|
| Visit | Mean | SD | SEM | Median | IQR | Min | Max |
| Baseline | 45.88 | 12.01 | 4.24 | 44.00 | 41.50 to 53.50 | 24.00 | 65.00 |
| Week 1 | 56.13 | 38.04 | 13.45 | 45.00 | 39.50 to 49.50 | 32.00 | 149.00 |
| Week 4 | 54.75 | 22.76 | 8.05 | 46.00 | 43.00 to 61.50 | 32.00 | 105.00 |
| Week 8 | 55.38 | 20.72 | 7.33 | 51.00 | 44.50 to 61.50 | 30.00 | 99.00 |
| Week 12 | 55.38 | 21.39 | 7.56 | 51.00 | 46.00 to 60.50 | 27.00 | 101.00 |
| Week 16 | 54.75 | 22.00 | 7.78 | 51.50 | 43.00 to 63.50 | 24.00 | 98.00 |
| Week 20 | 49.63 | 22.54 | 7.97 | 43.50 | 39.50 to 53.00 | 25.00 | 100.00 |
| Week 24 | 48.38 | 12.94 | 4.57 | 46.50 | 39.50 to 56.00 | 31.00 | 72.00 |

Serum triglyceride levels did not change appreciably throughout the whole observation period (Table 16).

**Table 16: Triglycerides (mg/dl)**

| Cohort 1 *(n = 10)* | | | | | | | |
|---|---|---|---|---|---|---|---|
| Visit | Mean | SD | SEM | Median | IQR | Min | Max |
| Baseline | 136.20 | 84.57 | 26.74 | 103.00 | 77.00 to 231.00 | 45.00 | 283.00 |
| Week 1 | 122.80 | 61.61 | 19.48 | 107.00 | 89.00 to 153.00 | 48.00 | 265.00 |
| Week 4 | 123.70 | 49.84 | 15.76 | 130.00 | 78.00 to 156.00 | 54.00 | 215.00 |
| Week 8 | 122.70 | 44.17 | 13.97 | 119.50 | 102.00 to 147.00 | 49.00 | 204.00 |
| Week 12 | 121.20 | 43.96 | 13.90 | 112.00 | 101.00 to 156.00 | 42.00 | 195.00 |

| Cohort 2 *(n = 8)* | | | | | | | |
|---|---|---|---|---|---|---|---|
| Visit | Mean | SD | SEM | Median | IQR | Min | Max |
| Baseline | 128.38 | 86.63 | 30.63 | 98.50 | 63.00 to 188.00 | 45.00 | 283.00 |
| Week 1 | 124.75 | 64.37 | 22.76 | 107.00 | 94.00 to 141.50 | 48.00 | 265.00 |
| Week 4 | 119.88 | 55.03 | 19.45 | 116.50 | 74.00 to 154.50 | 54.00 | 215.00 |
| Week 8 | 125.63 | 45.71 | 16.16 | 119.50 | 104.00 to 152.50 | 49.00 | 204.00 |
| Week 12 | 123.63 | 46.55 | 16.46 | 112.00 | 105.50 to 158.50 | 42.00 | 195.00 |
| Week 16 | 100.38 | 37.39 | 13.22 | 94.50 | 78.00 to 123.50 | 44.00 | 167.00 |
| Week 20 | 109.38 | 51.22 | 18.11 | 114.00 | 66.00 to 146.00 | 38.00 | 185.00 |
| Week 24 | 117.13 | 66.64 | 23.56 | 112.50 | 51.50 to 168.50 | 51.00 | 221.00 |

### Systolic and Diastolic Blood Pressure

Systolic and diastolic blood pressure did not change appreciably in both cohorts throughout the whole study period (Table 17 and 18).

**Table 17: Systolic Blood Pressure (mmHg)**

| Cohort 1 *(n = 10)* | | | | | | | |
|---|---|---|---|---|---|---|---|
| Visit | Mean | SD | SEM | Median | IQR | Min | Max |
| Baseline | 120.87 | 13.58 | 4.29 | 124.33 | 112.00 to 131.67 | 98.00 | 138.00 |
| Week 1 | 115.83 | 13.27 | 4.20 | 115.17 | 108.00 to 126.00 | 91.67 | 136.00 |
| Week 2 | 120.25 | 14.35 | 4.54 | 124.67 | 104.33 to 133.33 | 98.50 | 134.33 |
| Week 3 | 114.63 | 14.69 | 4.65 | 109.33 | 101.67 to 130.00 | 98.33 | 136.67 |
| Week 4 | 116.17 | 17.07 | 5.40 | 115.50 | 105.00 to 131.67 | 93.00 | 139.33 |
| Week 6 | 113.43 | 15.37 | 4.86 | 115.17 | 101.00 to 125.00 | 89.67 | 136.67 |
| Week 8 | 119.97 | 13.68 | 4.33 | 123.17 | 106.67 to 128.33 | 98.00 | 142.33 |
| Week 10 | 115.42 | 13.49 | 4.77 | 118.17 | 108.17 to 120.50 | 93.00 | 136.67 |
| Week 12 | 119.00 | 17.98 | 6.36 | 115.67 | 108.00 to 129.83 | 94.33 | 150.67 |

| Cohort 2 *(n = 8)* | | | | | | | |
|---|---|---|---|---|---|---|---|
| Visit | Mean | SD | SEM | Median | IQR | Min | Max |
| Baseline | 122.83 | 14.65 | 5.18 | 128.67 | 112.17 to 132.50 | 98.00 | 138.00 |
| Week 1 | 116.71 | 14.72 | 5.21 | 117.17 | 107.33 to 128.50 | 91.67 | 136.00 |
| Week 2 | 122.85 | 14.74 | 5.21 | 131.17 | 110.50 to 133.33 | 98.50 | 134.33 |
| Week 3 | 117.08 | 15.46 | 5.47 | 118.33 | 102.17 to 130.33 | 98.33 | 136.67 |
| Week 4 | 118.67 | 18.40 | 6.51 | 124.17 | 100.17 to 134.17 | 93.00 | 139.33 |
| Week 6 | 115.46 | 16.58 | 5.86 | 121.50 | 100.83 to 126.33 | 89.67 | 136.67 |
| Week 8 | 120.04 | 15.25 | 5.39 | 123.17 | 106.17 to 130.67 | 98.00 | 142.33 |
| Week 10 | 115.42 | 13.49 | 4.77 | 118.17 | 108.17 to 120.50 | 93.00 | 136.67 |
| Week 12 | 119.00 | 17.98 | 6.36 | 115.67 | 108.00 to 129.83 | 94.33 | 150.67 |
| Week 14 | 118.71 | 15.46 | 5.47 | 122.67 | 103.50 to 129.50 | 98.00 | 140.33 |
| Week 16 | 120.46 | 17.30 | 6.12 | 121.17 | 104.50 to 133.33 | 99.00 | 146.67 |
| Week 18 | 113.25 | 13.59 | 4.81 | 113.33 | 105.67 to 124.83 | 88.33 | 130.00 |
| Week 20 | 116.81 | 18.51 | 6.55 | 115.83 | 105.50 to 128.50 | 88.33 | 146.50 |
| Week 22 | 116.00 | 14.51 | 5.13 | 109.67 | 107.50 to 129.50 | 96.67 | 138.00 |
| Week 24 | 118.19 | 21.05 | 7.96 | 116.00 | 103.00 to 138.33 | 87.67 | 142.67 |

**Table 18: Diastolic Blood Pressure (mmHg)**

| Cohort 1 *(n = 10)* | | | | | | | |
|---|---|---|---|---|---|---|---|
| Visit | Mean | SD | SEM | Median | IQR | Min | Max |
| Baseline | 74.87 | 12.77 | 4.04 | 75.67 | 61.33 to 84.33 | 55.33 | 92.33 |
| Week 1 | 73.70 | 11.75 | 3.72 | 75.17 | 64.33 to 80.67 | 54.67 | 91.67 |
| Week 2 | 77.43 | 13.63 | 4.31 | 78.17 | 65.00 to 90.00 | 56.00 | 94.33 |
| Week 3 | 70.13 | 17.53 | 5.54 | 70.33 | 59.00 to 86.33 | 35.33 | 91.67 |
| Week 4 | 72.53 | 14.76 | 4.67 | 75.50 | 57.33 to 87.33 | 55.00 | 92.00 |
| Week 6 | 73.07 | 13.00 | 4.11 | 75.50 | 65.67 to 80.67 | 49.33 | 93.33 |
| Week 8 | 75.37 | 11.42 | 3.61 | 74.00 | 66.67 to 83.33 | 55.00 | 95.00 |
| Week 10 | 74.29 | 15.90 | 5.62 | 77.17 | 72.17 to 82.83 | 38.33 | 91.67 |
| Week 12 | 76.65 | 14.12 | 4.99 | 75.92 | 64.83 to 83.83 | 60.67 | 103.33 |
| Cohort 2 *(n = 8)* | | | | | | | |
| Visit | Mean | SD | SEM | Median | IQR | Min | Max |
| Baseline | 77.83 | 11.94 | 4.22 | 78.83 | 68.33 to 87.50 | 61.00 | 92.33 |
| Week 1 | 75.96 | 10.95 | 3.87 | 77.50 | 67.50 to 83.50 | 59.00 | 91.67 |
| Week 2 | 80.58 | 13.50 | 4.77 | 84.17 | 71.33 to 91.67 | 56.00 | 94.33 |
| Week 3 | 76.21 | 12.35 | 4.37 | 76.17 | 66.17 to 87.17 | 59.00 | 91.67 |
| Week 4 | 76.62 | 13.57 | 4.80 | 79.67 | 64.50 to 87.83 | 57.00 | 92.00 |
| Week 6 | 74.79 | 14.14 | 5.00 | 78.83 | 66.33 to 82.67 | 49.33 | 93.33 |
| Week 8 | 76.54 | 12.46 | 4.40 | 78.00 | 69.17 to 84.00 | 55.00 | 95.00 |
| Week 10 | 74.29 | 15.90 | 5.62 | 77.17 | 72.17 to 82.83 | 38.33 | 91.67 |
| Week 12 | 76.65 | 14.12 | 4.99 | 75.92 | 64.83 to 83.83 | 60.67 | 103.33 |
| Week 14 | 78.38 | 10.02 | 3.54 | 79.67 | 75.50 to 86.17 | 56.67 | 87.67 |
| Week 16 | 74.54 | 13.79 | 4.87 | 76.50 | 67.67 to 80.50 | 50.33 | 96.67 |
| Week 18 | 75.88 | 9.44 | 3.34 | 75.33 | 68.33 to 83.50 | 63.00 | 89.67 |
| Week 20 | 74.65 | 12.18 | 4.30 | 75.83 | 66.83 to 79.67 | 56.00 | 96.50 |
| Week 22 | 75.92 | 11.21 | 3.96 | 75.33 | 66.33 to 84.33 | 63.00 | 92.33 |
| Week 24 | 75.90 | 11.44 | 4.32 | 73.00 | 64.67 to 86.67 | 60.00 | 89.33 |

### 2. Progression to Endpoints

At the time of the present report, two patients had achieved partial remission (24-h urinary protein excretion <3.5grams with at least 50% reduction compared to baseline). The endpoint was achieved at week 12. Three additional patients approximated the endpoint at month six, since their 24-hour proteinuria had decreased to less than 3.5 grams, but the reduction was still less than 50% compared with baseline.

### 3. Safety

Treatment was generally well tolerated. In no case did the treatment have to be discontinued and all patients completed all the planned infusions according to the study protocol guidelines. One episode of transient temporal emyanopsia that fully recovered over two hours was observed during one single infusion in one patient and was interpreted as the clinical manifestation of an emicranic episode. The episode was considered treatment-related and required patient hospitalization. One episode of transient headache was reported in one other patient dating the first two infusions. Both episodes were considered as non-serious and fully recovered spontaneously. During the following treatment administrations the rate of drug infusion was slowest and the event did not recur any longer.

### 4. Discussion

Treatment normalized complement activation in all patients and was safe. Complement inhibition was associated with a significant and clinically relevant improvement of proteinuria, albuminuria, glomerular filtration and sieving function, serum albumin and dyslipidemia (an abnormal amount of lipids (e.g., triglycerides, cholesterol and/or fat phospholipids) in the blood) in nine out of the ten patients included in the study. The fact that the treatment effect was sustained over time for all considered parameters and that the observed changes achieved the nominal significance (despite the relatively small number of patients) provides convincing evidence of the robustness of the study findings. This is further confirmed by the finding that two patients had already achieved partial remission of the nephrotic syndrome at 12 weeks of treatment and that two additional patients were approximating this endpoint at week 24. The finding that changes in serum albumin (and total protein) levels mirrored the changes observed in serum total and LDL cholesterol levels strongly suggests that amelioration of dyslipidemia was mediated by amelioration of hypoalbuminemia, which in turn was sustained by improved kidney sieving function and proteinuria.

An ancillary but intriguing finding was that the increase in GFR was already apparent at week one after inclusion. Although the change failed to achieve the statistical significance, it was consistently observed across all included patients. On the other hand, despite the significant GFR increase, no significant change in serum creatinine levels was observed throughout the whole study period. These findings confirm that serum creatinine is an extremely poor marker of kidney function and that clinically relevant effects can be missed when kidney function is monitored by serum creatinine levels (or creatinine-based GFR estimation formulas), in particular in pathophysiology studies in a relatively small number of patients.

Of note, in the nine responsive patients, baseline serum C5b9 levels largely exceeded (by two to five folds) the limit for inclusion (1000 ng/ml), whereas serum C5b9 level (998 ng/ml) only approximated this limit in the single patient who did not appear to appreciably benefit from treatment. A plausible interpretation of the above findings is that in patients responsive to eculizumab therapy, proteinuria and other disease manifestations were largely sustained by strong complement activation, whereas in the (apparently) non-responsive patient, they were predominantly explained by chronic (remnant-kidney-like) mechanisms. The above data, however, must be interpreted with caution since primary membranoproliferative glomerulonephritis is associated with fast renal function deterioration over time, in particular when the disease is associated with persistent nephrotic syndrome. The finding that in the "non-responsive" patient, the GFR and the other clinical manifestations of the disease did not change appreciably throughout 12-month observation period could be evidence of some treatment benefit.

Altogether, the study findings provide convincing evidence of a strong and clinically relevant benefit of eculizumab therapy in patients with primary membranoproliferative glomerulonephritis, nephrotic syndrome, and complement activation. In the long term, these effects are expected to slow and hopefully halt the progression to end stage kidney disease, and to reduce or prevent the risk of complications of the nephrotic syndrome.

### EXAMPLE 3: "Eagle Study" Extension

Interim analyses of the study described in Example 1 (including the data and results discussed in Example 2) consistently found that Eculizumab treatment normalized complement activation in all patients and was safe. Complement inhibition was associated with a significant and clinically relevant improvement of proteinuria, albuminuria, glomerular filtration and sieving function, serum albumin and dyslipidemia in nine out of the ten patients included in the study. Accordingly, in this extended follow-up of the study described in Example 1, patients completing the first 1-year treatment period and the 3-month Recovery period enter a second 1-year treatment period (Extended Eculizumab treatment), followed by a second 3-month Recovery Period.

### 1. Objectives

The primary objective of this extension study is to evaluate whether re-introduction of Eculizumab therapy may reduce 24-hour proteinuria, considered as a continuous variable, at 6 months (week-24) and 12 months (week-48) of the Eagle Extension as compared to Recovery values.

The co-primary objective is to assess whether over 3- month wash-out from 12-month Eculizumab therapy (Recovery from the EAGLE Study: Recovery Period 1) and over 3-month wash-out from the Extended 1-year Eculizumab Treatment Period (Recovery Period 1) 24-hour urinary protein excretion increases towards baseline, pre-treatment levels.

Secondary objectives include:
i. Assessing whether re-introduction of Eculizumab therapy may again achieve persistent, either complete (defined as 24-hour urinary protein excretion reduction to <0.3 grams for adults and <4mg/h/m2 for children) or partial (defined as 24 hour urinary protein excretion reduction to < 3.5grams with at least 50% reduction from baseline for adults or <40mg/h/m2 with at least 50% reduction from baseline for children) remission of the nephrotic syndrome;
ii. Assessing the effect of Eculizumab therapy on relapses of nephrotic syndrome defined as increase of 24-hour urinary protein excretion to> 3.5 grams for adults or >40mg/h/m2 for children after a period of complete or partial remission;
iii. Assessing the effect of Eculizumab therapy on clinical (body weight, systolic and diastolic blood pressure) and laboratory parameters (urinary albumin/creatinine ratio, serum creatinine, creatinine clearance, serum total proteins, serum albumin, LDL, HDL cholesterol and triglycerides levels, hematocrit and haemoglobin concentration);
iv. Assessing the effect of Eculizumab therapy on renal functional parameters (Glomerular Filtration Rate (GFR) directly measured by the Iohexol plasma clearance technique and estimated by creatinine and cistatin C based estimation formulas, Albumin, IgG, Sodium, Potassium fractional clearance, renal resistivity index by ultrasound evaluation);
v. Assessing the effect of Eculizumab therapy on markers of complement activity (C3, C4, C3a, C5a, Bb and sC5b9);
vi. Assessing the safety profile of the Eculizumab treatment;
vii. Evaluating the cost/effectiveness of the study treatment;
viii. Evaluating biomarkers to be tested in case of significant treatment effect on the primary efficacy variable.

### 2. Patients

Exclusion criteria mirror those of the Eagle Study (see Example 1). Inclusion criteria are as follows:
A. Completion of Eagle Study (see Example 1);
B. Written informed consent (by parents or tutors if underage) to extended Eculizumab treatment.

### 3. Study Design

This Eagle Extension Study is organized in 3-month Recovery Phase (Recovery 1) after completion of the Eagle Study (see Example 1), followed by a second 1-year Extended Eculizumab Treatment period and by a second 3-month Recovery Phase (Recovery 2). Ten patients completing the Eagle Study enter the present Extension Study.

### Recovery Phase 1

After completion of the Eagle study, the parameters evaluated at the final visit of the Eagle study are re-evaluated at 1, 2 and 3 months after eculizumab withdrawal (Recovery period). GFR, albumin, Ig and sodium fractional clearance are evaluated only at the end of the Recovery period (month 3). After completion of the Recovery period, patients receive the first intravenous infusion of Eculizumab and enter a second 1-year Eculizumab treatment period. The investigators, however, will have the possibility to anticipate eculizumab administration before completion of the 3-month recovery period in case of events conceivably related to eculizumab withdrawal that might harm the study patient. These events might include an increase in 24-hour urinary protein excretion to the nephrotic range in patients who had previously achieved complete remission and/or an increase exceeding 50% as compared to level achieved at the end of the 1-year treatment period. Other changes that might indicate anticipated eculizumab administration might include serum creatinine increases (confirmed in at least two consecutive measurements) exceeding by more than 20% the serum creatinine levels observed at the end of the treatment period or other changes in components of the nephrotic syndrome that in the investigator judgment could be harmful for the patient.

### Extended 1-year Eculizumab Treatment Period

During the Extended 1-year treatment period, patients are treated exactly as described for the Eagle study. Evaluations performed at the end of the Recovery Period are repeated at week 24 and at week 48. During the induction phase (4 weeks) safety parameters and markers of complement activity are measured weekly. During the maintenance phase (44 weeks) safety parameters are measured monthly. Additional evaluations are allowed whenever deemed clinically appropriate, in particular for safety reasons.

Additional plasma, serum and urine samples are collected, before the first Eculizumab administration, at week 2, 4, 12, 24, 36 and 48, for the evaluations detailed in Table 19. The samples are stored and, on the basis of the study findings, to explore the mechanisms of the expected effects of Eculizumab on the primary or secondary efficacy variables.

**Table 19: Biomarker Assessments**

| **Purpose** | **Biomarker** | **Matrix** | **Timepoints** |
|---|---|---|---|
| PD | C3/C4 | Serum | D-3, Wks 1,2, 3, 4, 12, 24, 36, 48 and at 3 monthly timepoints following discontinuation of EC |
| PD | C5 | Plasma BP100 | " |
| PD | C5a | Plasma BP100 | " |
| PD | sC5b-9 | Plasma BP100 | " |
| Exploratory PD Alternative Pathway | Ba, Bb | Plasma BP100 | D-3, Wks 2, 4, 12, 24, 36, 48 and at 3 monthly timepoints following discontinuation of EC |
| Exploratory PD Local vs. Systemic | C5a | Urine/protease inhibitor | " |
| Exploratory PD Local vs. Systemic | C5b-9 | Urine/protease inhibitor | " |
| Exploratory PD Alternative Pathway Local vs. Systemic | Ba | Urine/protease inhibitor | " |
| Normalization | Urinary creatinine | Urine/protease inhibitor | " |
| Exploratory PD | Markers of inflammation/platelet or endothelial activation which may include, but are not limited to chemokines or cytokines | Serum Urine/protease inhibitor Plasma BP100 | " |
| Exploratory PD | Markers of inflammation/renal injury, which may include but are not limited to chemokines, cytokines, kidney injury molecule-1, osteopontin, cystatin C, albumin, beta-2-microglobulin | Urine/protease inhibitor | " |
| PD | C3 | IHC | D-2, Wk 48 |
| PD | C4d | IHC | " |
| PD | sC5b-9 | IHC | " |
| PD | IgG | IHC | " |
| Exploratory PD | Other inflammatory markers, which may include but are not limited to CD21, C5aR | IHC | " |

### Recovery Phase 2

After completion of the 1-year Extended Eculizumab Treatment Period, the parameters evaluated at the final visit of the Extended Treatment Period are re-evaluated at 1, 2 and 3 months after eculizumab withdrawal (Recovery Period 2). GFR, albumin, Ig and sodium fractional clearance are evaluated only at the end of the Recovery period (month 3).

### 4. Outcome Variable

Primary efficacy variables include change in 24 hour proteinuria, considered as a continuous variable, at 6 months (week-24) and 12 months (week-48) of the EAGLE Extension as compared to Recovery values.

A co-primary efficacy variable includes changes in 24 hour proteinuria, considered as a continuous variable, during the first and second Recovery periods.

Secondary efficacy variables include: (1) complete or partial remission of the nephrotic syndrome, (2) normalization (reduction to <303 ng/ml) of sC5b-9 plasma levels, (3) normalization in plasma levels of other components of the complement system, including C3, C4, C3a, C5a, and Bb, (4) amelioration of kidney function/perfusion parameters, including measured and estimated glomerular filtration rate (GFR); albumin, IgG, sodium and potassium fractional clearance and renal resistivity index; (5) changes in serum albumin, lipids and other clinical and laboratory parameters.

Safety outcomes include serious and non serious adverse events, including acute reactions during Eculizumab infusion, infectious episodes (including meningoencephalitis). Outcome data and treatment costs will be used for cost/effectiveness analyses.

### 5. Methods

The methods for the Eagle Extension Study mirror those used in the Eagle Study (see Example 1).

### 6. Investigational Medicinal Product (IMP)

The IMP and administration protocol for the Eagle Extension Study mirror those used in the Eagle Study (see Example 1).

The dosing regimen for adult patients (≥18 years of age) consists of a 4 week initial phase (900 mg of Eculizumab via a 25 - 45 minute intravenous infusion every week for the first 4 weeks) followed by a maintenance phase (1200 mg of Eculizumab administered via a 25 - 45 minute intravenous infusion for the fifth week, followed by 1200 mg of Eculizumab administered via a 25 - 45 minute intravenous infusion every 14 ± 2 days).

In pediatric patients (less than 18 years), the Eculizumab dosing regimen consists of:

**Table 20: Pediatric Dosing Regimen**

| **Patient Body Weight** | **Initial Phase** | **Maintenance Phase** |
|---|---|---|
| ≥40 kg | 900 mg weekly x 4 | 1200 mg at week 5; then 1200 mg every 2 weeks |
| 30 - <40 kg | 600 mg weekly x 2 | 900 mg at week 3; then 900 mg every 2 weeks |
| 20 - <30 kg | 600 mg weekly x 2 | 600 mg at week 3; then 600 mg every 2 weeks |
| 10 - <20 kg | 600 mg weekly x 1 | 300 mg at week 2; then 300 mg every 2 weeks |
| 5 - <10 kg | 300 mg Weekly x 1 | 300 mg at week 2; then 300 mg every 3 weeks |

### 7. Statistical Methods

The statistical methods for the Eagle Extension Study mirror those used in the Eagle Study (see Example 1).

### 8. Withdrawal of Patients

The withdrawal protocol for the Eagle Extension Study mirrors the protocol described in the Eagle Study (see Example 1).

### 9. Premature Discontinuation of Study

The premature discontinuation protocol for the Eagle Extension Study mirrors the protocol described in the Eagle Study (see Example 1).

### 10. Adverse Events

The adverse event criteria for the Eagle Extension Study mirrors the criteria described in the Eagle Study (see Example 1).

### EXAMPLE 4: Results of "Eagle Study" Extension

An extension study was conducted substantially according to the protocol described above in Example 3.

### 1. Study Participants

Study participants were identified among patients referred to in the Italian Registry of MPGN of the Clinical Research Center (CRC) for Rare Diseases "Aldo e Cele Daccò" of the IRCCS-Istituto di Ricerche Farmacologiche Mario Negri of Bergamo, in Italy. Subjects who had biopsy-proven MPGN with creatinine clearance >20 ml/min per 1.73m², proteinuria persistently exceeding 3.5 g/24-hours in adults or 40 mg/h/m² (or 2 mg/mg protein/creatinine ratio in spot urine samples) in children, serum C3 levels below the lower limit of normal range and serum sC5b9 levels exceeding 1000 ng/ml (a level exceeding the mean + 10 SD of values in our healthy controls) were included in at least two consecutive measurements. Patients ≥75-year-old, those with evidence of secondary MPGN, too severe chronic kidney histological changes that were not expected to be affected by study treatment, steroid or immunosuppressive therapy over the last six months, or any clinical condition expected to affect completion of the study or confound the study findings were excluded. Exclusion criteria included inability to understand the potential risks and benefits of the study and legal incapacity of patients or their parents or tutors. Pregnant or lactating women or fertile women without effective contraception were not included. Included patients received a conjugated tetravalent meningococcal vaccine against serotypes A, C, Y and W135 and a monovalent vaccine against the serotype B at least two weeks before the first Eculizumab infusion.

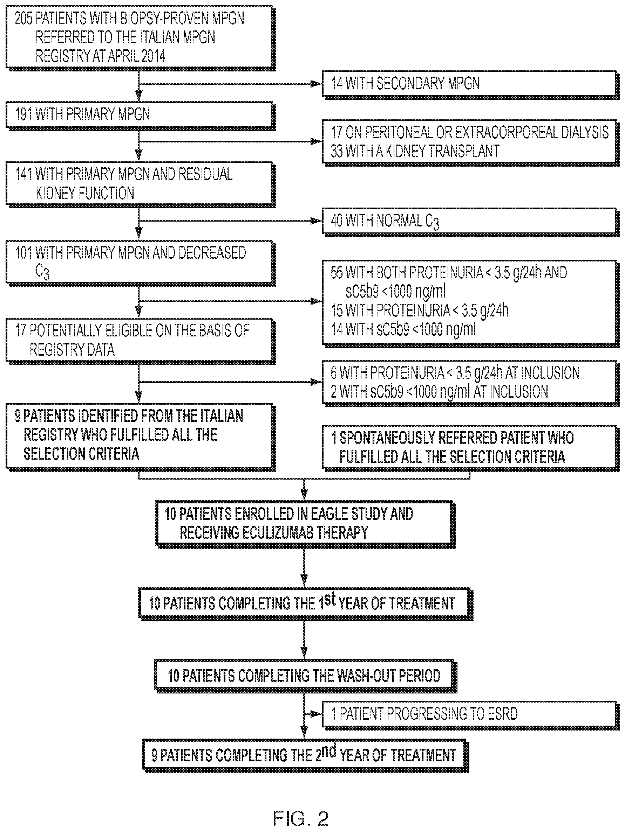

From March 4, 2014 to January 7, 2015 ten patients (six males and four females) were included from six centers, as shown in Figure 2. Six patients had IC-MPGN and four had C3GN. One p.D1625H heterozygous mutation in the C3 gene and one p.R78G homozygous mutation in CFH were identified in two patients with C3GN, respectively. C3 nephritic factors were observed in six patients: three with IC-MPGN and three with C3GN. Age at inclusion ranged from 13 to 33 years and five patients were underage. Baseline characteristics of patients with IC-MPGN and C3GN were similar, as shown in Table 21. No patient was on immunosuppressive treatment.

**Table 21: Baseline Characteristics of Study Patients Considered as a Whole (Overall) and According to Histological Diagnosis (C3GN or IC-MPGN).**

| | Overall *(n=10)* | C3GN *(n=4)* | IC-MPGN *(n=6)* |
|---|---|---|---|
| ***Demography and clinical*** | | | |
| Age *(years)* | 20.0 ± 6.9 | 21.7 ± 8.6 | 18.8 ± 6.1 |
| Gender *(M*/*F)* | 6/4 | 2/2 | 4/2 |
| Weight (Kg) | 59.1 ± 14.6 | 62.3 ± 18.2 | 56.9 ± 13.1 |
| BMI *(Kg*/*m²)* | 21.5 ± 3.3 | 23.3 ± 3.1 | 20.3 ± 3.0 |
| Systolic Blood Pressure | 120.8 ± 13.6 | 127.9 ± 11.2 | 116.1 ± 13.7 |
| Diastolic Blood Pressure | 74.8 ± 12.8 | 83.9 ± 9.8 | 68.8 ± 11.3 |
| Pulse Rate *(bpm)* | 71.4 ± 10.7 | 71.8 ± 11.4 | 71.2 ± 11.3 |

| ***Laboratory Parameters*** | | | |
|---|---|---|---|
| Sc5b9 *(nglml)* | 2420 [1915 to | 3069 [2534 to | 2107 [1693 to |
| Total Cholesterol *(mg*/*dL)* | 228.4 ± 29.7 | 236.3 ± 34.2 | 223.2 ± 28.4 |
| HDL Cholesterol *(mg*/*dL)* | 47.5 ± 12.9 | 50.0 ± 11.5 | 45.8 ± 14.6 |
| LDL Cholesterol *(mg*/*dL)* | 148.6 ± 38.7 | 145.0 ± 50.6 | 151.0 ± 33.8 |
| Triglycerides *(mg*/*dL)* | 103.0 [77.0 to | 160.5 [67.5 to | 103.0 [77.0 to |
| Blood Glucose *(mg*/*dL)* | 88.3 ± 10.8 | 88.8 ± 4.8 | 88.0 ± 14.0 |
| Hemoglobin *(g*/*dL)* | 11.3 ± 1.7 | 10.4 ± 0.6 | 11.8 ± 1.9 |

| | Overall *(n=10)* | C3GN *(n=4)* | IC-MPGN *(n=6)* |
|---|---|---|---|
| Serum Calcium *(mg*/*dL)* | 8.3 ± 0.4 | 8.1 ± 0.4 | 8.5 ± 0.3 |
| Serum Phosphate *(mg*/*dL)* | 5.5 ± 0.7 | 5.2 ± 0.7 | 5.6 ± 0.7 |
| Serum Potassium *(mEq*/*dL)* | 4.7 ± 0.7 | 4.8 ± 1.1 | 4.7 ± 0.5 |
| Serum Creatinine *(mg*/*dL)* | 0.75 [0.44 to | 1.38 [0.75 to | 0.47 [0.41 to |
| Serum Albumin *(g*/*dL)* | 2.4 ± 0.5 | 2.1 ± 0.7 | 2.6 ± 0.3 |
| Serum Proteins *(g*/*dL)* | 4.6 ± 0.8 | 4.2 ± 1.0 | 4.9 ± 0.5 |

| ***Kidney Function parameters*** | | | |
|---|---|---|---|
| Measured GFR | 78.0 [30.7 to | 44.8 [29.0 to | 91.5 [78.0 to |
| Estimated GFR | 139.0 [35.2 to | 68.7 [31.4 to | 219.9 [57.5 to |
| Urinary Creatinine *(mg*/*24h)* | 1130.7 [903 to | 1197.1 [769 to | 1130.7 [1040 to |
| Urinary Protein *(g*/*24h)* | 6.1 [4.8 to 11.6] | 9.9 [4.7 to 14.3] | 5.5 [4.8 to 6.2] |
| Urinary Albumin *(µg*/*min)* | 3199 [2302 to | 4625 [2218 to | 2854 [2302 to |
| Urinary Sodium *(mEq*/*24h)* | 107.4 [92.5 to | 107.4 [94.4 to | 125.6 [67.6 to |
| Albumin Fractional Clearance | 237.7 [116.5 to | 653.5 [356.9 to | 116.5 [85.7 to |
| IgG Fractional Clearance | 42.3 [22.6 to | 292.4 [110.8 to | 22.6 [12.9 to |

| - ***Antihypertensive agents*** | | | |
|---|---|---|---|
| - Diuretics | 9 (90%) | 4 (100%) | 5 (83%) |
| - Calcium-channel blockers | 6 (60%) | 3 (75%) | 3 (50%) |
| - Beta blockers | 3 (30%) | 1 (25%) | 2 (33%) |
| - ACE inhibitors or ARBs | 10 (100%) | 4 (100%) | 6 (100%) |

| *- **Lipid-lowering agents:*** | | | |
|---|---|---|---|
| - Any | 7 (70%) | 3 (75%) | 4 (67%) |
| - Statins | 7 (70%) | 3 (75%) | 4 (67%) |
| - Omega-3 fatty acid | 1 (10%) | 1 (25%) | 0 |
| - Ezetimibe | 1 (10%) | 0 | 1 (17%) |

| | | | |
|---|---|---|---|
| By the iohexol plasma clearance technique; ° by the CKD-EPI equation. Data are means ± SD, median [IQR] or counts. | | | |

### 2. Study Design

This pilot, phase-2, single arm, prospective, open, longitudinal study was organized in two 48-week treatment periods with eculizumab divided by a 12-week wash-out period in the context of an OFF-ON-OFF-ON design (see, *e.g.,* van der Lee JH, et al., J. Clin. Epidemiol. 2008;61:324-30 and Gupta S, et al., J. Clin. Epidemiol. 2011;64:1085-94). An optional, voluntary kidney biopsy was proposed at inclusion and study end to patients who, in the investigator judgment, had no contraindications to the procedure. All baseline clinical and laboratory measurements were centralized at the CRC. Three consecutive 24-hour urine collections were submitted to measure protein, albumin, sodium, urea and phosphate excretion. The median of the three measurements was recorded. sC5b-9 plasma, C3 and C4 serum levels and routine laboratory parameters were measured in the morning after an overnight fasting. Glomerular filtration rate (GFR) was directly measured by the iohexol plasma clearance technique (see, *e.g.,* Gaspari F, et al., J. Am. Soc. Nephrol. 1995;6:257-63) and estimated with the serum-creatinine-based Chronic Kidney Disease-Epidemiology (CKD-Epi) equation. IgG and albumin fractional clearances were calculated by standard formulas. Then patients were transferred to the Unit of Nephrology of the Azienda Socio Sanitaria Territoriale (ASST) Ospedale Papa Giovanni XXIII of Bergamo where they received the first intravenous infusion of eculizumab. Adults and underage patients who weighted 40 kg or more received 900 mg of eculizumab every week for four weeks (Induction period), 1200 mg at week 5 and then 1200 mg every 14±2 days (Maintenance period) up to completion of 48 weeks of treatment. The dosing regimen of the drug in children who weighted five to less than 40 kg is shown in Table 2. A second identical 48-week treatment course was started after 12-week eculizumab withdrawal ("washout period"). The clinical and laboratory parameters evaluated at baseline were centrally re-assessed at weeks 1, 24 and 48 of the first treatment period, 12 weeks after treatment withdrawal, and at weeks 24 and 48 of the second treatment period. The same parameters, with the exception of GFR and albumin and IgG fractional clearances, were evaluated at each reference center at week 12 and 36 of both treatment periods. sC5b-9 plasma and C3 and C4 serum levels were centrally evaluated at each study visit. Kidney biopsies were performed and evaluated. No systematic change in diet or concomitant medications was allowed during the study.

### 3. Sample Size Estimation and Statistical Analyses

This was a pilot, exploratory study in a very rare disease and the sample size was calculated on the basis on the number of potentially available patients during a pre-defined recruitment period of approximately one year. Continuous outcome variables were evaluated with paired t test, Wilcoxon signed rank test, Repeated Measures ANOVA or linear mixed-effect models which included pre-defined baseline covariates, as appropriate. McNemar's test or chi-square test or Fisher's Exact test were used for categorical variables. Baseline characteristics were presented as numbers and percentages, means and SDs, or medians and interquartile ranges (IQRs). The multiple comparisons issue was addressed by means of Bonferroni adjustment. Normality for continuous variables was assessed by means of the Q-Q plot and Shapiro-Wilk test. All p values were two sided.

### 4. Safety

All patients completed the planned infusions. The patient progressing to ESRD stopped eculizumab at the first dialysis session. Overall, there were acute reactions during eight of 69 eculizumab infusions (11.6%). In all cases, symptoms recovered spontaneously and without sequelae. Acute chest pain and nausea ensued in one patient during the first infusion. The patient did not require hospitalization and the event was considered as non-serious. Headache with blurred vision and transient temporal hemianopsia ensued in the same patient at week 20 of the first treatment period. The patient was hospitalized for one day and the event was categorized as serious. Five other episodes of headache, one with blurred vision, and one case of hypotension were observed during eculizumab infusion. All events were non-serious. During the washout period one patient was hospitalized because of a pneumococcal pneumonia associated with transient worsening of kidney function. The event was considered be serious and treatment-related. He fully recovered with antimicrobial therapy.

During the washout, proteinuria increased in all patients. According to pre-defined protocol-guidelines treatment with eculizumab was anticipated in three patients because of worsening of kidney function.

### 5. Outcomes/Results

Primary efficacy outcome was 24-hour urinary protein excretion at 24 and 48 weeks of the first treatment period. sC5b-9 plasma levels were measured to monitor terminal complement pathway activity. GFR and albumin and IgG fractional clearances and progression to complete (24-hour proteinuria <0.3 grams) or partial (24-hour proteinuria <3.5 grams with >50% reduction from baseline) remission of the nephrotic syndrome were among secondary outcome.

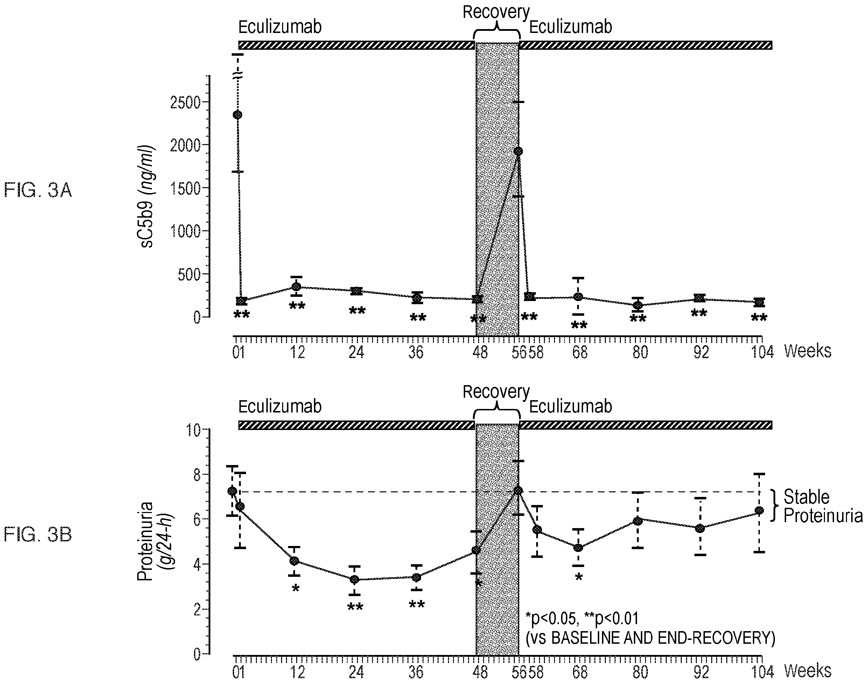

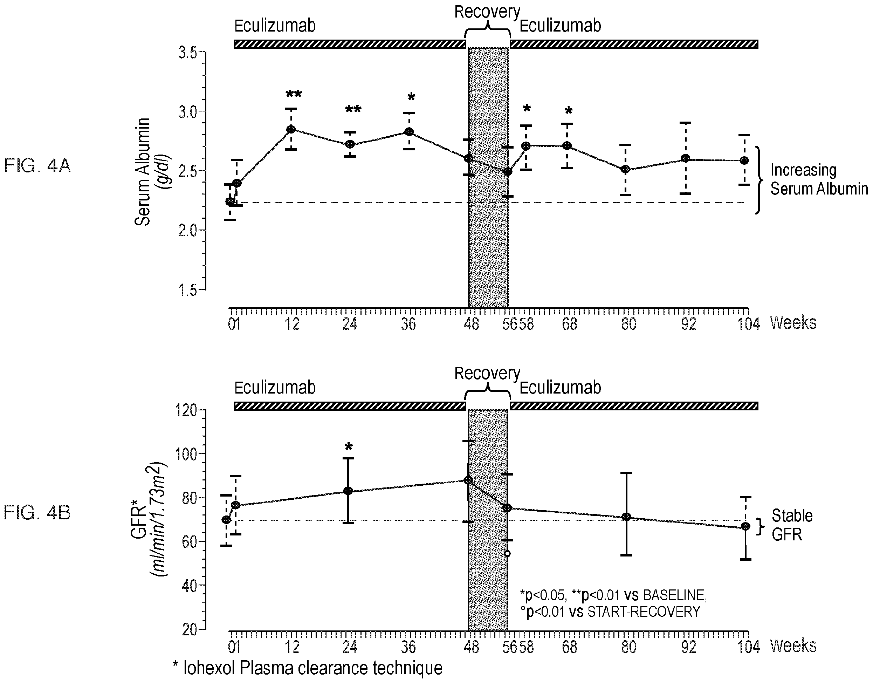

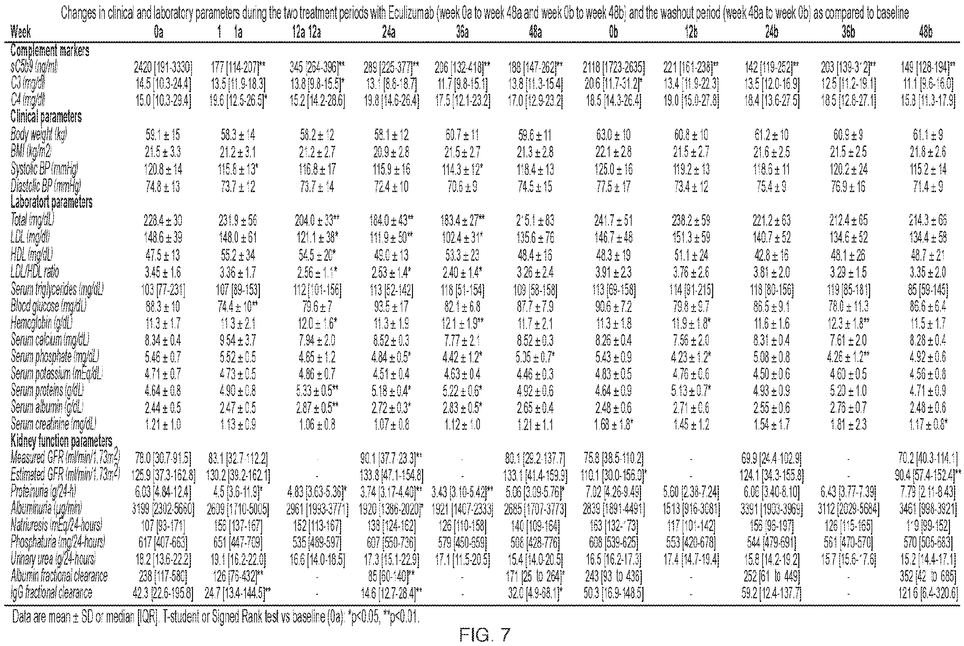

Figure 7 sets forth the changes in clinical and laboratory parameters during the two treatment periods with eculizumab (week 0a to week 48a and week 0b to week 48b) and the washout period (week 48a to week 0b), as compared to baseline. Results of the analyses of serum C5b9 levels are also reported in Table 22 and Figure 3A, along with data on the primary efficacy variable, 24-hour urinary protein excretion (see Tables 23-25 and Figure 3B) and other key efficacy variables, including serum 24-hour urinary albumin excretion (Tables 26-27 and Figure 4A), measured glomerular filtration rate (GFR) (Tables 28-29 and Figure 4B), albumin and IgG fractional clearances (Tables 30-31), serum creatinine (Table 32), albumin (Table 33), total proteins (Table 34), total, LDL and IIDL cholesterol (Tables 35-37), triglyceride levels (Table 38), and sytolic and diastolic blood pressure (Tables 39-40).

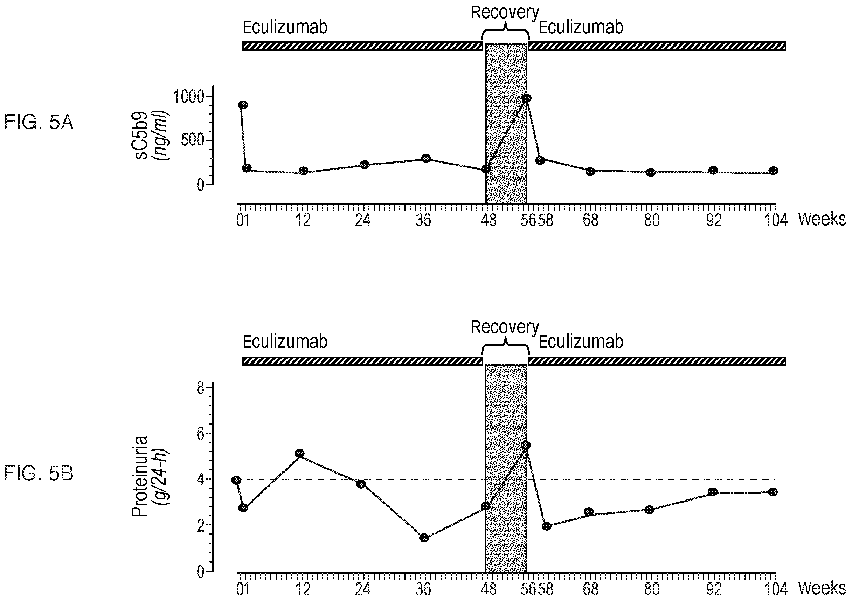

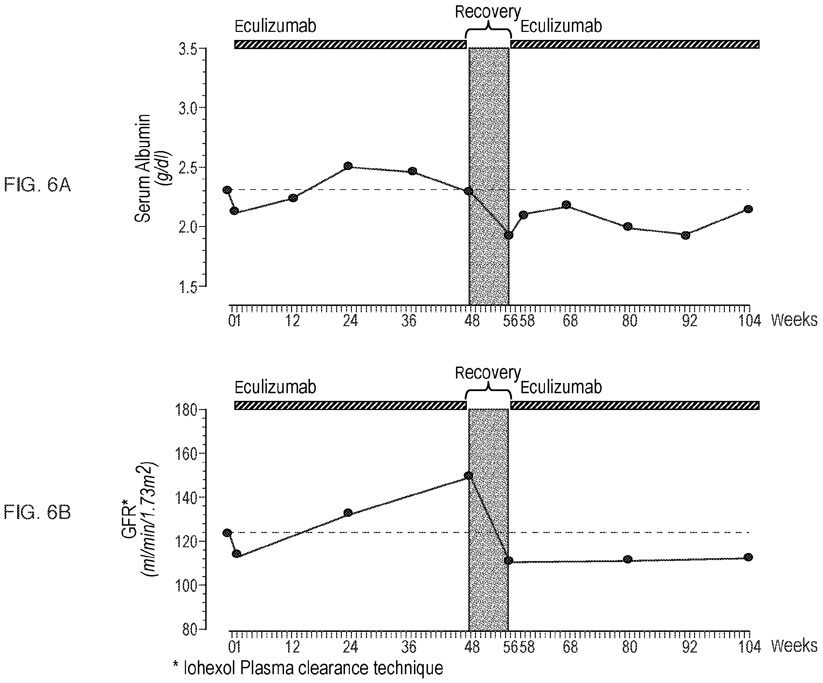

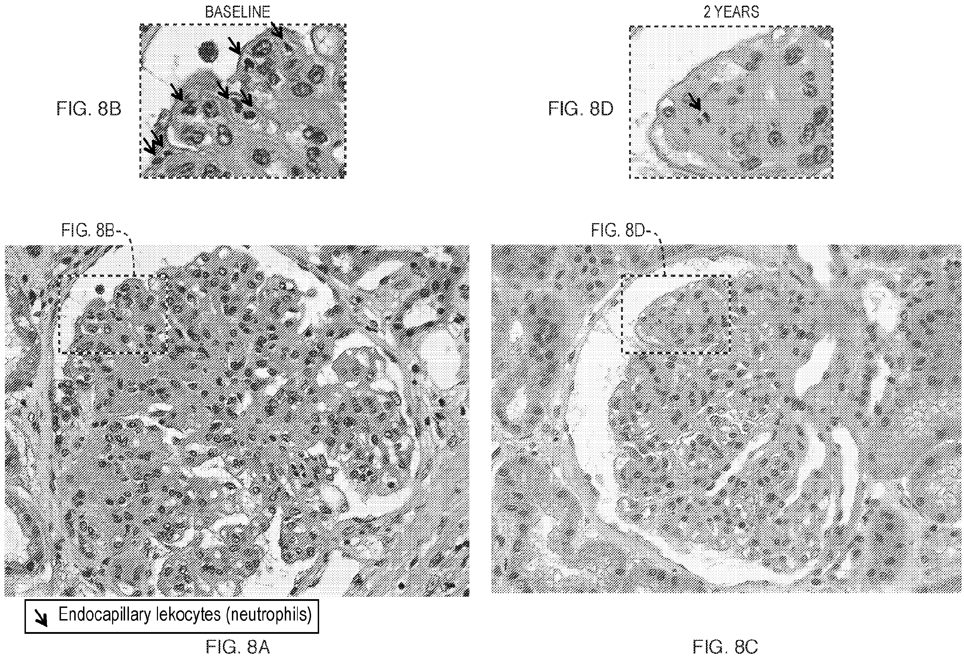

Serum C5b9 levels, 24-hour urinary protein excretion, serum 24-hour urinary albumin excretion, and measured glomerular filtration rate for one apparent "non-responder" are shown in Figures 5A, 5B, 6A, and 6B, respectively. Histology for the "non-responder" is shown in Figures 8A-8D.

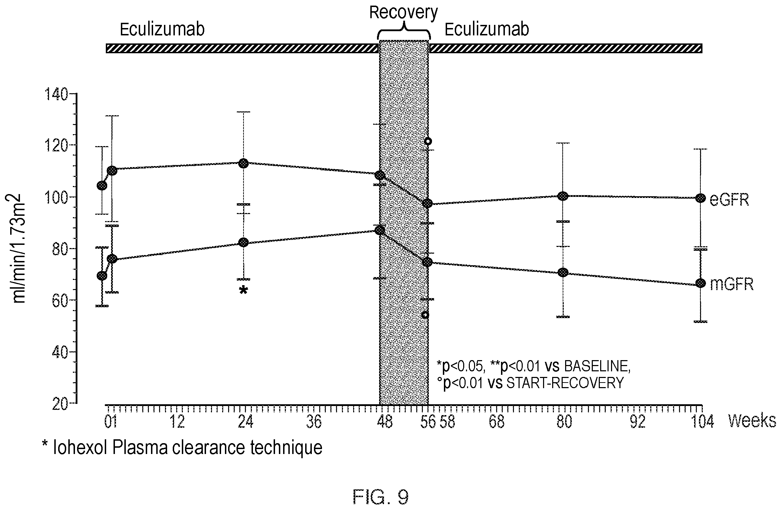

Figure 9 depicts the estimated (eGFR) and measured glomerular filtration rates (mGFR) over the course of treatment (1 year), recovery (3 months), and the extension phase (1 year) for eculizumab treated patients.

**Table 22: Serum sC5b9 (ng/mL)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 3099.4 | 2072.6 | 655.4 | 2420.2 | 1915.7 to 3330.6 | 987.9 | 8130.5 |
| V02 | 1 Week | 10 | 186.9 | 82.4 | 26.0 | 176.7** | 114.4 to 206.6 | 86.2 | 347.9 |
| V09 | 12 Weeks | 10 | 423.5 | 347.7 | 110.0 | 345.2** | 264.4 to 395.6 | 139.9 | 1387.0 |
| V15 | 24 Weeks | 10 | 304.3 | 102.4 | 32.4 | 289.4** | 224.9 to 377.5 | 174.1 | 474.7 |
| V21 | 36 Weeks | 10 | 268.5 | 165.1 | 52.2 | 206.2** | 132.3 to 418.3 | 108.9 | 527.5 |
| V27 | 48 Weeks - R | 10 | 213.9 | 106.1 | 33.5 | 188.1** | 147.0 to 262.3 | 106.2 | 469.9 |
| V31 | R - Baseline 2Y | 9 | 2423.0 | 1628.0 | 542.7 | 1938.4°° | 1722.8 to 2423.5 | 1034.4 | 6441.4 |
| V35 | 2 Weeks | 9 | 233.5 | 122.9 | 41.0 | 217.2** | 130.8 to 318.2 | 103.4 | 415.4 |
| V39 | 12 Weeks | 9 | 415.7 | 638.3 | 212.8 | 221.4** | 161.5 to 237.5 | 135.4 | 2110.5 |
| V45 | 24 Weeks | 10 | 188.8 | 114.9 | 36.3 | 141.9** | 118.8 to 251.8 | 88.9 | 454.3 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 23: 24-h Urinary protein excretion (grams), Individual Patients**

| *Patient No, Diagnosis, NeF** | **0a** | **1a** | **12a** | **24a** | **36a** | **48a** | **0b** | **12b** | **24b** | **36b** | **48b** |
|---|---|---|---|---|---|---|---|---|---|---|---|
| *A, IC-MPGN, Pos* | 3.83 | 2.96 | 4.78 | 4.06 | 2.23 | 3.09 | 5.83 | 4.22 | 3.40 | 3.77 | 3.81 |
| *B, C3GN, Pos* | 14.82 | 14.80 | 5.36 | 4.40 | 5.42 | 5.38 | 8.57 | 7.80 | 6.44 | 7.39 | 8.86 |
| *C, IC-MPGN, Neg* | 6.19 | 3.85 | 4.90 | 3.43 | 3.25 | 5.58 | 13.65 | 5.46 | 8.23 | 9.93 | 16.24 |
| *D, IC-MPGN, Pos* | 4.95 | 1.68 | 2.25 | 1.12 | 1.21 | 0.92 | 3.98 | 2.38 | 2.07 | 0.62 | 1.80 |
| *E, C3GN, Pos* | 13.72 | 11.86 | 8.02 | 7.85 | 5.64 | 8.54 | 9.49 | 6.83 | 13.41 | - | - |
| *F, IC-MPGN, Pos* | 12.41 | 12.66 | 5.96 | 5.04 | 5.51 | 8.03 | 8.22 | 7.88 | 8.10 | 6.43 | 8.43 |
| *G, IC-MPGN, Neg* | 6.00 | 4.93 | 3.63 | 3.17 | 5.05 | 5.75 | 10.67 | 7.24 | 7.25 | 6.89 | 7.79 |
| *H, C3GN, Neg* | 6.06 | 5.17 | 4.87 | 3.36 | 3.10 | 3.94 | 4.04 | 2.05 | 1.85 | 2.18 | 1.32 |
| *I, C3GN, Pos* | 3.28 | 4.00 | 4.29 | 4.35 | 3.53 | 4.73 | 5.42 | 5.75 | 5.51 | 10.13 | 8.14 |
| *J, IC-MPGN, Neg* | 4.84 | 3.61 | 2.41 | 2.77 | 3.34 | 2.13 | 4.26 | 1.89 | 5.67 | 5.61 | 2.12 |
| **Mean ± SD** | 7.61 ± 4.31 | 6.55 ± 4.68 | 4.65 ± 1.69 | 3.95 ± 1.74 | 3.83 ± 1.52 | 4.81 ± 2.41 | 7.41 + 3.26 | 5.15 ± 2.38 | 6.19 ± 3.42 | 5.88 ± 3.24 | 6.50 ± 4.80 |
| *P (t-student)* | - | *0.0464** | *0.0223** | *0.0051*** | *0.0003*** | *0.0256** | *0.4579* | *0.1602* | *0.5280* | *0.8615* | *0.8040* |
| **Median [IQR]** | 6.03 9;10.9] | 4.47 [3.6;11.9] | 4.83 [3.8; 5.3] | 3.74 [3.2; 4.4] | 3.44 [3.1; 5.3] | 5.06 [3.3; 5.7] | 7.02 [4.6; 9.3] | 5.60 [2.8; 7.11 | 6.06 [3.9; 7.9] | 6.43 [3.8; 7.41 | 7.79 [2.1; 8.4] |
| *P (signed rank)* | - | *0.0488** | *0.0371** | *0.0098*** | *0.0039*** | *0.0371** | *0.5566* | *0.1934* | *0.5566* | *0.8203* | *0.7344* |

| | | | | | | | | | | | |
|---|---|---|---|---|---|---|---|---|---|---|---|
| **Weeks** Nef--Nephritic factor. Pos=positive; Neg= negative | | | | | | | | | | | |

**Table 24: 24-h Urinary protein excretion (grams)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 7.61 | 4.31 | 1.36 | 6.03 | 4.84 to 12.41 | 3.28 | 14.82 |
| V02 | 1 Week | 10 | 6.55* | 4.68 | 1.48 | 4.47 | 3.61 to 11.86 | 1.68 | 14.80 |
| V09 | 12 Weeks | 10 | 4.65* | 1.69 | 0.53 | 4.83 | 3.63 to 5.36 | 2.25 | 8.02 |
| V15 | 24 Weeks | 10 | 3.95** | 1.74 | 0.55 | 3.74 | 3.17 to 4.40 | 1.12 | 7.85 |
| V21 | 36 Weeks | 10 | 3.83** | 1.52 | 0.48 | 3.44 | 3.10 to 5.42 | 1.21 | 5.64 |
| V27 | 48 Weeks - R | 10 | 4.81* | 2.41 | 0.76 | 5.06 | 3.09 to 5.75 | 0.92 | 8.54 |
| V31 | R - Baseline 2Y | 10 | 7.41°° | 3.26 | 1.03 | 7.02 | 4.26 to 9.49 | 3.98 | 13.65 |
| V35 | 2 Weeks | 10 | 5.51 | 3.12 | 0.99 | 6.03 | 2.43 to 6.93 | 1.61 | 12.08 |
| V39 | 12 Weeks | 10 | 5.15* | 2.38 | 0.75 | 5.60 | 2.38 to 7.24 | 1.89 | 7.88 |
| V45 | 24 Weeks | 10 | 6.19 | 3.42 | 1.08 | 6.06 | 3.40 to 8.10 | 1.85 | 13.41 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 25: 24-h Urinary protein excretion (grams)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 7.52 | 4.07 | 1.29 | 6.12 | 4.84 to 11.63 | 3.30 | 14.55 |
| V02 | 1 Week | 10 | 6.59 | 4.77 | 1.51 | 4.51 | 3.43 to 11.41 | 1.98 | 16.08 |
| V09 | 12 Weeks | 10 | 4.54* | 1.78 | 0.56 | 4.52 | 2.96 to 5.36 | 2.27 | 8.02 |
| V15 | 24 Weeks | 10 | 3.77** | 1.79 | 0.56 | 3.66 | 2.73 to 4.40 | 1.01 | 7.85 |
| V21 | 36 Weeks | 10 | 3.87** | 1.52 | 0.48 | 3.72 | 3.36 to 5.40 | 1.00 | 5.64 |
| V27 | 48 Weeks - R | 10 | 4.93* | 2.48 | 0.79 | 4.93 | 3.16 to 6.06 | 1.18 | 9.03 |
| V31 | R - Baseline 2Y | 10 | 7.48° | 3.33 | 1.05 | 6.71 | 4.31 to 9.49 | 4.02 | 13.82 |
| V35 | 2 Weeks | 10 | 5.70 | 3.14 | 0.99 | 6.15 | 2.54 to 6.93 | 1.58 | 12.08 |
| V39 | 12 Weeks | 10 | 5.03* | 2.41 | 0.76 | 5.89 | 2.38 to 6.83 | 1.89 | 7.88 |
| V45 | 24 Weeks | 10 | 6.31 | 3.60 | 1.14 | 6.80 | 3.12 to 8.03 | 1.34 | 13.29 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 26: 24-h Urinary Albumin Excretion (mg)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 3726.6 | 1950.1 | 616.7 | 3133.7 | 2433.0 to 6069.0 | 1339.3 | 6781.0 |
| V02 | 1 Week | 10 | 3216.6 | 2106.0 | 666.0 | 2512.3* | 1716.7 to 5494.3 | 904.0 | 6953.3 |
| V09 | 12 Weeks | 9 | 2851.4 | 1046.6 | 348.9 | 2831.7 | 2024.3 to 3509.3 | 1274.3 | 4645.0 |
| V15 | 24 Weeks | 10 | 1952.2 | 749.4 | 237.0 | 1996.2** | 1611.7 to 2115.3 | 568.3 | 3536.5 |
| V21 | 36 Weeks | 8 | 1833.0 | 779.7 | 275.7 | 1820.8* | 1401.0 to 2349.6 | 499.5 | 3021.5 |
| V27 | 48 Weeks - R | 10 | 2490.1 | 1202.3 | 380.2 | 2683.0* | 1575.3 to 3461.3 | 469.0 | 4117.7 |
| V31 | R - Baseline 2Y | 8 | 3429.3 | 1731.9 | 612.3 | 3218.0° | 1833.8 to 4520.5 | 1700.0 | 6589.7 |
| V35 | 2 Weeks | 8 | 2699.8 | 1668.2 | 589.8 | 2501.8 | 1371.8 to 3814.8 | 630.6 | 5591.0 |
| V39 | 12 Weeks | 7 | 2068.0 | 1001.8 | 390.0 | 1972.0* | 915.5 to 2843.8 | 899.7 | 3592.7 |
| V45 | 24 Weeks | 9 | 3011.3 | 1481.7 | 493.9 | 3266.7* | 1707.3 to 3705.0 | 819.3 | 5594.3 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 27: 24-h Urinary Albumin Excretion (mg)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 3623.7 | 1877.7 | 593.8 | 3199.0 | 2302.0 to 5660.0 | 1238.0 | 6765.0 |
| V02 | 1 Week | 10 | 3279.5 | 2135.8 | 675.4 | 2609.0 | 1710.0 to 5005.0 | 1086.0 | 7505.0 |
| V09 | 12 Weeks | 9 | 2968.8 | 1180.4 | 393.5 | 2961.0 | 1993.0 to 3770.8 | 1127.0 | 4645.0 |
| V15 | 24 Weeks | 10 | 1861.0 | 753.9 | 238.4 | 1919.5** | 1386.0 to 2020.0 | 622.0 | 3536.5 |
| V21 | 36 Weeks | 8 | 1855.1 | 780.1 | 275.8 | 1920.5* | 1406.5 to 2332.7 | 499.5 | 3021.5 |
| V27 | 48 Weeks - R | 10 | 2553.9 | 1223.5 | 386.9 | 2684.5* | 1707.0 to 3773.0 | 592.0 | 4129.0 |
| V31 | R - Baseline 2Y | 8 | 3357.1 | 1739.4 | 615.0 | 2838.5° | 1890.5 to 4491.0 | 1745.0 | 6672.0 |
| V35 | 2 Weeks | 8 | 2833.6 | 1704.5 | 602.6 | 2730.0 | 1411.5 to 4087.9 | 618.8 | 5591.0 |
| V39 | 12 Weeks | 7 | 2002.4 | 1065.3 | 402.6 | 1512.8* | 915.5 to 3081.0 | 906.0 | 3213.0 |
| V45 | 24 Weeks | 9 | 3066.6 | 1477.9 | 492.6 | 3391.0 | 1903.0 to 3969.0 | 778.0 | 5440.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 28: Glomerular Filtration Rate (ml/min/1.73 m)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 9 | 62.7 | 30.1 | 10.0 | 65.3 | 34.8 to 79.8 | 23.8 | 119.1 |
| V02 | 1 Week | 9 | 68.0 | 30.4 | 10.1 | 78.0 | 36.5 to 89.5 | 23.3 | 108.3 |
| V15 | 24 Weeks | 9 | 76.0* | 37.8 | 12.6 | 74.5 | 42.2 to 97.6 | 22.0 | 133.7 |
| V27 | 48 Weeks - R | 9 | 79.9 | 47.8 | 15.9 | 82.4 | 29.5 to 109.8 | 21.1 | 150.9 |
| V31 | R - Baseline 2Y | 8 | 70.5° | 38.2 | 13.5 | 71.3 | 38.8 to 104.8 | 17.6 | 116.9 |
| V45 | 24 Weeks | 8 | 68.1 | 44.4 | 15.7 | 69.6 | 25.9 to 98.1 | 17.1 | 140.5 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 29: Glomerular Filtration Rate (ml/min/1.73 m²) norm**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 9 | 69.7 | 35.2 | 11.7 | 78.0 | 30.7 to 91.5 | 27.3 | 123.4 |
| V02 | 1 Week | 9 | 76.5 | 38.2 | 12.7 | 83.1 | 32.7 to 112.2 | 26.9 | 122.8 |
| V15 | 24 Weeks | 9 | 83.3* | 43.9 | 14.6 | 90.1 | 37.7 to 123.3 | 25.3 | 137.9 |
| V27 | 48 Weeks - R | 9 | 87.4 | 55.1 | 18.4 | 80.1 | 29.2 to 137.7 | 24.2 | 164.4 |
| V31 | R - Baseline 2Y | 8 | 75.8° | 42.7 | 15.1 | 75.8 | 38.5 to 110.2 | 20.1 | 137.5 |
| V45 | 24 Weeks | 8 | 71.2 | 48.6 | 17.2 | 69.9 | 24.4 to 102.9 | 19.7 | 155.8 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 30: Albumin Fractional Clearance**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 9 | 384.6 | 358.4 | 119.5 | 237.7 | 116.5 to 580.1 | 72.3 | 1111.7 |
| V02 | 1 Week | 9 | 276.4 | 254.0 | 84.7 | 125.7** | 75.8 to 431.9 | 38.9 | 737.0 |
| V15 | 24 Weeks | 9 | 116.4 | 87.2 | 29.1 | 84.5** | 60.3 to 139.9 | 14.8 | 279.2 |
| V27 | 48 Weeks - R | 8 | 183.9 | 176.0 | 62.2 | 170.7* | 25.4 to 264.1 | 18.3 | 532.5 |
| V31 | R - Baseline 2Y | 7 | 309.3 | 295.7 | 111.8 | 243.3° | 92.6 to 435.8 | 62.2 | 901.7 |
| V45 | 24 Weeks | 7 | 328.0 | 272.7 | 103.1 | 252.3 | 60.7 to 448.5 | 59.0 | 851.4 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 31: IgG Fractional Clearance**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 9 | 139.0 | 166.7 | 55.6 | 42.3 | 22.6 to 195.8 | 10.3 | 435.5 |
| V02 | 1 Week | 9 | 85.4 | 96.6 | 32.2 | 24.7** | 13.4 to 144.5 | 3.9 | 250.5 |
| V15 | 24 Weeks | 9 | 31.7 | 36.7 | 12.2 | 14.6** | 12.7 to 28.4 | 4.0 | 120.6 |
| V27 | 48 Weeks - R | 8 | 51.1 | 64.6 | 22.9 | 32.0 | 4.9 to 68.1 | 3.0 | 196.0 |
| V31 | R - Baseline 2Y | 7 | 111.0 | 150.7 | 57.0 | 50.3 ° | 16.9 to 148.5 | 8.7 | 433.0 |
| V45 | 24 Weeks | 7 | 131.4 | 198.5 | 75.0 | 59.1 | 12.4 to 137.7 | 7.0 | 570.6 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 32: Serum creatinine (mg/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 1.21 | 0.97 | 0.31 | 0.75 | 0.44 to 1.76 | 0.36 | 3.05 |
| V02 | 1 Week | 10 | 1.13 | 0.89 | 0.28 | 0.73 | 0.44 to 1.69 | 0.25 | 2.84 |
| V09 | 12 Weeks | 10 | 1.06 | 0.81 | 0.26 | 0.68 | 0.41 to 1.88 | 0.31 | 2.40 |
| V15 | 24 Weeks | 10 | 1.07 | 0.84 | 0.26 | 0.69 | 0.43 to 1.88 | 0.32 | 2.63 |
| V21 | 36 Weeks | 10 | 1.12 | 1.02 | 0.32 | 0.61 | 0.38 to 1.99 | 0.31 | 3.20 |
| V27 | 48 Weeks - R | 10 | 1.21 | 1.09 | 0.34 | 0.68 | 0.44 to 1.95 | 0.38 | 3.72 |
| V31 | R - Baseline 2Y | 10 | 1.68 | 1.79 | 0.57 | 0.92 | 0.53 to 2.11 | 0.38 | 5.90 |
| V35 | 2 Weeks | 10 | 1.35 | 1.20 | 0.38 | 0.75 | 0.48 to 2.10 | 0.39 | 4.00 |
| V39 | 12 Weeks | 10 | 1.45 | 1.20 | 0.38 | 1.20 | 0.43 to 2.03 | 0.38 | 4.10 |
| V45 | 24 Weeks | 10 | 1.54 | 1.66 | 0.53 | 0.79 | 0.50 to 2.22 | 0.37 | 5.75 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 33: Serum Albumin (g/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 2.29 | 0.53 | 0.17 | 2.36 | 2.20 to 2.65 | 1.00 | 2.83 |
| V02 | 1 Week | 10 | 2.39 | 0.57 | 0.18 | 2.44 | 2.23 to 2.80 | 1.00 | 3.00 |
| V09 | 12 Weeks | 9 | 2.85** | 0.51 | 0.17 | 2.90 | 2.58 to 3.19 | 2.04 | 3.60 |
| V15 | 24 Weeks | 10 | 2.72** | 0.32 | 0.10 | 2.79 | 2.54 to 2.95 | 2.07 | 3.13 |
| V21 | 36 Weeks | 10 | 2.83* | 0.47 | 0.15 | 2.71 | 2.47 to 3.30 | 2.15 | 3.50 |
| V27 | 48 Weeks - R | 10 | 2.61 | 0.49 | 0.15 | 2.70 | 2.26 to 3.05 | 1.70 | 3.20 |
| V31 | R - Baseline 2Y | 10 | 2.48 | 0.63 | 0.20 | 2.38 | 1.91 to 3.00 | 1.77 | 3.70 |
| V35 | 2 Weeks | 10 | 2.70* | 0.60 | 0.19 | 2.52 | 2.19 to 3.10 | 2.05 | 3.66 |
| V39 | 12 Weeks | 10 | 2.71* | 0.61 | 0.19 | 2.78 | 2.23 to 3.11 | 1.68 | 3.60 |
| V45 | 24 Weeks | 10 | 2.53 | 0.62 | 0.20 | 2.35 | 1.99 to 2.90 | 1.94 | 3.72 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 34: Serum protein (g/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 4.56 | 0.77 | 0.24 | 4.65 | 4.40 to 5.20 | 2.80 | 5.40 |
| V02 | 1 Week | 10 | 4.87 | 0.76 | 0.24 | 4.95 | 4.60 to 5.20 | 3.00 | 5.90 |
| V09 | 12 Weeks | 10 | 5.33** | 0.54 | 0.17 | 5.25 | 4.90 to 5.80 | 4.50 | 6.10 |
| V15 | 24 Weeks | 10 | 5.18** | 0.44 | 0.14 | 5.15 | 4.90 to 5.50 | 4.30 | 5.80 |
| V21 | 36 Weeks | 10 | 5.22* | 0.63 | 0.20 | 5.30 | 4.70 to 5.70 | 4.30 | 6.10 |
| V27 | 48 Weeks - R | 10 | 4.91 | 0.62 | 0.20 | 5.05 | 4.40 to 5.30 | 3.80 | 5.90 |
| V31 | R - Baseline 2Y | 10 | 4.64 | 0.86 | 0.27 | 4.45 | 4.00 to 5.40 | 3.60 | 6.10 |
| V35 | 2 Weeks | 10 | 5.22** | 0.85 | 0.27 | 5.20 | 4.40 to 5.90 | 3.90 | 6.30 |
| V39 | 12 Weeks | 10 | 5.13* | 0.67 | 0.21 | 5.25 | 4.30 to 5.70 | 4.30 | 6.10 |
| V45 | 24 Weeks | 10 | 4.90 | 0.88 | 0.28 | 4.70 | 4.10 to 5.70 | 4.00 | 6.50 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 35: Total cholesterol (mg/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 228.4 | 29.7 | 9.4 | 234.0 | 198.0 to 241.0 | 191.0 | 280.0 |
| V02 | 1 Week | 10 | 231.9 | 55.9 | 17.7 | 236.0 | 190.0 to 246.0 | 150.0 | 357.0 |
| V09 | 12 Weeks | 10 | 204.0** | 33.5 | 10.6 | 194.5 | 177.0 to 236.0 | 160.0 | 257.0 |
| V15 | 24 Weeks | 10 | 184.0** | 43.0 | 13.6 | 179.0 | 150.0 to 207.0 | 137.0 | 276.0 |
| V21 | 36 Weeks | 10 | 183.4** | 26.9 | 8.5 | 179.5 | 164.0 to 215.0 | 138.0 | 216.0 |
| V27 | 48 Weeks - R | 10 | 215.1 | 83.3 | 26.3 | 184.0 | 164.0 to 245.0 | 144.0 | 429.0 |
| V31 | R - Baseline 2Y | 10 | 241.7 | 51.2 | 16.2 | 229.5 | 211.0 to 285.0 | 170.0 | 327.0 |
| V35 | 2 Weeks | 10 | 259.3 | 95.5 | 30.2 | 241.5 | 195.0 to 301.0 | 154.0 | 490.0 |
| V39 | 12 Weeks | 10 | 238.2 | 59.3 | 18.8 | 237.0 | 191.0 to 275.0 | 146.0 | 326.0 |
| V45 | 24 Weeks | 10 | 221.2 | 62.5 | 19.8 | 217.5 | 176.0 to 261.0 | 125.0 | 341.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 36: HDL cholesterol (mg/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 47.5 | 12.9 | 4.1 | 44.0 | 41.0 to 54.0 | 24.0 | 68.0 |
| V02 | 1 Week | 10 | 55.2 | 34.1 | 10.8 | 45.0 | 39.0 to 51.0 | 32.0 | 149.0 |
| V09 | 12 Weeks | 10 | 54.5 | 19.8 | 6.3 | 51.0 | 44.0 to 63.0 | 27.0 | 101.0 |
| V15 | 24 Weeks | 10 | 49.0 | 12.9 | 4.1 | 46.5 | 39.0 to 59.0 | 31.0 | 72.0 |
| V21 | 36 Weeks | 10 | 53.3 | 22.8 | 7.2 | 49.5 | 42.0 to 63.0 | 26.0 | 109.0 |
| V27 | 48 Weeks - R | 10 | 48.4 | 15.7 | 4.9 | 49.0 | 35.0 to 52.0 | 28.0 | 76.0 |
| V31 | R - Baseline 2Y | 10 | 48.3 | 19.1 | 6.0 | 48.0 | 29.0 to 62.0 | 25.0 | 84.0 |
| V35 | 2 Weeks | 10 | 53.3 | 24.4 | 7.7 | 51.5 | 37.8 to 62.0 | 17.0 | 109.0 |
| V39 | 12 Weeks | 10 | 51.1 | 24.3 | 7.7 | 49.0 | 32.8 to 59.0 | 23.0 | 106.0 |
| V45 | 24 Weeks | 10 | 42.8 | 16.1 | 5.1 | 39.5 | 30.0 to 53.0 | 22.0 | 78.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 37: LDL cholesterol (mg/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 148.6 | 38.7 | 12.2 | 132.5 | 113.0 to 179.0 | 111.0 | 219.0 |
| V02 | 1 Week | 10 | 148.0 | 60.5 | 19.1 | 136.0 | 105.0 to 193.0 | 54.0 | 251.0 |
| V09 | 12 Weeks | 9 | 121.1* | 38.1 | 12.7 | 116.0 | 95.0 to 150.0 | 65.0 | 185.0 |
| V15 | 24 Weeks | 10 | 111.9** | 49.7 | 15.7 | 101.5 | 66.0 to 125.0 | 59.0 | 215.0 |
| V21 | 36 Weeks | 7 | 102.4* | 31.1 | 11.8 | 112.0 | 72.0 to 134.0 | 59.0 | 135.0 |
| V27 | 48 Weeks - R | 10 | 135.6 | 76.4 | 24.1 | 113.5 | 86.0 to 160.0 | 64.0 | 324.0 |
| V31 | R - Baseline 2Y | 9 | 146.7 | 47.6 | 15.9 | 164.0 | 112.0 to 175.0 | 64.0 | 216.0 |
| V35 | 2 Weeks | 9 | 155.2 | 62.5 | 20.8 | 133.0 | 112.0 to 177.0 | 88.0 | 280.0 |
| V39 | 12 Weeks | 9 | 151.3 | 59.0 | 19.6 | 128.0 | 106.0 to 189.0 | 77.8 | 251.0 |
| V45 | 24 Weeks | 10 | 140.7 | 51.8 | 16.4 | 142.0 | 91.0 to 174.0 | 79.0 | 218.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 38: Triglycerides (mg/dl)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 136.2 | 84.6 | 26.7 | 103.0 | 77.0 to 231.0 | 45.0 | 283.0 |
| V02 | 1 Week | 10 | 122.8 | 61.6 | 19.5 | 107.0 | 89.0 to 153.0 | 48.0 | 265.0 |
| V09 | 12 Weeks | 10 | 121.2 | 44.0 | 13.9 | 112.0 | 101.0 to 156.0 | 42.0 | 195.0 |
| V15 | 24 Weeks | 10 | 114.7 | 61.5 | 19.5 | 112.5 | 52.0 to 142.0 | 51.0 | 221.0 |
| V21 | 36 Weeks | 10 | 113.3 | 68.8 | 21.7 | 118.0 | 51.0 to 154.0 | 32.0 | 238.0 |
| V27 | 48 Weeks - R | 10 | 117.9 | 70.3 | 22.2 | 109.0 | 58.0 to 158.0 | 37.0 | 256.0 |
| V31 | R - Baseline 2Y | 10 | 134.2 | 102.8 | 32.5 | 112.5 | 69.0 to 158.0 | 27.0 | 393.0 |
| V35 | 2 Weeks | 10 | 182.8 | 103.2 | 32.6 | 159.5 | 84.0 to 296.0 | 74.0 | 342.0 |
| V39 | 12 Weeks | 10 | 151.4 | 88.0 | 27.8 | 114.0 | 91.0 to 215.0 | 45.0 | 296.0 |
| V45 | 24 Weeks | 10 | 118.0 | 51.2 | 16.2 | 117.5 | 80.0 to 156.0 | 41.0 | 206.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 39: Systolic Blood Pressure (mmHg)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 120.8 | 13.6 | 4.3 | 124.0 | 112.0 to 131.7 | 98.0 | 138.0 |
| V02 | 1 Week | 10 | 115.8* | 13.3 | 4.2 | 115.2 | 108.0 to 126.0 | 91.7 | 136.0 |
| V09 | 12 Weeks | 10 | 116.8 | 16.6 | 5.3 | 111.2 | 106.7 to 123.0 | 94.3 | 150.7 |
| V15 | 24 Weeks | 10 | 115.9 | 16.5 | 5.2 | 116.2 | 104.3 to 134.0 | 87.7 | 138.3 |
| V21 | 36 Weeks | 10 | 114.3* | 12.1 | 3.8 | 114.3 | 105.3 to 121.3 | 95.0 | 136.0 |
| V27 | 48 Weeks - R | 10 | 118.4 | 13.2 | 4.2 | 116.5 | 106.7 to 126.7 | 102.7 | 143.0 |
| V31 | R - Baseline 2Y | 9 | 125.0 | 16.5 | 5.5 | 120.0 | 115.0 to 135.0 | 103.0 | 149.0 |
| V35 | 2 Weeks | 10 | 116.5 | 8.9 | 2.8 | 115.5 | 113.0 to 120.0 | 100.0 | 132.0 |
| V39 | 12 Weeks | 10 | 119.2 | 12.8 | 4.0 | 120.0 | 107.0 to 131.0 | 97.0 | 133.0 |
| V45 | 24 Weeks | 10 | 118.7 | 10.9 | 3.5 | 118.0 | 115.0 to 122.0 | 98.0 | 143.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

**Table 40: Diastolic Blood Pressure (mmHg)**

| **Visit** | **Period** | **N** | **Mean** | **SD** | **SE** | **Median** | **IQR** | **Min** | **Max** |
|---|---|---|---|---|---|---|---|---|---|
| V01 | Baseline 1Y | 10 | 74.8 | 12.8 | 4.0 | 75.5 | 61.0 to 84.3 | 55.3 | 92.3 |
| V02 | 1 Week | 10 | 73.7 | 11.8 | 3.7 | 75.2 | 64.3 to 80.7 | 54.7 | 91.7 |
| V09 | 12 Weeks | 10 | 73.7 | 13.9 | 4.4 | 70.9 | 63.3 to 79.3 | 59.7 | 103.3 |
| V15 | 24 Weeks | 10 | 72.4 | 10.4 | 3.3 | 72.7 | 63.0 to 79.0 | 60.0 | 89.3 |
| V21 | 36 Weeks | 10 | 70.6 | 9.0 | 2.8 | 72.0 | 63.7 to 76.7 | 56.3 | 84.7 |
| V27 | 48 Weeks - R | 10 | 74.5 | 15.5 | 4.9 | 74.7 | 66.7 to 86.7 | 43.7 | 97.0 |
| V31 | R - Baseline 2Y | 9 | 77.5 | 17.2 | 5.7 | 75.0 | 69.0 to 89.0 | 46.0 | 102.0 |
| V35 | 2 Weeks | 10 | 69.4* | 15.8 | 5.0 | 75.2 | 58.0 to 83.0 | 42.7 | 89.0 |
| V39 | 12 Weeks | 10 | 73.4 | 11.7 | 3.7 | 72.0 | 70.0 to 83.0 | 47.0 | 87.0 |
| V45 | 24 Weeks | 10 | 75.4 | 8.7 | 2.7 | 77.0 | 69.0 to 78.0 | 62.0 | 94.0 |

| | | | | | | | | | |
|---|---|---|---|---|---|---|---|---|---|
| T-Student: *p < 0.05 **p< 0.01 vs. Baseline; °p < 0.05 °°p< 0.01 vs. Start Recovery | | | | | | | | | |

Body weight, BMI and blood pressure were relatively stable throughout the study. sC5b-9 plasma levels, which were extremely elevated at inclusion, promptly and persistently normalized during the first 48-week treatment period, recovered towards baseline values at the end of the washout period and again normalized during the second 48-week treatment period up to study end (Figure 3A and Figure 7). Despite a transient increase at the end of the washout period, C3 serum levels were persistently reduced throughout the whole study period. C4 serum levels were stable and always in normal range.

Proteinuria significantly decreased during the first treatment period and sharply increased towards baseline values at the end of the washout period. This trend to increase was stopped and reverted during the second treatment period, but proteinuria reduction never achieved the nominal significance up to study end (Figure 3A, Figure 7, and Table 23). Two patients achieved partial remission of the nephrotic syndrome at completion of both treatment periods and one additional patient achieved the endpoint at completion of the second treatment period (see Table 23). Changes in albuminuria and albumin and IgG fractional clearances paralleled the change in proteinuria (see Figure 7). Consistently, serum albumin and total proteins increased during the first treatment period slightly decreased during the washout period and did not differ significantly from baseline during the second treatment period (Figures 4 and 7). Total, LDL and LDL/HDL cholesterol significantly decreased during the first treatment period, recovered towards baseline during the washout period and never differed from baseline during the second treatment period. Serum triglycerides levels as well as 24-hour urinary sodium, urea and phosphate excretion were stable throughout the study.

Measured GFR transiently increased at week 24 of the first treatment period, decreased towards baseline values after the washout period and did not differ appreciably from baseline throughout the second treatment period. Estimated GFR showed a similar trend but largely overestimated true GFR.

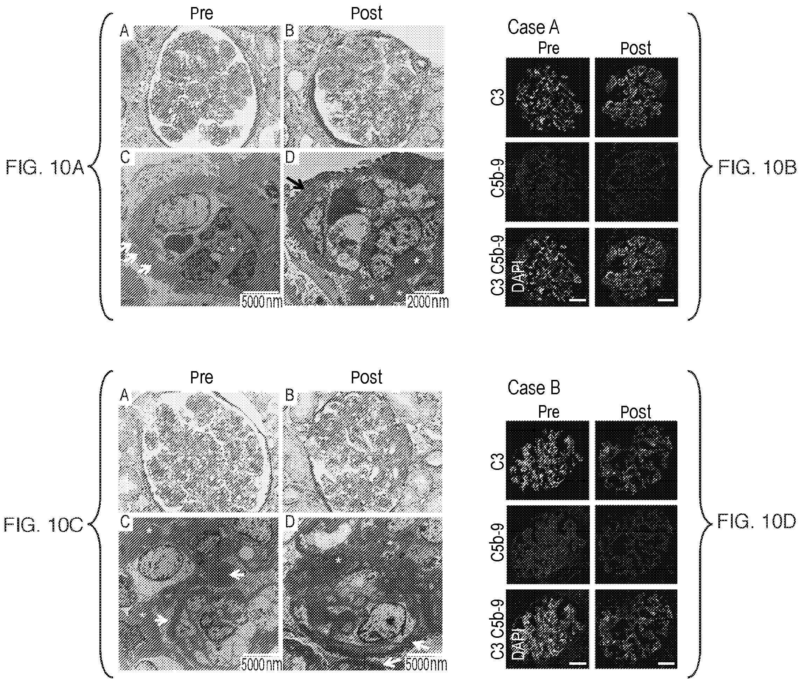

Voluntary kidney biopsies were available at inclusion and at a study end from two patients with IC-MPGN. In both cases, baseline kidney biopsy showed initial glomerular lobulation with moderate mesangial proliferation and exudative features including focally severe endocapillary proliferation with neutrophils infiltration (Figure 10A, 10B, 10C, and 10D).

For "Case A", baseline kidney biopsy showed initial glomerular lobulation, segmental duplication of the GBM, moderate mesangial proliferation and exudative features including focally severe endocapillary proliferation with neutrophils infiltration (Figure 10A-Pre). In addition, one glomerulus exhibited a tuft-to-capsule adhesion with associated segmental sclerosis. Sparse inflammatory infiltrates were observed in the interstitium. Immunofluorescence evaluation showed marked C3 and C5b-9 glomerular parietal deposits (3+) with a similar pattern and distribution (Figure 10B-Pre), together with glomerular parietal IgM, IgG and C1q (2+) and less intense kappa and lambda light chains (1+). Electron microscopy detected frequent intramembranous and focal subendothelial electron dense deposits. The mesangium was expanded due to accumulation of scattered electron dense deposits, increased cellularity, and matrix (Figure 10A-Pre). At repeat biopsy inflammatory features improved, with reduced mesangial and endocapillary proliferation, but increased mesangial matrix, more accentuated glomerular lobulation, multiple adhesions to Bowman's capsule and an increase in segmental glomerular sclerosis from 6% at baseline to 30%. Sparse interstitial inflammatory infiltrates observed in the first biopsy were replaced by focal interstitial fibrosis and tubular atrophy (Figure 10A-Post). The intensity of immunofluorescence staining for C3 did not change appreciably at repeat biopsy. Conversely median (IQR) staining for C5b-9 significantly (p=0.019) decreased from 15.8% (15.2 to 16.5%) at baseline to 10.7% (8.5 to 15.0 %] at post treatment biopsy (Figure 10B-Post). C1q deposits almost disappeared. At repeat biopsy intramembranous and subendothelial deposits were more electron-dense than at baseline evaluation, GBM was diffusely duplicated and isolated powdery subendothelial deposits appeared de novo (Figure 10B-Post). There was a slightly increase in the number of mesangial deposits.

For "Case B", pre-treatment biopsy showed mild mesangial proliferation and diffuse endocapillary proliferation with marked neutrophils infiltration, conferring a vaguely lobulated appearance to the glomeruli. In addition, there was focal moderate tubulointerstitial inflammation (Figure 10C-Pre). Immunofluorescence showed diffuse C3 and C5b-9 glomerular deposits (3+) with a similar pattern and distribution, together with glomerular parietal IgM staining of mild intensity (1+) (Figure 8D-Pre). Electron microscopy detected frequent subendothelial electron dense deposits accompanied by focal duplication of the GBM and occasional intramembranous band-like electron dense deposits. The mesangium was expanded due to accumulation of scattered electron dense deposits, increased cellularity, and matrix (Figure 10C-Pre). As observed in the aforementioned IC-MPGN case, post treatment repeat-biopsy showed amelioration of inflammatory features, with reduced mesangial and endocapillary proliferation, but increased mesangial matrix, more accentuated glomerular lobulation, adhesions to Bowman's capsule with an increase in segmental glomerular sclerosis from 15% at baseline to 40%) (Figure 8C-Post). Tubulointerstitial damage did not change appreciably as compared to baseline. Also the pattern and intensity of immunofluorescence staining for C3 and IgM were similar between the two biopsies. Conversely staining for C5b-9 significantly (p=0.021) decreased from 23.6% (22.7 to 24.9%) at baseline to 18.22% (14.8 to 20.6 %) at post treatment biopsy (Figure 10D-Post). The posttreatment biopsy was notable also for the deposition of segmental IgG and kappa light chain (1+) in glomerular capillaries and more electron-dense subendothelial and intramembranous deposits, along with a mild decrease of mesangial deposits. Some subendothelial deposits had a punctuate powdery texture similar to those described in aforementioned IC-MPGN case. In addition, occasional scattered electron dense deposits were identified in glomerular subepithelial location, in the tubular basement membranes and Bowman's capsule (Figure 10C-Post).

### 6. Discussion

As evidenced by the data described above, eculizumab fully inhibited fluid phase complement activity, reduced proteinuria, improved serum albumin levels, and stabilized the GFR over the two-year follow up. However, the treatment effect was fully exhausted during the three-month treatment withdrawal (Recovery Period). In addition, re-exposure to Eculizumab after the Recovery Period did not appear to be as effective as initial treatment.

The fact that changes in fluid phase complement activity paralleled changes in all considered clinical parameters provided convincing evidence of a causal relationship between eculizumab-induced complement inhibition and treatment effect. Conceivably, the extent of fluid phase complement activation reflects disease activity and may help predict response to eculizumab therapy.

Specifically, in ten patients with strong activation of the terminal complement pathway and nephrotic range proteinuria, sC5b-9 plasma levels promptly and fully normalized during the first eculizumab treatment period, recovered towards baseline after eculizumab withdrawal and, again, promptly and persistently normalized during the second treatment period. C3 serum levels were persistently reduced, whereas C4 levels were in normal range during the whole study period. 24-hour urinary protein excretion (primary efficacy variable of the study) significantly and persistently decreased throughout the first treatment period and sharply increased towards baseline after eculizumab withdrawal. During the second treatment period, the first administration of eculizumab stopped and actually reversed the trend of proteinuria to increase observed during the washout period. At subsequent visits, however, proteinuria was numerically, but non-significantly, lower than at baseline. Notably, two patients achieved partial remission of the nephrotic syndrome at the end of the first treatment period and, despite disease relapse after eculizumab withdrawal, again achieved partial remission during the second period. One additional patient achieved the endpoint at the end of the second treatment period. Finding that both albumin and IgG fractional clearances significantly decreased during the first treatment period and recovered towards baseline after eculizumab withdrawal provided convincing evidence that the antiproteinuric effect of eculizumab was at least in part mediated by improved selectivity of the glomerular barrier to plasma macromolecules (see, e.g., Ruggenenti P, Cravedi P, Remuzzi G. Mechanisms and treatment of CKD. J Am Soc Nephrol 2012;23:1917-28).

The reduction in proteinuria observed during the first treatment period was associated with an increase in serum albumin and total protein levels and a significant and clinically relevant reduction in serum total and low density lipoprotein (LDL) cholesterol levels. Again, these effects weaned after treatment withdrawal. Body weight and body mass index, blood pressure and 24-hour urinary sodium, phosphate and urea excretion, as well as concomitant treatment with RAS inhibitors or statins did not change throughout the study. Thus, amelioration of proteinuria and of signs of the nephrotic syndrome during the first treatment period appeared to reflect a genuine effect of eculizumab therapy that was unlikely confounded by changes in diet or concomitant treatments.

Thus, stratification on the basis of sC5b-9 plasma levels allowed identifying patients with activation of the terminal complement pathway who could benefit from treatment with a blocker of the C5 convertase such as eculizumab. Evidence of effect in patients with overt nephrotic syndrome may have clinical implications since these are the patients at highest risk of accelerated progression to ESRD (see, *e.g.,* Riedl et al., Pediatr. Nephrol. 2017;32:43-57 and Appel GB, et al., J. Am. Soc. Nephrol. 2005;16:1392-403). Amelioration of dysprotidemia and dyslipidemia might also translate into a reduction in the excess risk of cardiovascular events that invariably associates with the nephrotic syndrome (see Vaziri ND, et al., Kidney Int. 2016;90:41-52). On the other hand, the sharp increase in proteinuria and sC5b-9 plasma levels and the concomitant worsening of markers of the nephrotic syndrome during treatment washout is consistent with a rebound of the disease, that required anticipated initiation of the second treatment period in two patients. Finding that the antiproteinuric effect of eculizumab was attenuated after this rebound, suggests that treatment should not be stopped, at least in patients with evidence of initial clinical benefit.

The question remains why the antiproteinuric effect was restricted to the first treatment period. Finding that sC5b-9 plasma levels were normalized during both treatment periods reasonably excludes the possibility that the inhibitory effect of eculizumab on the terminal complement pathway progressively exhausted during the study. This hypothesis was consistent with finding that in both patients who consented to voluntary pre and post treatment kidney biopsies, glomerular staining for C5b-9 was significantly decreased at repeat biopsy as compared to baseline. Notably, reduced C5b-9 deposition associated with an amelioration of the glomerular inflammatory lesions that tended to be replaced by chronic changes. Unfortunately, there is no histology data at the end of the washout period to assess whether the increases in sC5b-9 plasma levels and proteinuria were associated with a rebound of sC5B9 deposition and glomerular inflammation. Thus, the hypothesis that reactivation of the disease after eculizumab withdrawal translated into further histology damage that failed to recover during the second course of eculizumab therapy is conceivable, but unproven.

Despite transient disease reactivation during eculizumab washout, kidney function was stable for at least two years. This finding is robust because it was based on direct GFR measurements rather than on serum creatinine-based GFR prediction equations that, as observed in the present study, fail to provide a reliable estimate of the GFR, in particular in patients with the nephrotic syndrome (see Hofstra JM, et al., Nephrol. Dial Transplant 2011;26:550-6). Thus, despite the lack of a control group does not allow to definitely conclude for a direct cause-and-effect relationship between eculizumab therapy and the observed outcome, the data suggest that patients were protected from the accelerated renal function loss that characterizes patients with MPGN and the nephrotic syndrome (see Riedl M, et al., Pediatr. Nephrol. 2017;32:43-57).

Another interesting finding of the study was that whereas sC5b-9 plasma levels and glomerular deposition were reduced by eculizumab, C3 serum levels and glomerular staining were not. These data are consistent with the hypothesis that MPGN is driven by a dysregulation of the C3 convertase of the alternative complement pathway resulting in accelerated C3 consumption and glomerular deposition of C3 activation fragments, that cannot be directly affected by targeted C5 inhibition (see Herlitz LC, et al., J. Am. Soc. Nephrol. 2012;23:1229-37). This may explain why in reported cases, including those described in the present study, eculizumab never achieved complete remission of the disease (see, *e.g.,* Daina E, et al., N. Engl. J. Med. 2012;366:1161-3, Vivarelli M, Pasini A et al, N. Engl. J. Med. 2012;366:1163-5, Radhakrishnan et al., N. Engl. J. Med 2012;366:1165-6, Bomback AS, et al., Clin. J. Am. Soc. Nephrol. 2012;7:748-56, and Le Quintrec M, et al., Am. J. Kidney Dis. 2018). Conceivably, new molecules under clinical development, such as factor D or Factor B inhibitors that target the C3 convertase of the alternative complement pathway might prove more nephroprotective than C5 antagonists and hopefully change the treatment paradigm for most patients with MPGN (see Ricklin D, et al., Semin. Immunol. 2016;28:208-22).

In conclusion, eculizumab may have a role in the treatment of patients with IC-MPGN or C3GN and terminal complement activation, as it appears that treatment with eculizumab may help to slow or even prevent progression of the disease, possibly by ameliorating glomerular inflammation. Treatment withdrawal, however, associates with a rebound of the disease that might off-set the possible long-term benefit of persistent C5 blockade. Stratification on the basis of sC5b-9 plasma levels and, possibly, C5b-9 deposition in renal tissue, might be a rational approach to identify patients who might benefit the most of eculizumab therapy. Moreover, continuous, chronic therapy is needed, since even transient treatment withdrawal associates with a rebound of disease activity, which does not appear to fully recover after treatment re-exposure.

| **SEQUENCE SUMMARY** |
|---|
| **SEQ ID NO:1** |
| amino acid sequence of heavy chain CDR1 of Eculizumab (as defined under combined Kabat-Chothia definition) |
| GYIFSNYWIQ |
| **SEQ ID NO:2** |
| amino acid sequence of heavy chain CDR2 of Eculizumab (as defined under Kabat definition) |
| EILPGSGSTEYTENFKD |
| **SEQ ID NO:3** |
| amino acid sequence of the heavy chain CDR3 of Eculizumab (as defined under combined Kabat definition). |
| YFFGSSPNWYFDV |
| **SEQ ID NO:4** |
| amino acid sequence of the light chain CDR1 of Eculizumab (as defined under Kabat definition) |
| GASENIYGALN |
| **SEQ ID NO:5** |
| amino acid sequence of light chain CDR2 of Eculizumab (as defined under Kabat definition) |
| GATNLAD |
| **SEQ ID NO:6** |
| amino acid sequence of light chain CDR3 of Eculizumab (as defined under Kabat definition) |
| QNVLNTPLT |
| **SEQ ID NO:7** |
| amino acid sequence of heavy chain variable region of Eculizumab |
| |
| **SEQ ID NO:8** |
| amino acid sequence of light chain variable region of Eculizumab, BNJ441 antibody, and BNJ421 antibody |
| |
| **SEQ ID NO:9** |
| amino acid sequence of heavy chain constant region of Eculizumab and BNJ421 antibody |
| ASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKC CVECPPCPAPPVAGPSVFLFPPKPKDTLMISRTPEVTCVVVDVSQEDPEVQF NWYVDGVEVHNAKTKPREEQFNSTYRVVSVLTVLHQDWLNGKEYKCKV SNKGLPSSIEKTISKAKGQPREPQVYTLPPSQEEMTKNQVSLTCLVKGFYPS DIAVEWESNGQPENNYKTTPPVLDSDGSFFLYSRLTVDKSRWQEGNVFSCS VMHEALHNHYTQKSLSLSLGK |
| **SEQ ID NO:10** |
| amino acid sequence of entire heavy chain of Eculizumab |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVMHEALHNHYTQKSLSLSLGK |
| **SEQ ID NO:11** |
| amino acid sequence of entire light chain of Eculizumab, BNJ441 antibody, and BNJ421 antibody |
| |
| **SEQ ID NO:12** |
| amino acid sequence of heavy chain variable region of BNJ441 antibody and BNJ421 antibody |
| |
| |
| **SEQ ID NO:13** |
| amino acid sequence of heavy chain constant region of BNJ441 antibody |
| ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCCVECPPCPAPPVAGPSVFLFPPKPKDTLMISRTPEVTCVVVDVSQEDPE VQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGN VFSCSVLHEALHSHYTQKSLSLSLGK |
| **SEQ ID NO:14** |
| amino acid sequence of entire heavy chain of BNJ441 antibody |
| QVQLVQSGAEVKKPGASVKVSCKASGHIFSNYWIQWVRQAPGQGLEWM GEILPGSGHTEYTENFKDRVTMTRDTSTSTVYMELSSLRSEDTAVYYCAR YFFGSSPNWYFDVWGQGTLVTVSS ASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVLHEALHSHYTQKSLSLSLGK |
| **SEQ ID NO:15** |
| amino acid sequence of IgG2 heavy chain constant region variant comprising YTE substitutions |
| ASTKGPSVFPLAPCSRSTSESTAALGCLVKDYFPEPVTVSWNSGALTSGVH TFPAVLQSSGLYSLSSVVTVTSSNFGTQTYTCNVDHKPSNTKVDKTVERKC CVECPPCPAPPVAGPSVFLFPPKPKDTLYITREPEVTCVVVDVSHEDPEVQF NWYVDGMEVHNAKTKPREEQFNSTFRVVSVLTVVHQDWLNGKEYKCKV SNKGLPAPIEKTISKTKGQPREPQVYTLPPSREEMTKNQVSLTCLVKGFYP SDIAVEWESNGQPENNYKTTPPMLDSDGSFFLYSKLTVDKSRWQQGNVF SCSVMHEALHNHYTQKSLSLSPGK |
| **SEQ ID NO:16** |
| amino acid sequence of entire heavy chain of Eculizumab variant comprising heavy chain constant region depicted in SEQ ID NO:15 (above) |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVTSSNF GTQTYTCNVDHKPSNTKVDKTVERKCCVECPPCPAPPVAGPSVFLFPPKP KDTLYITREPEVTCVVVDVSHEDPEVQFNWYVDGMEVHNAKTKPREEQ FNSTFRVVSVLTVVHQDWLNGKEYKCKVSNKGLPAPIEKTISKTKGQPRE PQVYTLPPSREEMTKNQVSLTCLVKGFYPSDIAVEWESNGQPENNYKTTP |
| PMLDSDGSFFLYSKLTVDKSRWQQGNVFSCSVMHEALHNHYTQKSLSLS PGK |
| **SEQ ID NO:17** |
| amino acid sequence of light chain CDR1 of Eculizumab (as defined under Kabat definition) with glycine to histidine substitution at position 8 relative to SEQ ID NO:4 |
| GASENIYHALN |
| **SEQ ID NO:18** |
| depicts amino acid sequence of heavy chain CDR2 of Eculizumab in which serine at position 8 relative to SEQ ID NO:2 is substituted with histidine |
| EILPGSGHTEYTENFKD |
| **SEQ ID NO:19** |
| amino acid sequence of heavy chain CDR1 of Eculizumab in which tyrosine at position 2 (relative to SEQ ID NO:1) is substituted with histidine |
| GHIFSNYWIQ |
| **SEQ ID NO:20** |
| amino acid sequence of entire heavy chain of BNJ421 antibody |
| QVQLVQSGAEVKKPGASVKVSCKASGHIFSNYWIQWVRQAPGQGLEW MGEILPGSGHTEYTENFKDRVTMTRDTSTSTVYMELSSLRSEDTAVYYC ARYFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVPSSNFGTQT YTCNVDHKPSNTKVDKTVERKCCVECPPCPAPPVAGPSVFLFPPKPKDTLMIS RTPEVTCVVVDVSQEDPEVQFNWYVDGVEVHNAKTKPREEQFNSTYRVVSVL TVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVMHEALHNHYTQKSLSLSLGK |

### Aspects of the invention

1. A method of treating an adult human patient with a Membranoproliferative glomerulonephritis (MPGN), the method comprising administering to the patient an anti-C5 antibody, or antigen binding fragment thereof, comprising CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively,
   wherein the patient has been determined to have biopsy-proven MPGN, creatinine clearance greater than 20 ml/min per 1.73 m2, and/or 24-hour proteinuria exceeding 3.5 g in adults, and
   wherein the method comprises an administration cycle comprising (a) an induction phase followed by (b) a maintenance phase, wherein:
      (a) the induction phase comprises a period of four weeks, wherein the anti-C5 antibody, or antigen binding fragment thereof, is administered at a dose of 900 mg once per week; and
      (b) during the maintenance phase, the anti-C5 antibody, or antigen binding fragment thereof, is administered once at a dose of 1200 mg on the fifth week of the administration cycle, followed by 1200 mg every 14 ± 2 days thereafter.
2. The method of aspect 1, wherein the patient has been determined to have persistently low C3 levels in at least two consecutive evaluations and persistently high sC5b9 levels (>1000 ng/ml) in at least two previous consecutive evaluations.
3. A method of treating a pediatric human patient with a Membranoproliferative glomerulonephritis (MPGN), the method comprising administering to the patient an anti-C5 antibody, or antigen binding fragment thereof, comprising CDR1, CDR2, and CDR3 heavy chain sequences as set forth in SEQ ID NOs:1, 2, and 3, respectively, and CDR1, CDR2, and CDR3 light chain sequences as set forth in SEQ ID NOs:4, 5, and 6, respectively,
   wherein the patient has been determined to have biopsy-proven MPGN, creatinine clearance greater than 20 ml/min per 1.73 m2, and/or 24-hour proteinuria exceeding 40 mg/h/m2 (or exceeding 2 mg protein/mg creatinine in spot urine samples), and
   wherein the method comprises an administration cycle comprising (a) an induction phase followed by (b) a maintenance phase, wherein:
      (a) the anti-C5 antibody, or antigen binding fragment thereof, is administered during the induction phase at a dose of:
         1. 900 mg once per week for four weeks to a ≥ 40 kg patient;
         2. 600 mg once per week for two weeks to a 30 kg to < 40 kg patient;
         3. 600 mg once per week for two weeks to a 20 kg to < 30 kg patient;
         4. 600 mg once per week for one week to a 10 kg to < 20 kg patient;
         5. 300 mg once per week for one week to a 5 kg to < 10 kg patient; and
      (b) the anti-C5 antibody, or antigen binding fragment thereof, is administered during the maintenance phase at a dose of:
         1. 1200 mg on the fifth week of the administration cycle, followed by 1200 mg every two weeks thereafter, to a ≥ 40 kg patient;
         2. 900 mg on the third week of the administration cycle, followed by 900 mg every two weeks thereafter, to a 30 kg to < 40 kg patient;
         3. 600 mg on the third week of the administration cycle, followed by 600 mg every two weeks thereafter, to a 20 kg to < 30 kg patient;
         4. 300 mg on the second week of the administration cycle, followed by 300 mg every two weeks thereafter, to a 10 kg to < 20 kg patient; or
         5. 300 mg on the second week of the administration cycle, followed by 300 mg every three weeks thereafter, to a 5 kg to < 10 kg patient.
4. The method of aspect 3, wherein the patient has been determined to have persistently low C3 levels in at least two consecutive evaluations and persistently high sC5b9 levels (>1000 ng/ml) in at least two previous consecutive evaluations.
5. The method of any of the preceding aspects, wherein the anti-C5 antibody, or antigen-binding fragment thereof, comprises a heavy chain variable region depicted in SEQ ID NO: 7 and a light chain variable region depicted in SEQ ID NO:8.
6. The method of any of the preceding aspects, wherein the anti-C5 antibody, or antigen-binding fragment thereof, further comprises a heavy chain constant region depicted in SEQ ID NO:9.
7. The method of any of the preceding aspects, wherein the antibody, or antigen-binding fragment thereof, comprises a heavy chain polypeptide comprising the amino acid sequence depicted in SEQ ID NO:10 and a light chain polypeptide comprising the amino acid sequence depicted in SEQ ID NO:11.
8. The method of any of the preceding aspects, wherein the anti-C5 antibody, or antigen binding fragment thereof, is administered by intravenous infusion.
9. The method of any of the preceding aspects, wherein the anti-C5 antibody, or antigen binding fragment thereof, is administered to an adult human patient by intravenous infusion over a 25 minute to 45 minute period.
10. The method of any of the preceding aspects, wherein the anti-C5 antibody, or antigen binding fragment thereof, is administered to an adult human patient by intravenous infusion over a period not to exceed two hours.
11. The method of aspect 3, wherein the anti-C5 antibody, or antigen binding fragment thereof, is administered to a pediatric human patient aged 12 years to under 18 years by intravenous infusion over a period not to exceed two hours.
12. The method of aspect 3, wherein the anti-C5 antibody, or antigen binding fragment thereof, is administered to a pediatric human patient less than 12 years old by intravenous infusion over a period not to exceed four hours.
13. The method of aspect 3, wherein the anti-C5 antibody, or antigen binding fragment thereof, is administered at a dose of:
   a) 900 mg once per week for four weeks during the induction phase, 1200 mg on the fifth week of the administration cycle, followed by 1200 mg every two weeks thereafter during the maintenance phase, to a ≥ 40 kg patient;
   b) 600 mg once per week for two weeks during the induction phase, 900 mg on the third week of the administration cycle, followed by 900 mg every two weeks thereafter during the maintenance phase, to a 30 kg to < 40 kg patient;
   c) 600 mg once per week for two weeks during the induction phase, 600 mg on the third week of the administration cycle, followed by 600 mg every two weeks thereafter during the maintenance phase, to a 20 kg to < 30 kg patient;
   d) 600 mg once per week for one week during the induction phase, 300 mg on the second week of the administration cycle, followed by 300 mg every two weeks thereafter during the maintenance phase, to a 10 kg to < 20 kg patient;
   e) 300 mg once per week for one week during the induction phase, 300 mg on the second week of the administration cycle, followed by 300 mg every three weeks thereafter during the maintenance phase, to a 5 kg to < 10 kg patient.
14. The method of any of the preceding aspects, wherein the anti-C5 antibody, or antigen binding fragment thereof, is administered on a monthly basis after the maintenance phase.
15. The method of any of the preceding aspects, wherein the treatment reduces 24 hour proteinuria at week 24 compared to baseline.
16. The method of any of the preceding aspects, wherein the treatment reduces 24 hour proteinuria at week 48 compared to baseline.
17. The method of any of the preceding aspects, wherein the treatment results in a complete or partial remission of MPGN.
18. The method of any of the preceding aspects, wherein the treatment produces a shift toward normal levels of urinary albumin/creatinine ratio, serum creatinine, creatinine clearance, serum total proteins, serum albumin, LDL, HDL cholesterol and triglycerides levels, hematocrit and/or hemoglobin concentration.
19. The method of any of the preceding aspects, wherein the treatment improves one or more renal functional parameters selected from the group consisting of Glomerular Filtration Rate (GFR) (as assessed by Iohexol plasma clearance measurement), Albumin, IgG, sodium, potassium fractional clearance, and renal resistivity index (as assessed by ultrasound evaluation).
20. The method of any of the preceding aspects, wherein the MPGN is immune-complex-mediated MPGN" (IC-mediated MPGN).
21. The method of any one of aspects 1-19, wherein the MPGN is a C3 glomerulopathy.
22. The method of aspect 21, wherein the C3 glomerulopathy is dense deposit disease (DDD) or C3 glomerulonephritis.
23. A kit for treating MPGN in a human patient, the kit comprising: a dose of an anti-C5 antibody, or antigen binding fragment thereof, comprising: CDR1, CDR2, and CDR3 heavy chain sequences as set forth in SEQ ID NOs:1, 2, and 3, respectively, and CDR1, CDR2, and CDR3 light chain sequences as set forth in SEQ ID NOs: 4, 5, and 6, and; and instructions for using the anti-C5 antibody, or antigen binding fragment thereof, in the method of any one of the preceding aspects.
24. A method of treating an adult human patient with a Membranoproliferative glomerulonephritis (MPGN), the method comprising administering to the patient an anti-C5 antibody, or antigen binding fragment thereof, comprising CDR1, CDR2, and CDR3 heavy chain sequences as set forth in SEQ ID NOs: 19, 18, and 3, respectively, and CDR1, CDR2, and CDR3 light chain sequences as set forth in SEQ ID NOs:4, 5, and 6, respectively,
   wherein the patient has been determined to have biopsy-proven MPGN, creatinine clearance greater than 20 ml/min per 1.73 m2, and/or 24-hour proteinuria exceeding 3.5 g, and
   wherein the method comprises an administration cycle and, wherein the anti-C5 antibody, or antigen binding fragment thereof, is administered:
      (a) once on Day 1 of the administration cycle at a dose of : 2400 mg to a patient weighing ≥ 40 to < 60 kg, 2700 mg to a patient weighing ≥ 60 to < 100 kg, or 3000 mg to a patient weighing ≥ 100 kg; and
      (b) on Day 15 of the administration cycle and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.
25. The method of aspect 24, wherein the patient has been determined to have persistently low C3 levels in at least two consecutive evaluations and persistently high sC5b9 levels (>1000 ng/ml) in at least two previous consecutive evaluations.
26. The method of aspect 24 or 25, wherein the anti-C5 antibody, or antigen binding fragment thereof, is administered to a patient weighing ≥ 40 to < 60 kg:
   (a) once on Day 1 of the administration cycle at a dose of 2400 mg; and
   (b) on Day 15 of the administration cycle and every eight weeks thereafter at a dose of 3000 mg.
27. The method of aspect 24 or 25, wherein the anti-C5 antibody, or antigen binding fragment thereof, is administered to a patient weighing ≥ 60 to < 100 kg:
   (a) once on Day 1 of the administration cycle at a dose of 2700 mg; and
   (b) on Day 15 of the administration cycle and every eight weeks thereafter at a dose of 3300 mg.
28. The method of aspect 24 or 25, wherein the anti-C5 antibody, or antigen binding fragment thereof, is administered to a patient weighing ≥ 100 kg:
   (a) once on Day 1 of the administration cycle at a dose of 3000 mg; and
   (b) on Day 15 of the administration cycle and every eight weeks thereafter at a dose of 3600 mg.
29. The method of any of aspects 24-28, wherein the anti-C5 antibody, or antigen binding fragment thereof, comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434, each in EU numbering.
30. The method of any of aspects 24-28, wherein the anti-C5 antibody, or antigen-binding fragment thereof, comprises a heavy chain variable region depicted in SEQ ID NO: 12 and a light chain variable region depicted in SEQ ID NO:8.
31. The method of any of aspects 24-28, wherein the anti-C5 antibody, or antigen-binding fragment thereof, further comprises a heavy chain constant region depicted in SEQ ID NO:13.
32. The method of any of aspects 24-28, wherein the anti-C5 antibody, or antigen-binding fragment thereof, further comprises a heavy chain constant region depicted in SEQ ID NO:9.
33. The method of any of aspects 24-28, wherein the antibody, or antigen-binding fragment thereof, comprises a heavy chain polypeptide comprising the amino acid sequence depicted in SEQ ID NO:14 and a light chain polypeptide comprising the amino acid sequence depicted in SEQ ID NO:11.
34. The method of any of aspects 24-28, wherein the antibody, or antigen-binding fragment thereof, comprises a heavy chain polypeptide comprising the amino acid sequence depicted in SEQ ID NO:20 and a light chain polypeptide comprising the amino acid sequence depicted in SEQ ID NO:11.
35. The method of any of aspects 24-34, wherein the anti-C5 antibody, or antigen binding fragment thereof, is administered by intravenous infusion.
36. The method of any of aspects 24-35, wherein the anti-C5 antibody, or antigen binding fragment thereof, is administered to an adult human patient by intravenous infusion over a 25 minute to 45 minute period.
37. The method of any of aspects 24-36, wherein the anti-C5 antibody, or antigen binding fragment thereof, is administered to an adult human patient by intravenous infusion over a period not to exceed two hours.
38. The method of any of aspects 24-37, wherein the anti-C5 antibody, or antigen binding fragment thereof, is administered on a monthly basis after the maintenance phase.
39. The method of any of aspects 24-38, wherein the treatment reduces 24 hour proteinuria at week 24 compared to baseline.
40. The method of any of aspects 24-39, wherein the treatment reduces 24 hour proteinuria at week 48 compared to baseline.
41. The method of any of aspects 24-40, wherein the treatment results in a complete or partial remission of MPGN.
42. The method of any of aspects 24-41, wherein the treatment produces a shift toward normal levels of urinary albumin/creatinine ratio, serum creatinine, creatinine clearance, serum total proteins, serum albumin, LDL, HDL cholesterol and triglycerides levels, hematocrit and/or hemoglobin concentration.
43. The method of any of aspects 24-42, wherein the treatment improves one or more renal functional parameters selected from the group consisting of Glomerular Filtration Rate (GFR) (as assessed by Iohexol plasma clearance measurement), Albumin, IgG, sodium, potassium fractional clearance, and renal resistivity index (as assessed by ultrasound evaluation).
44. The method of any of aspects 24-43, wherein the MPGN is immune-complex-mediated MPGN" (IC-mediated MPGN).
45. The method of any of aspects 24-43, wherein the MPGN is a C3 glomerulopathy.
46. The method of aspect 45, wherein the C3 glomerulopathy is dense deposit disease (DDD) or C3 glomerulonephritis.
47. A kit for treating MPGN in a human patient, the kit comprising: a dose of an anti-C5 antibody, or antigen binding fragment thereof, comprising CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6; and instructions for using the anti-C5 antibody, or antigen binding fragment thereof, in the method of any one of aspects 25-46.

## Claims

1. An anti-C5 antibody, or antigen binding fragment thereof, for use in a method of treating an adult human patient with a Membranoproliferative glomerulonephritis (MPGN), wherein the anti-C5 antibody, or antigen binding fragment thereof, comprises CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6, respectively,
wherein the patient has been determined to have biopsy-proven MPGN, creatinine clearance greater than 20 ml/min per 1.73 m2, and/or 24-hour proteinuria exceeding 3.5 g, and
wherein the method comprises an administration cycle and, wherein the anti-C5 antibody, or antigen binding fragment thereof, is administered:
(a) once on Day 1 of the administration cycle at a dose of: 2400 mg to a patient weighing ≥ 40 to < 60 kg, 2700 mg to a patient weighing ≥ 60 to < 100 kg, or 3000 mg to a patient weighing ≥ 100 kg; and
(b) on Day 15 of the administration cycle and every eight weeks thereafter at a dose of 3000 mg to a patient weighing ≥ 40 to < 60 kg, 3300 mg to a patient weighing ≥ 60 to < 100 kg, or 3600 mg to a patient weighing ≥ 100 kg.

2. The anti-C5 antibody, or antigen binding fragment thereof, for use according to claim 1, wherein the patient has been determined to have persistently low C3 levels in at least two consecutive evaluations and persistently high sC5b9 levels (>1000 ng/ml) in at least two previous consecutive evaluations.

3. The anti-C5 antibody, or antigen binding fragment thereof, for use according to claim 1 or 2, wherein the anti-C5 antibody, or antigen binding fragment thereof, is administered to a patient weighing ≥ 40 to < 60 kg:
(a) once on Day 1 of the administration cycle at a dose of 2400 mg; and
(b) on Day 15 of the administration cycle and every eight weeks thereafter at a dose of 3000 mg.

4. The anti-C5 antibody, or antigen binding fragment thereof, for use according to claim 1 or 2, wherein the anti-C5 antibody, or antigen binding fragment thereof, is administered to a patient weighing ≥ 60 to < 100 kg:
(a) once on Day 1 of the administration cycle at a dose of 2700 mg; and
(b) on Day 15 of the administration cycle and every eight weeks thereafter at a dose of 3300 mg.

5. The anti-C5 antibody, or antigen binding fragment thereof, for use according to claim 1 or 2, wherein the anti-C5 antibody, or antigen binding fragment thereof, is administered to a patient weighing ≥ 100 kg:
(a) once on Day 1 of the administration cycle at a dose of 3000 mg; and
(b) on Day 15 of the administration cycle and every eight weeks thereafter at a dose of 3600 mg.

6. The anti-C5 antibody, or antigen binding fragment thereof, for use according to any one of claims 1 to 5, wherein the anti-C5 antibody, or antigen binding fragment thereof, comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434, each in EU numbering.

7. The anti-C5 antibody, or antigen binding fragment thereof, for use according to any one of claims 1 to 5, wherein the anti-C5 antibody, or antigen-binding fragment thereof, comprises a heavy chain variable region depicted in SEQ ID NO: 12 and a light chain variable region depicted in SEQ ID NO:8.

8. The anti-C5 antibody, or antigen binding fragment thereof, for use according to any one of claims 1 to 5, wherein the anti-C5 antibody, or antigen-binding fragment thereof, further comprises a heavy chain constant region depicted in SEQ ID NO:13.

9. The anti-C5 antibody, or antigen binding fragment thereof, for use according to any one of claims 1 to 5, wherein the anti-C5 antibody, or antigen-binding fragment thereof, further comprises a heavy chain constant region depicted in SEQ ID NO:9.

10. The anti-C5 antibody, or antigen binding fragment thereof, for use according to any one of claims 1 to 5, wherein the antibody, or antigen-binding fragment thereof, comprises a heavy chain polypeptide comprising the amino acid sequence depicted in SEQ ID NO:14 and a light chain polypeptide comprising the amino acid sequence depicted in SEQ ID NO:11.

11. The anti-C5 antibody, or antigen binding fragment thereof, for use according to any one of claims 1 to 5, wherein the antibody, or antigen-binding fragment thereof, comprises a heavy chain polypeptide comprising the amino acid sequence depicted in SEQ ID NO:20 and a light chain polypeptide comprising the amino acid sequence depicted in SEQ ID NO:11.

12. The anti-C5 antibody, or antigen binding fragment thereof, for use according to any one of the preceding claims, wherein the anti-C5 antibody, or antigen binding fragment thereof, is administered by intravenous infusion.

13. The anti-C5 antibody, or antigen binding fragment thereof, for use according to any one of the preceding claims, wherein the treatmentu:
(a) reduces 24 hour proteinuria at week 24 compared to baseline;
(b) reduces 24 hour proteinuria at week 48 compared to baseline;
(c) results in a complete or partial remission of MPGN;
(d) produces a shift toward normal levels of urinary albumin/creatinine ratio, serum creatinine, creatinine clearance, serum total proteins, serum albumin, LDL, HDL cholesterol and triglycerides levels, hematocrit and/or hemoglobin concentration; and/or
(e) improves one or more renal functional parameters selected from the group consisting of Glomerular Filtration Rate (GFR) (as assessed by Iohexol plasma clearance measurement), Albumin, IgG, sodium, potassium fractional clearance, and renal resistivity index (as assessed by ultrasound evaluation).

14. The anti-C5 antibody, or antigen binding fragment thereof, for use according to any one of the preceding claims, wherein the MPGN is immune-complex-mediated MPGN" (IC-mediated MPGN).

15. The anti-C5 antibody, or antigen binding fragment thereof, for use according to any one of claims 1 to 13, wherein the MPGN is a C3 glomerulopathy, optionally wherein the C3 glomerulopathy is dense deposit disease (DDD) or C3 glomerulonephritis.
